# EUROPEAN PATENT SPECIFICATION

(11) **EP 2 333 118 B1**
(45) Date of publication and mention of the grant of the patent: **18.12.2013**
(21) Application number: 10179789.2
(22) Date of filing: 04.03.2002
(51) Int. Cl.: C12Q 1/68, C12Q 1/70, C12P 19/34, G01N 33/53, G01N 33/566, C07H 21/02, C07K 5/00

(54) **Method for rapid detection and identification of bioagents**
Schnellverfahren zum Nachweis und zur Identifizierung von Bioagentien
Procédé de détection et d'identification rapides de bioagents

(30) Priority: 02.03.2001 US 798007
(43) Date of publication of application: 15.06.2011
(62) Divisional of application: 02709785.6
(73) Proprietor: Ibis Biosciences, Inc., Carlsbad, CA 92008 (US)
(72) Inventor: Ecker, David J., Encinitas, CA 92024 (US); Griffey, Richard H., Vista, CA 92084 (US); Sampath, Rangarajan, Carlsbad, CA 92008 (US); Hofstadler, Steven, Carlsbad, CA 92008 (US); McNeil, John, La Jolla, CA 92037 (US)
(74) Representative: Hallybone, Huw George

(56) References cited:
- JOHNSON Y A ET AL: "Precise molecular weight determination of PCR products of the rRNA intergenic spacer region using electrospray quadrupole mass spectrometry for differentiation of B. subtilis and B. atrophaeus, closely related species of bacilli.", JOURNAL OF MICROBIOLOGICAL METHODS. MAY 2000, vol. 40, no. 3, May 2000 (2000-05), pages 241-254, XP002343081, ISSN: 0167-7012
- VAN BAAR B L M: "Characterisation of bacteria by matrix-assisted laser desorption/ionisation and electrospray mass spectrometry", FEMS MICROBIOLOGY REVIEWS, ELSEVIER, AMSTERDAM, NL, vol. 24, 1 January 2000 (2000-01-01), pages 195-219, XP002980425, ISSN: 0168-6445, DOI: DOI:10.1016/S0168-6445(99)00036-4
- MUDDIMAN D C ET AL: "Characterization of PCR Products from Bacilli Using Electrospray Ionization FTICR Mass Spectrometry", ANALYTICAL CHEMISTRY, AMERICAN CHEMICAL SOCIETY, US, vol. 68, no. 21, 1 November 1996 (1996-11-01), pages 3705-3712, XP002980423, ISSN: 0003-2700, DOI: DOI:10.1021/AC960689J
- MUDDIMAN ET AL: "length and base composition of PCR-amplified nucleic acids using mass measurements from electrospray ionization mass spectromety", ANALYTICAL CHEMISTRY, AMERICAN CHEMICAL SOCIETY, US, vol. 69, no. 8, 15 April 1997 (1997-04-15) , pages 1543-1549, XP003000271, ISSN: 0003-2700, DOI: DOI:10.1021/AC961134R
- HURST G B ET AL: "MALDI-TOF Analysis of Polymerase Chain Reaction Products from Methanotrophic Bacteria", ANALYTICAL CHEMISTRY, AMERICAN CHEMICAL SOCIETY, US, vol. 70, no. 13, 1 July 1998 (1998-07-01), pages 2693-2698, XP002980421, ISSN: 0003-2700, DOI: DOI:10.1021/AC980044E
- HURST G B ET AL: "Detection of bacterial DNA polymerase chain reaction products by matrix assisted laser desorption/ionization mass spectrometry", RAPID COMMUNICATIONS IN MASS SPECTROMETRY, HEYDEN, LONDON, GB, vol. 10, 1 January 1996 (1996-01-01), pages 377-382, XP002955968, ISSN: 0951-4198, DOI: DOI:10.1002/(SICI)1097-0231(199602)10:3<37 7::AID-RCM481>3.3.CO;2-O
- MCLAFFERTY ET AL: "Comparison of algorithms and databases for matching unknown mass spectra", JOURNAL OF THE AMERICAN SOCIETY FOR MASS SPECTROMETRY, ELSEVIER SCIENCE INC, US, vol. 9, no. 1, 1 January 1998 (1998-01-01) , pages 92-95, XP005497106, ISSN: 1044-0305, DOI: DOI:10.1016/S1044-0305(97)00235-3
- VAN ERT MATTHEW N ET AL: "Mass spectrometry provides accurate characterization of two genetic marker types in Bacillus anthracis", BIOTECHNIQUES, vol. 37, no. 4, October 2004 (2004-10), pages 642-644, 646, XP009053164, ISSN: 0736-6205

## Description

### FIELD OF THE INVENTION

The present invention relates to methods for rapid detection and identification of bioagents from environmental, clinical or other samples. The methods provide for detection and characterization of a unique base composition signature (BCS) from any bioagent, including bacteria and viruses. The unique BCS is used to rapidly identify the bioagent.

### BACKGROUND OF THE INVENTION

Rapid and definitive microbial identification is desirable for a variety of industrial, medical, environmental, quality, and research reasons. Traditionally, the microbiology laboratory has functioned to identify the etiologic agents of infectious diseases through direct examination and culture of specimens. Since the mid-1980s, researchers have repeatedly demonstrated the practical utility of molecular biology techniques, many of which form the basis of clinical diagnostic assays. Some of these techniques include nucleic acid hybridization analysis, restriction enzyme analysis, genetic sequence analysis, and separation and purification of nucleic acids (See, e.g., J. Sambrook, E. F. Fritsch, and T. Maniatis, Molecular Cloning: A Laboratory Manual, 2nd Ed., Cold Spring Harbor Laboratory Press, Cold Spring Harbor, N.Y., 1989). These procedures, in general, are time-consuming and tedious. Another option is the polymerase chain reaction (PCR) or other amplification procedure which amplifies a specific target DNA sequence based on the flanking primers used. Finally, detection and data analysis convert the hybridization event into an analytical result.

Other techniques for detection of bioagents include high-resolution mass spectrometry (MS), low-resolution MS, fluorescence, radioiodination, DNA chips and antibody techniques. None of these techniques is entirely satisfactory.

Mass spectrometry provides detailed information about the molecules being analyzed, including high mass accuracy. It is also a process that can be easily automated. However, high-resolution MS alone fails to perform against unknown or bioengineered agents, or in environments where there is a high background level of bioagents ("cluttered" background). Low-resolution MS can fail to detect some known agents, if their spectral lines are sufficiently weak or sufficiently close to those from other living organisms in the sample. DNA chips with specific probes can only determine the presence or absence of specifically anticipated organisms. Because there are hundreds of thousands of species of benign bacteria, some very similar in sequence to threat organisms, even arrays with 10,000 probes lack the breadth needed to detect a particular organism.

Antibodies face more severe diversity limitations than arrays. If antibodies are designed against highly conserved targets to increase diversity, the false alarm problem will dominate, again because threat organisms are very similar to benign ones. Antibodies are only capable of detecting known agents in relatively uncluttered environments.

Several groups have described detection of PCR products using high resolution electrospray ionization -Fourier transform- ion cyclotron resonance mass spectrometry (ESI-FT-ICR MS). Accurate measurement of exact mass combined with knowledge of the number of at least one nucleotide allowed calculation of the total base composition for PCR duplex products of approximately 100 base pairs. (Aaserud et al., J. Am. Soc. Mass Spec. 7:1266-1269, 1996; Muddiman et al., Anal. Chem. 69:1543-1549, 1997; Wunschel et al., Anal. Chem. 70:1203-1207, 1998; Muddiman et al., Rev. Anal. Chem. 17:1-68, 1998). Electrospray ionization-Fourier transform-ion cyclotron resistance (ESI-FT-ICR) MS may be used to determine the mass of double-stranded, 500 base-pair PCR products via the average molecular mass (Hurst et al., Rapid Commun. Mass Spec. 10:377-382, 1996). The use of matrix-assisted laser desorption ionization-time of flight (MALDI-TOF) mass spectrometry for characterization of PCR products has been described. (Muddiman et al., Rapid Common. Mass Spec. 13:1201-1204,1999). However, the degradation of DNAs over about 75 nucleotides observed with MALDI limited the utility of this method.

Johnson et al. (J. Microbiol. Methods, 2000, Vol. 40, p. 241-254) describes the precise molecular weight determination of PCR products of the rRNA intergenic spacer region using electrospray quadrupole mass spectrometry for differentiation of *B. subtilis* and *B. atrophaeus*, closely related species of bacteria.

U.S. Pat. No. 5,849,492 describes a method for retrieval of phylogentically informative DNA sequences which comprise searching for a highly divergent segment of genomic DNA surrounded by two highly conserved segments, designing the universal primers for PCR amplification of the highly divergent region, amplifying the genomic DNA by PCR technique using universal primers, and then sequencing the gene to determine the identity of the organism.

U.S. Patent No. 5,965,363 discloses methods for screening nucleic acids for polymorphisms by analyzing amplified target nucleic acids using mass spectrometric techniques and to procedures for improving mass resolution and mass accuracy of these methods.

WO 99/14375 describes methods, PCR primers and kits for use in analyzing preselected DNA tandem nucleotide repeat alleles by mass spectrometry.

WO 98/12355 discloses methods of determining the mass of a target nucleic acid by mass spectrometric analysis, by cleaving the target nucleic acid to reduce its length, making the target single-stranded and using MS to determine the mass of the single-stranded shortened target. Also disclosed are methods of preparing a double-stranded target nucleic acid for MS analysis comprising amplification of the target nucleic acid, binding one of the strands to a solid support, releasing the second strand and then releasing the first strand which is then analyzed by MS. Kits for target nucleic acid preparation are also provided.

PCT WO97/33000 discloses methods for detecting mutations in a target nucleic acid by nonrandomly fragmenting the target into a set of single-stranded nonrandom length fragments and determining their masses by MS.

U.S. Patent No. 5,605,798 describes a fast and highly accurate mass spectrometer-based process for detecting the presence of a particular nucleic acid in a biological sample for diagnostic purposes.

WO 98/21066 describes processes for determining the sequence of a particular target nucleic acid by mass spectrometry. Processes for detecting a target nucleic acid present in a biological sample by PCR amplification and mass spectrometry detection are disclosed, as are methods for detecting a target nucleic acid in a sample by amplifying the target with primers that contain restriction sites and tags, extending and cleaving the amplified nucleic acid, and detecting the presence of extended product, wherein the presence of a DNA fragment of a mass different from wild-type is indicative of a mutation. Methods of sequencing a nucleic acid via mass spectrometry methods are also described.

WO 97/37041, WO 99/31278 and US Patent No. 5,547,835 describe methods of sequencing nucleic acids using mass spectrometry. US Patent Nos. 5,622,824, 5,872,003 and 5,691,141 describe methods, systems and kits for exonuclease-mediated mass spectrometric sequencing.

Thus, there is a need for a method for bioagent detection and identification which is both specific and rapid, and in which no nucleic acid sequencing is required. The present invention addresses this need.

### SUMMARY OF THE INVENTION

The invention provides a method of identifying an unknown bioagent comprising:
a) aligning a plurality of nucleic acid gene sequences of a plurality of organisms of known identity;
b) analyzing said plurality of sequences for regions of conservation and variation;
c) identifying a plurality of primer binding sites flanking variable regions for priming and obtaining one or more amplifiable regions within said plurality of sequences;
d) identifying the base compositions of said one or more amplifiable regions for members of said plurality of known organisms to obtain a plurality of base compositions of amplifiable regions indexed to the identity of one or more members of said plurality of organisms of known identity;
e) preparing a database containing said base compositions and indexed identities of members of said plurality of organisms obtained in step d).
f) determining a base composition of a nucleic acid of said unknown bioagent, by contacting the nucleic acid with at least one pair of oligonucleotide primers which hybridize to sequences of said one or more amplifiable regions, amplifying the variable nucleic acid sequence to produce an amplification product, determining the molecular mass of said amplification product by mass spectrometry and then determining the base composition of the amplification product; and
g) identifying said unknown bioagent by comparing said base composition of said nucleic acid of said unknown bioagent to said database.

Other features of the invention are set out in the appended claims.

### SUMMARY OF THE DISCLOSURE

One embodiment of the present disclosure is a method of identifying an unknown bioagent comprising (a) contacting nucleic acid from the bioagent with at least one pair of oligonucleotide primers which hybridize to sequences of the nucleic acid and flank a variable nucleic acid sequence; (b) amplifying the variable nucleic acid sequence to produce an amplification product; (c) determining the molecular mass of the amplification product; and (d) comparing the molecular mass to one or more molecular masses of amplification products obtained by performing steps (a)-(c) on a plurality of known organisms, wherein a match identifies the unknown bioagent. In one aspect of this preferred embodiment, the sequences to which the at least one pair of oligonucleotide primers hybridize are highly conserved. Preferably, the amplifying step comprises polymerase chain reaction. Alternatively, the amplifying step comprises ligase chain reaction or strand displacement amplification. In one aspect of this preferred embodiment, the bioagent is a bacterium, virus, cell or spore. Advantageously, the nucleic acid is ribosomal RNA. In another aspect, the nucleic acid encodes RNase P or an RNA-dependent RNA polymerase. Preferably, the amplification product is ionized prior to molecular mass determination. The method may further comprise the step of isolating nucleic acid from the bioagent prior to contacting the nucleic acid with the at least one pair of oligonucleotide primers. The method may further comprise the step of performing steps (a)-(d) using a different oligonucleotide primer pair and comparing the results to one or more molecular mass amplification products obtained by performing steps (a)-(c) on a different plurality of known organisms from those in step (d). Preferably, the one or more molecular mass is contained in a database of molecular masses. In another aspect of this preferred embodiment, the amplification product is ionized by electrospray ionization, matrix-assisted laser desorption or fast atom bombardment. Advantageously, the molecular mass is determined by mass spectrometry. Preferably, the mass spectrometry is Fourier transform ion cyclotron resonance mass spectrometry (FT-ICR-MS), ion trap, quadrupole, magnetic sector, time of flight (TOF), Q-TOF or triple quadrupole. The method may further comprise performing step (b) in the presence of an analog of adenine, thymidine, guanosine or cytidine having a different molecular weight than adenosine, thymidine, guanosine or cytidine. In one aspect, the oligonucleotide primer comprises a base analog or substitute base at positions 1 and 2 of each triplet within the primer, wherein the base analog or substitute base binds with increased affinity to its complement compared to the native base. Preferably, the primer comprises a universal base at position 3 of each triplet within the primer. The base analog or substitute base may be 2,6-diaminopurine, propyne T, propyne G, phenoxazines or G-clamp. Preferably, the universal base is inosine, guanidine, uridine, 5-nitroindole, 3-nitropyrrole, d**P** or d**K**, or 1-(2-deoxy-β-D-ribofuranosyl)-imidazole-4-carboxamide.

Another embodiment of the present disclosure is a method of identifying an unknown bioagent comprising (a) contacting nucleic acid from the bioagent with at least one pair of oligonucleotide primers which hybridize to sequences of the nucleic acid and flank a variable nucleic acid sequence; (b) amplifying the variable nucleic acid sequence to produce an amplification product; (c) determining the base composition of the amplification product; and (d) comparing the base composition to one or more base compositions of amplification products obtained by performing steps (a)-(c) on a plurality of known organisms, wherein a match identifies the unknown bioagent. In one aspect of this preferred embodiment, the sequences to which the at least one pair of oligonucleotide primers hybridize are highly conserved. Preferably, the amplifying step comprises polymerase chain reaction. Alternatively, the amplifying step comprises ligase chain reaction or strand displacement amplification. In one aspect of this preferred embodiment, the bioagent is a bacterium, virus, cell or spore. Advantageously, the nucleic acid is ribosomal RNA. In another aspect, the nucleic acid encodes RNase P or an RNA-dependent RNA polymerase. Preferably, the amplification product is ionized prior to molecular mass determination. The method may further comprise the step of isolating nucleic acid from the bioagent prior to contacting the nucleic acid with the at least one pair of oligonucleotide primers. The method may further comprise the step of performing steps (a)-(d) using a different oligonucleotide primer pair and comparing the results to one or more base composition signatures of amplification products obtained by performing steps (a)-(c) on a different plurality of known organisms from those in step (d). Preferably, the one or more base compositions is contained in a database of base compositions. In another aspect of this preferred embodiment, the amplification product is ionized by electrospray ionization, matrix-assisted laser desorption or fast atom bombardment. Advantageously, the molecular mass is determined by mass spectrometry. Preferably, the mass spectrometry is Fourier transform ion cyclotron resonance mass spectrometry (FT-ICR-MS), ion trap, quadrupole, magnetic sector, time of flight (TOF), Q-TOF or triple quadrupole. The method may further comprise performing step (b) in the presence of an analog of adenine, thymidine, guanosine or cytidine having a different molecular weight than adenosine, thymidine, guanosine or cytidine. In one aspect, the oligonucleotide primer comprises a base analog or substitute base at positions 1 and 2 of each triplet within the primer, wherein the base analog or substitute base binds with increased affinity to its complement compared to the native base. Preferably, the primer comprises a universal base at position 3 of each triplet within the primer. The base analog or substitute base may be 2,6-diaminopurine, propyne T, propyne G, phenoxazines or G-clamp. Preferably, the universal base is inosine, guanidine, uridine, 5-nitroindole, 3-nitropyrrole, d**P** or d**K**, or 1-(2-deoxy-β-D-ribofuranosyl)-imidazole-4-carboxamide.

The present disclosure also provides a method for detecting a single nucleotide polymorphism in an individual, comprising the steps of (a) isolating nucleic acid from the individual; (b) contacting the nucleic acid with oligonucleotide primers which hybridize to regions of the nucleic acid which flank a region comprising the potential polymorphism; (c) amplifying the region to produce an amplification product; (d) determining the molecular mass of the amplification product; and (e) comparing the molecular mass to the molecular mass of the region in an individual known to have the polymorphism, wherein if the molecular masses are the same then the individual has the polymorphism.

In one aspect of this preferred embodiment, the primers hybridize to highly conserved sequences. Preferably, the polymorphism is associated with a disease. Alternatively, the polymorphism is a blood group antigen. In one aspect of the preferred embodiment, the amplifying step is polymerase chain reaction. Alternatively, the amplification step is ligase chain reaction or strand displacement amplification. Preferably, the amplification product is ionized prior to mass determination. In one aspect, the amplification product is ionized by electrospray ionization, matrix-assisted laser desorption or fast atom bombardment. Advantageously, the molecular mass is determined by mass spectrometry. Preferably, the mass spectrometry is Fourier transform ion cyclotron resonance mass spectrometry (FT-ICR-MS), ion trap, quadrupole, magnetic sector, time of flight (TOF), Q-TOF or triple quadrupole.

### BRIEF DESCRIPTION OF THE DRAWINGS

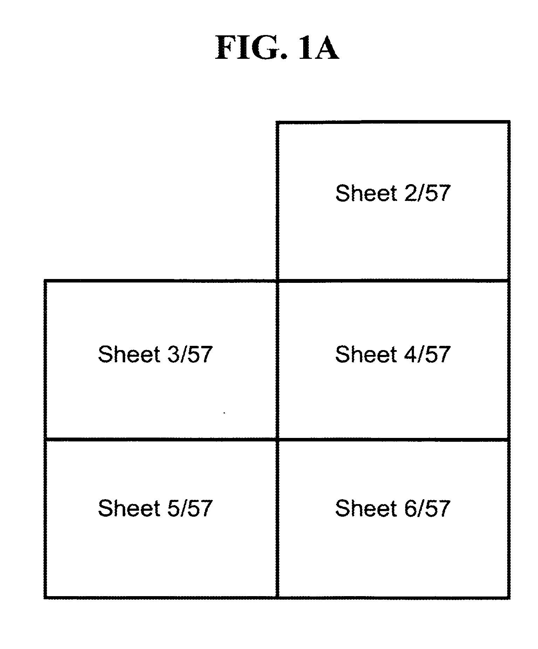

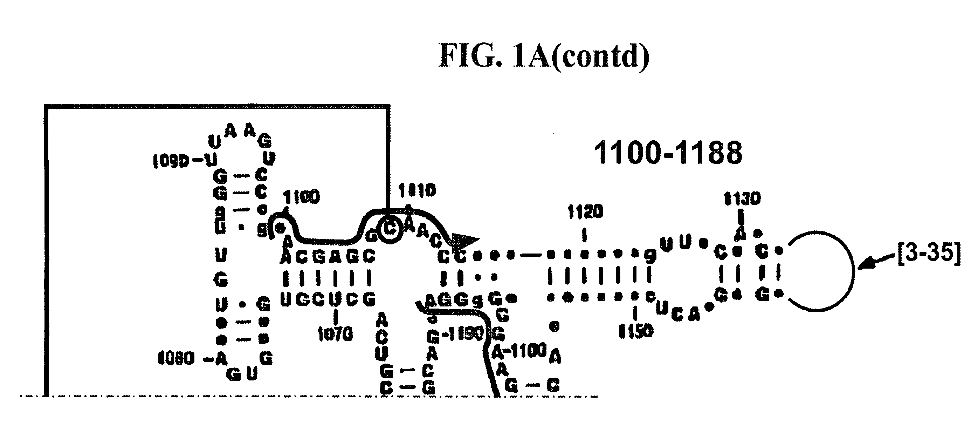

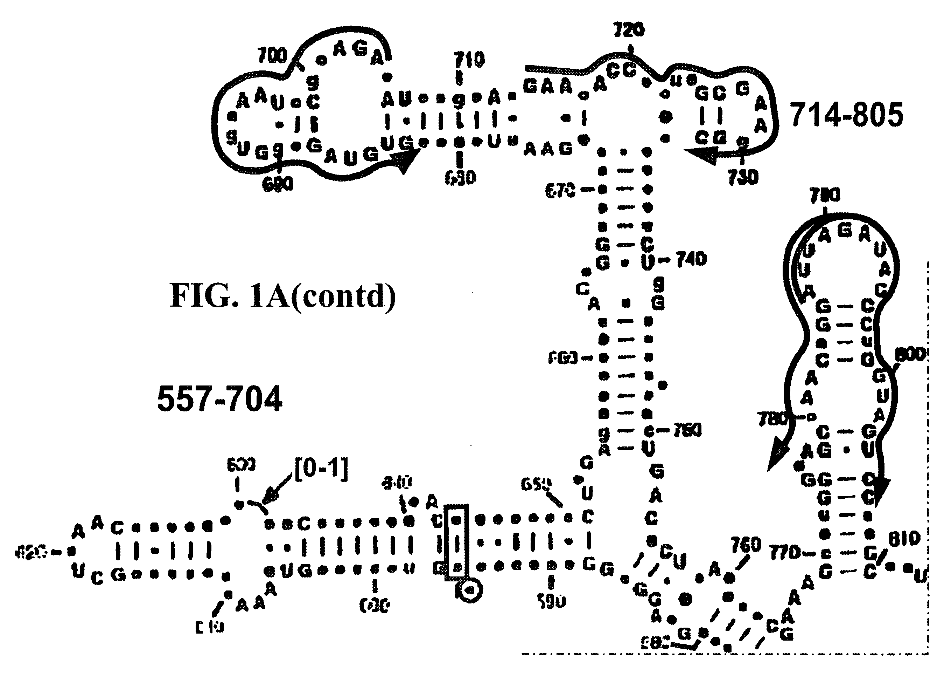

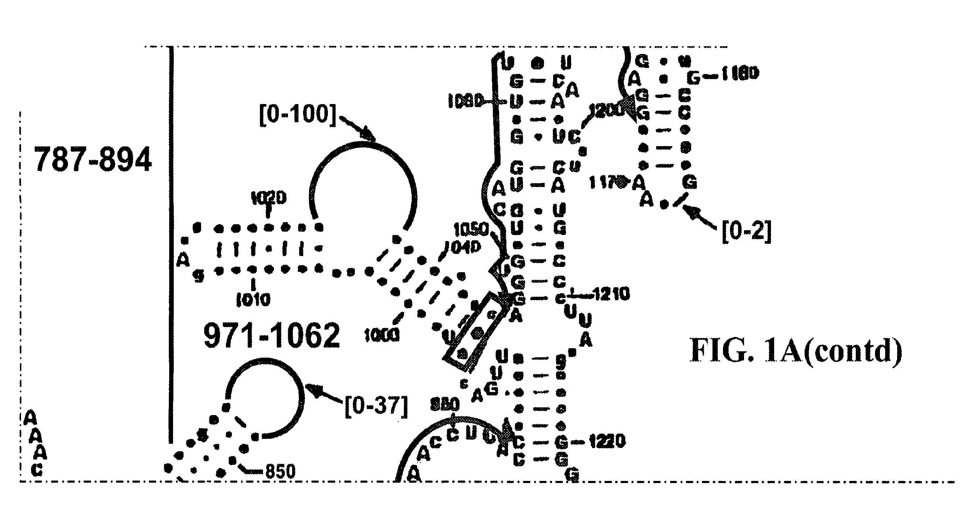

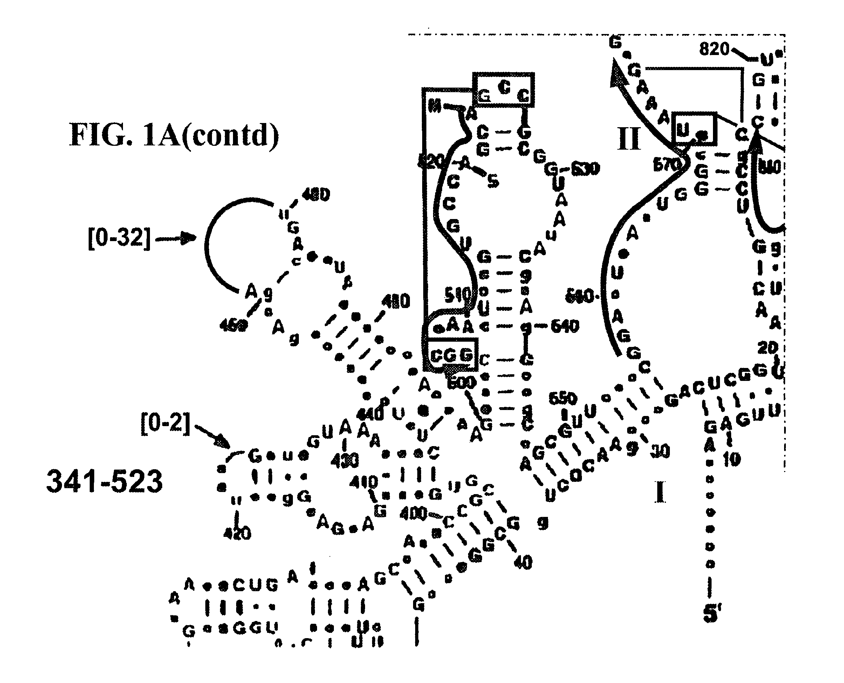

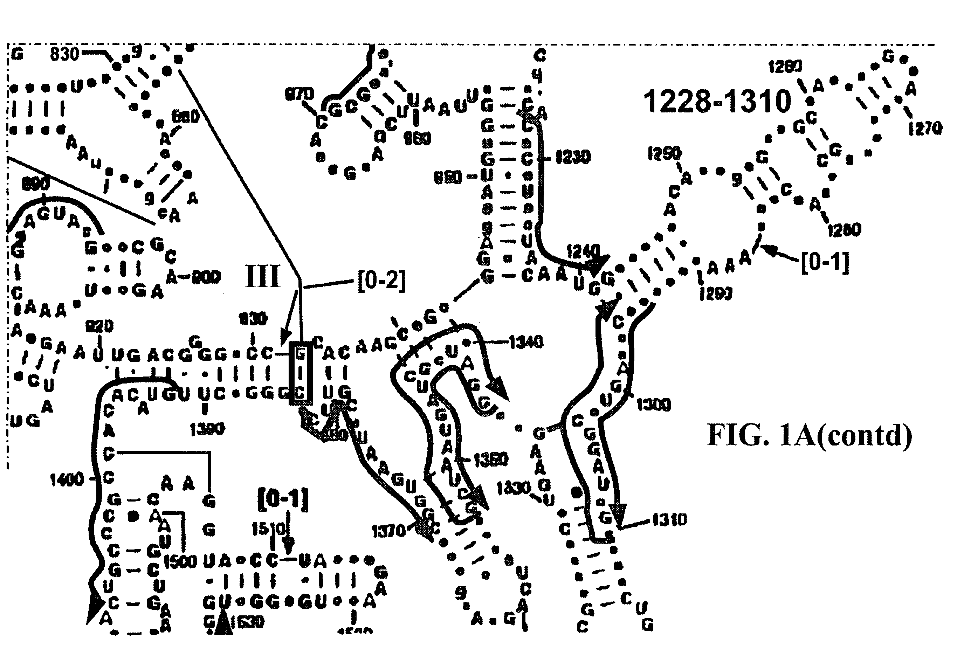

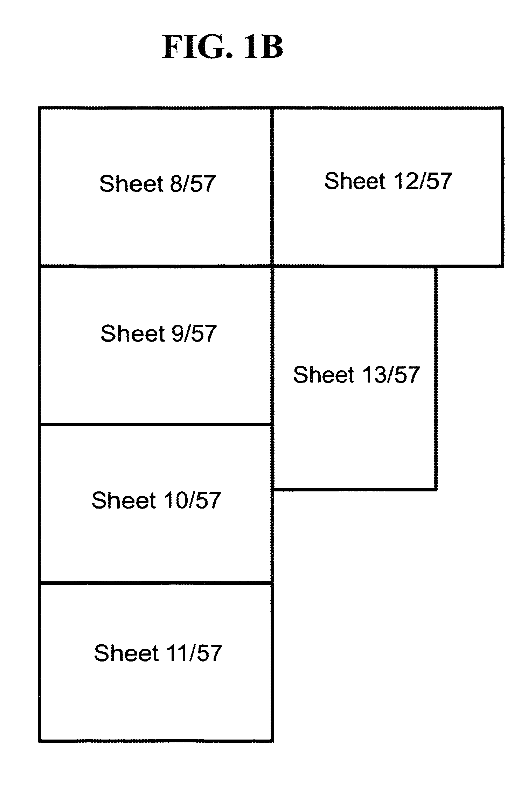

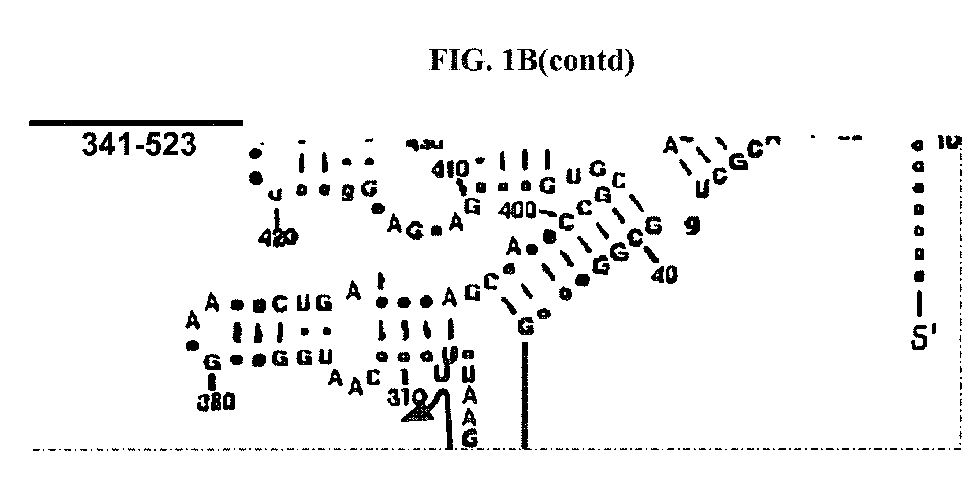

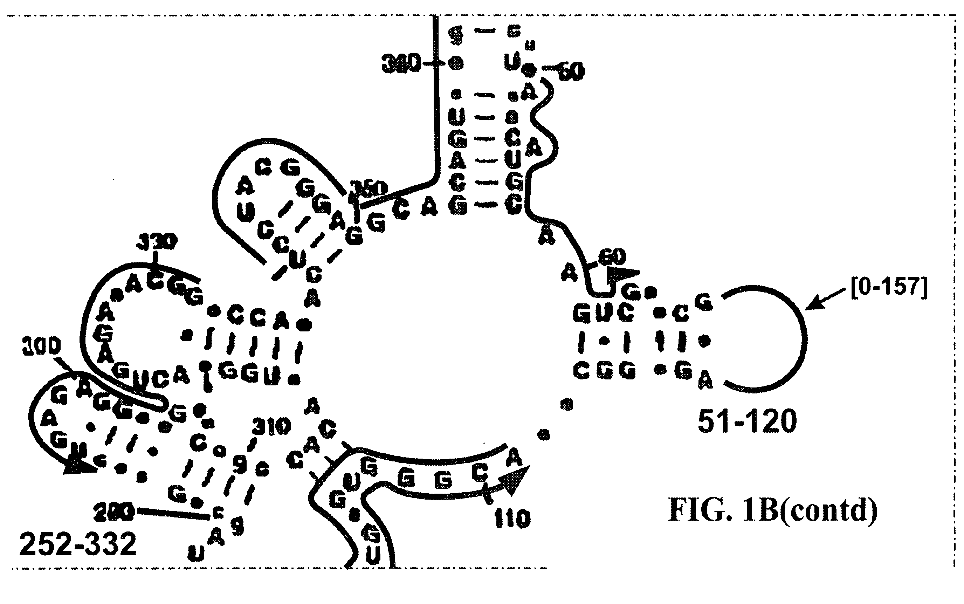

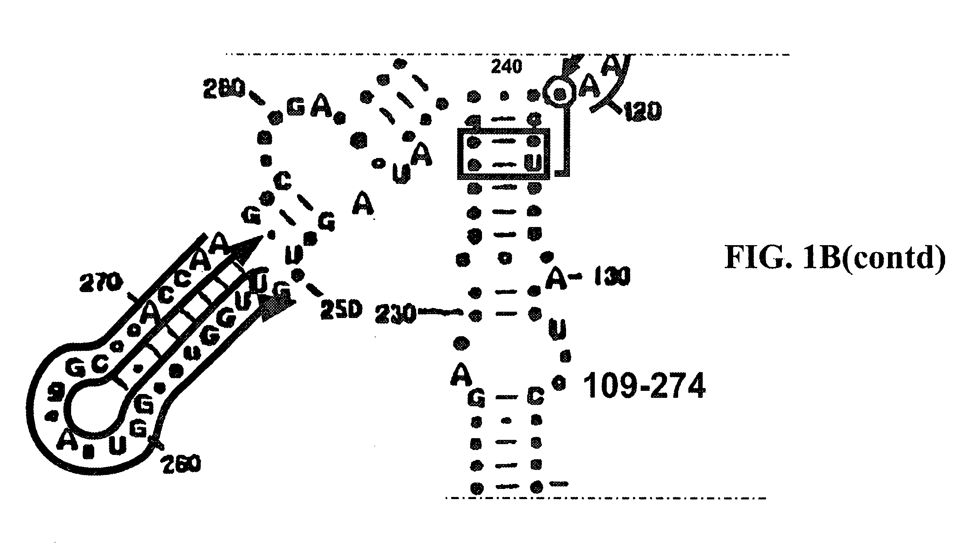

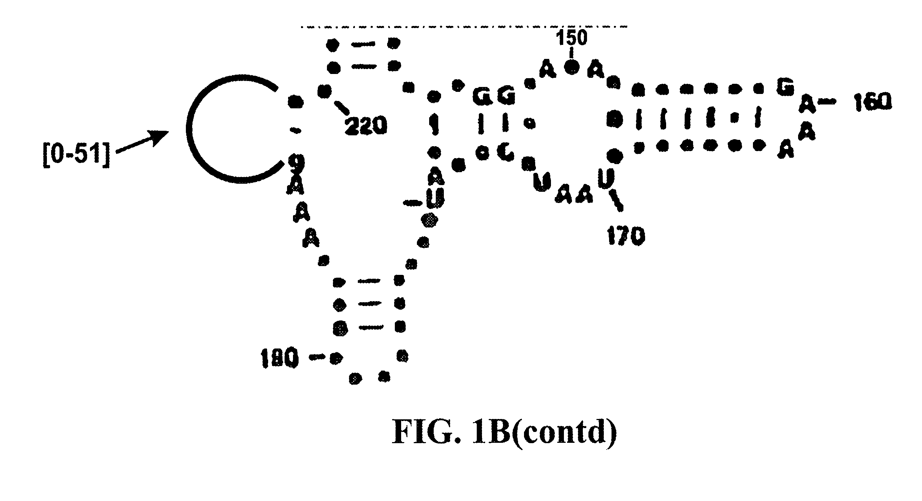

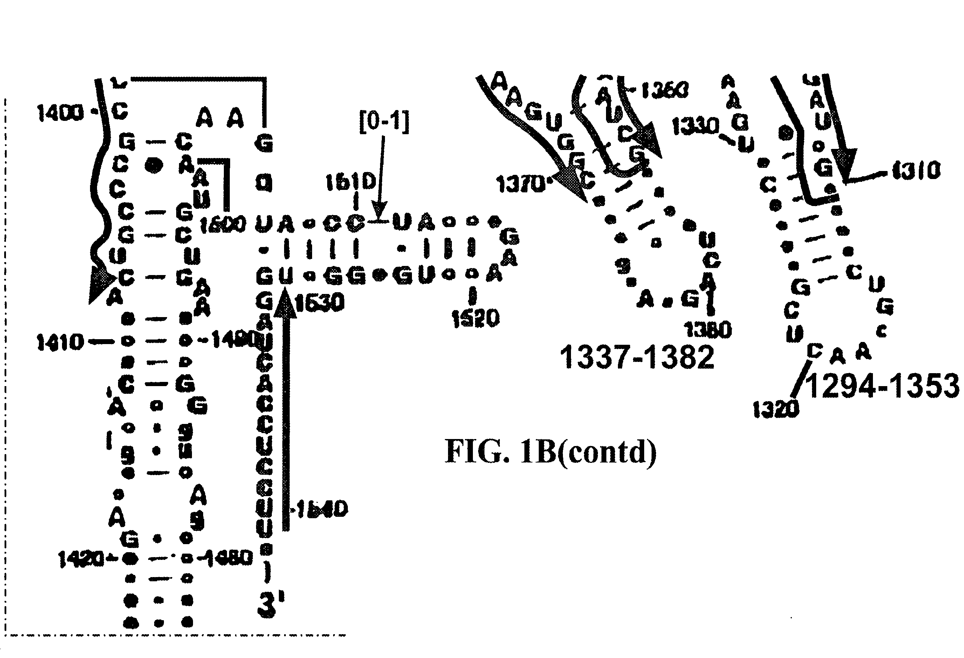

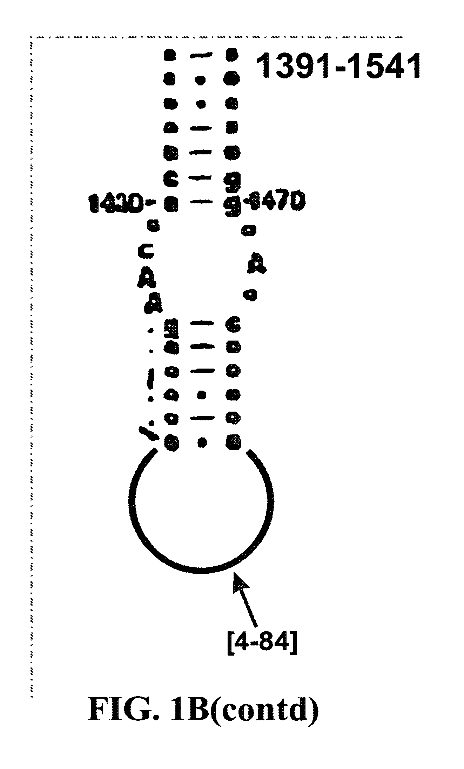

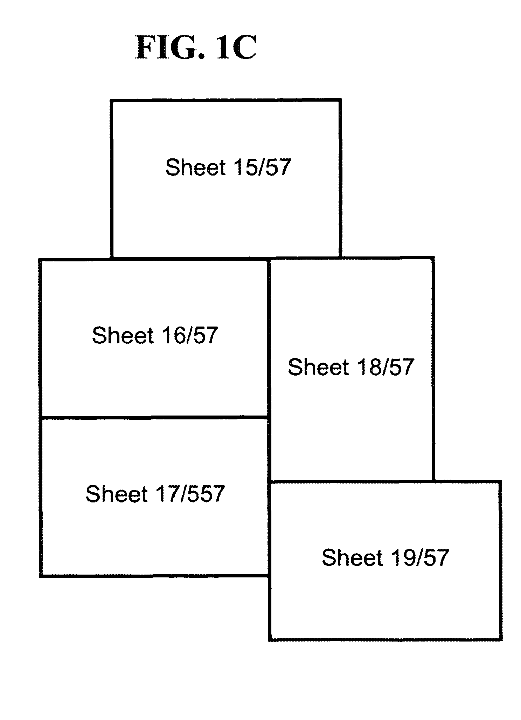

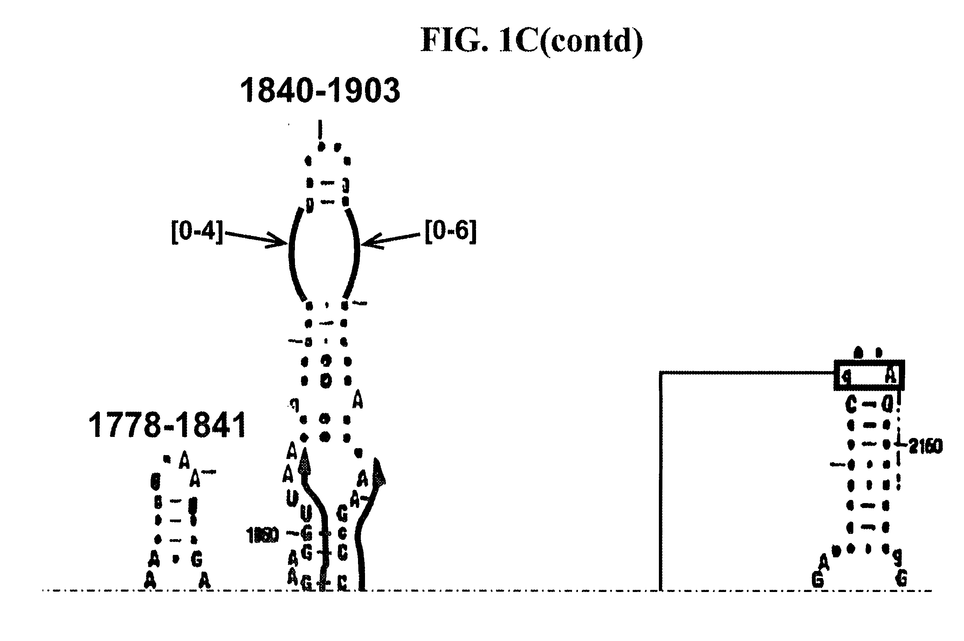

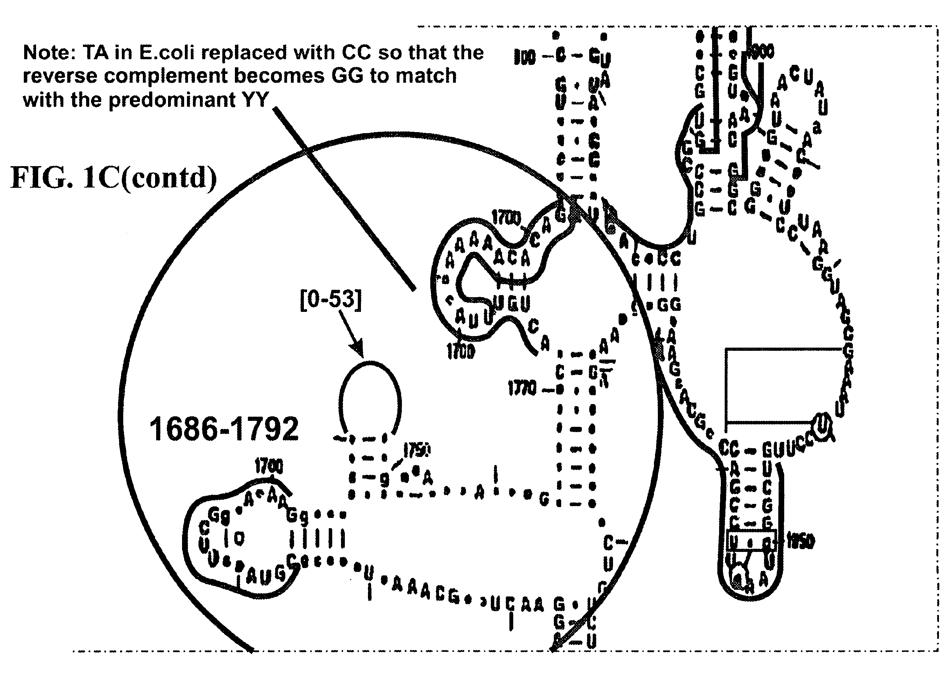

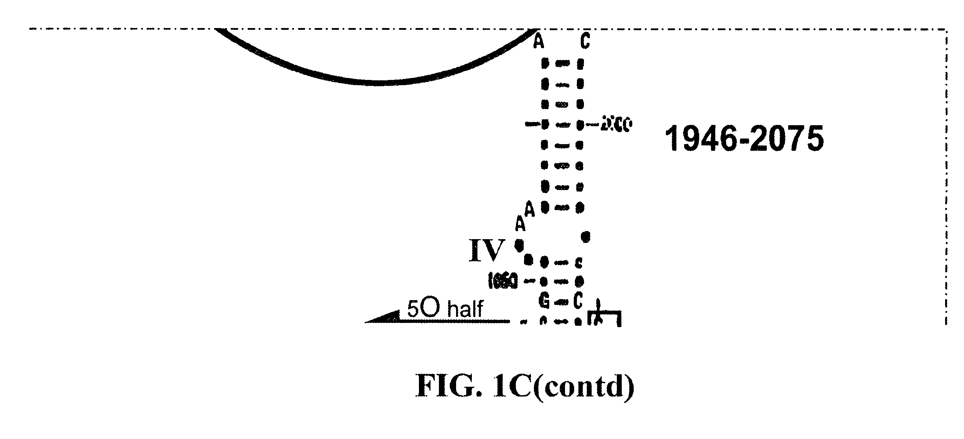

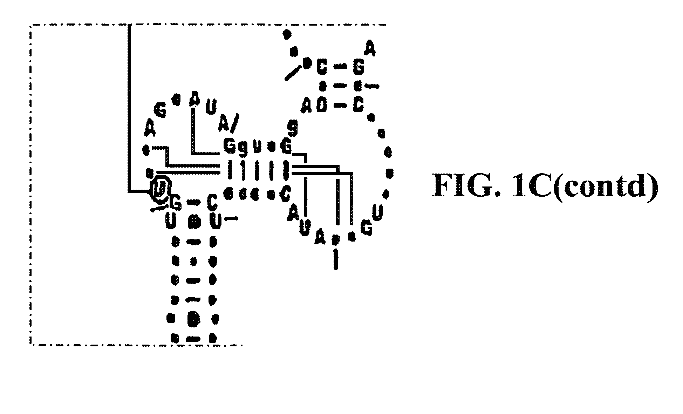

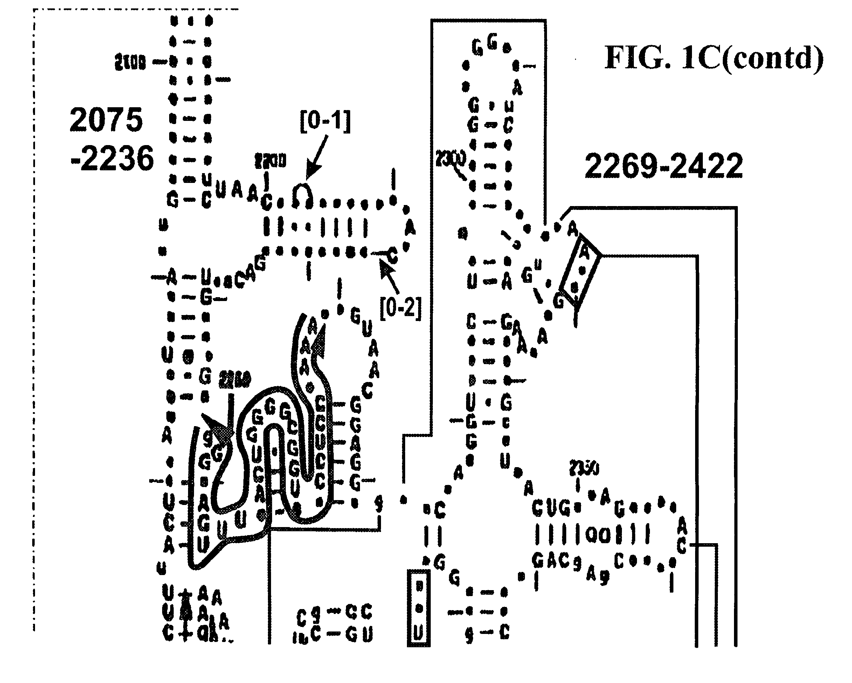

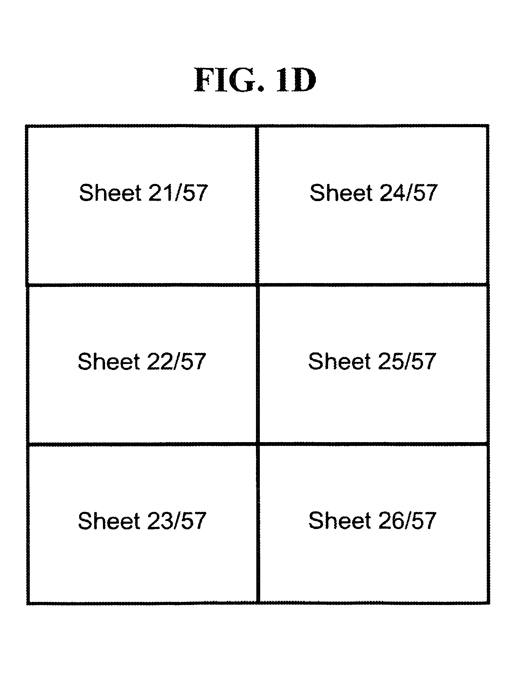

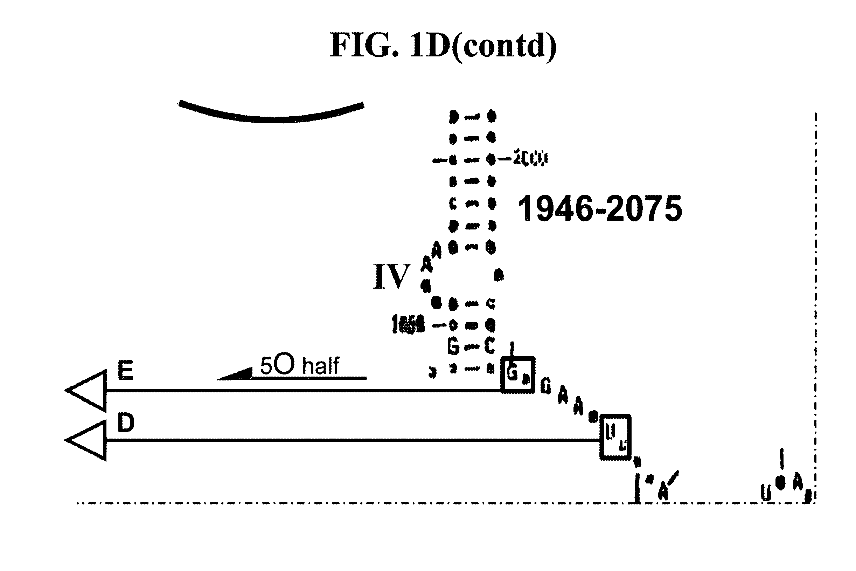

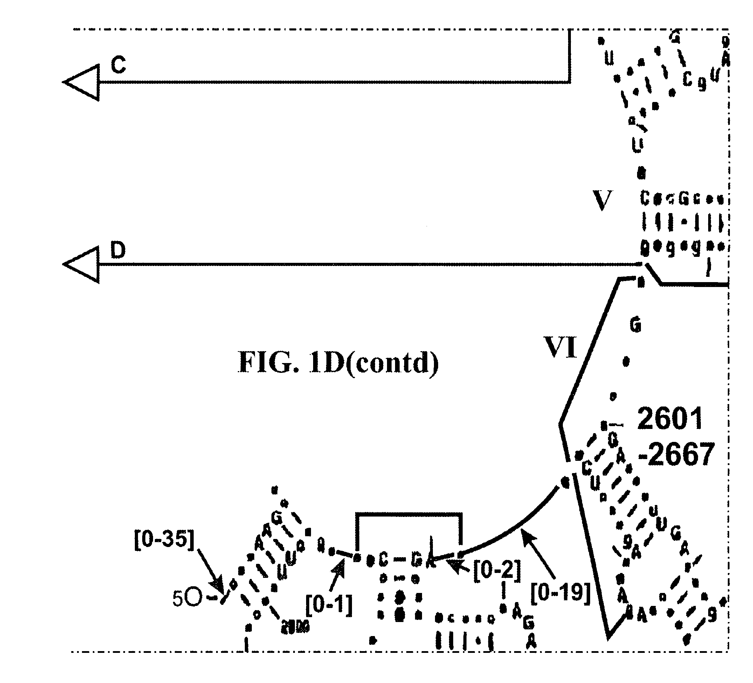

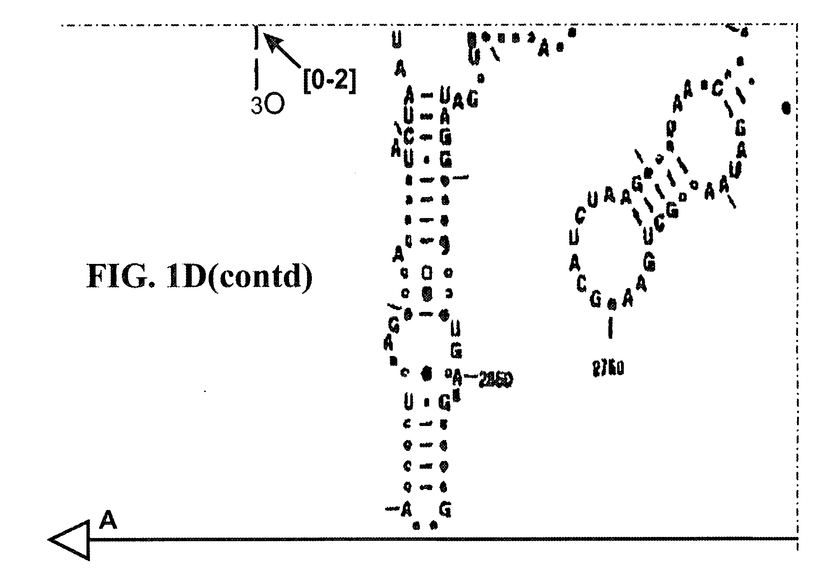

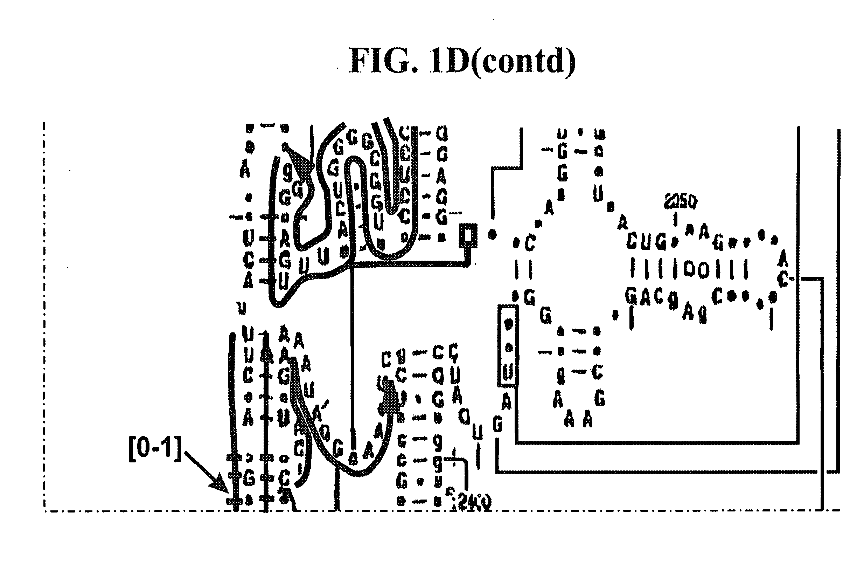

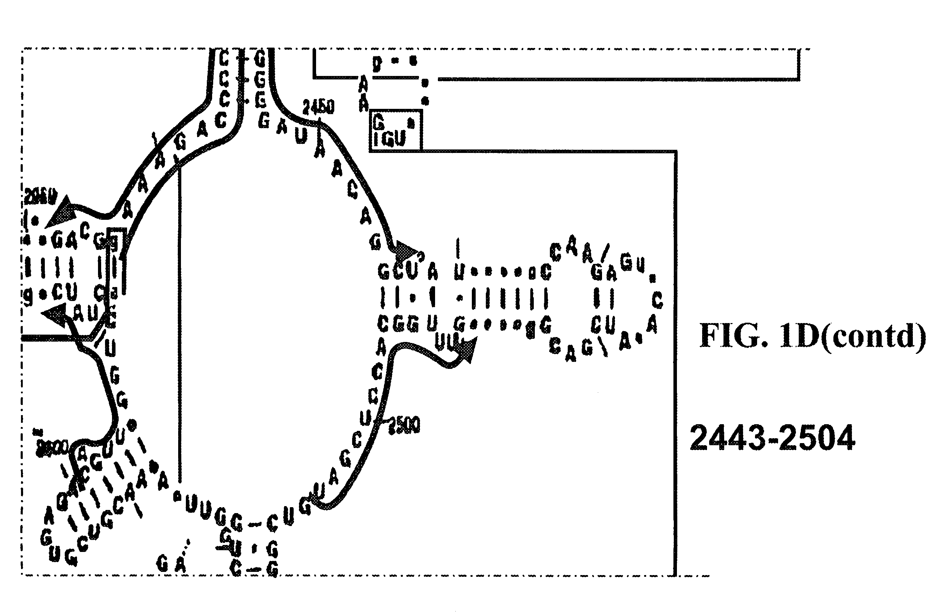

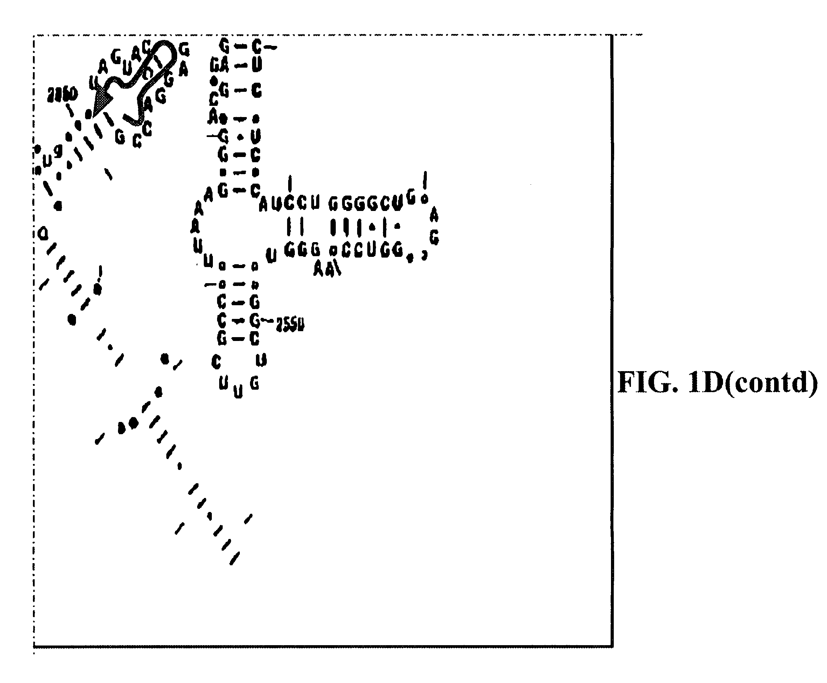

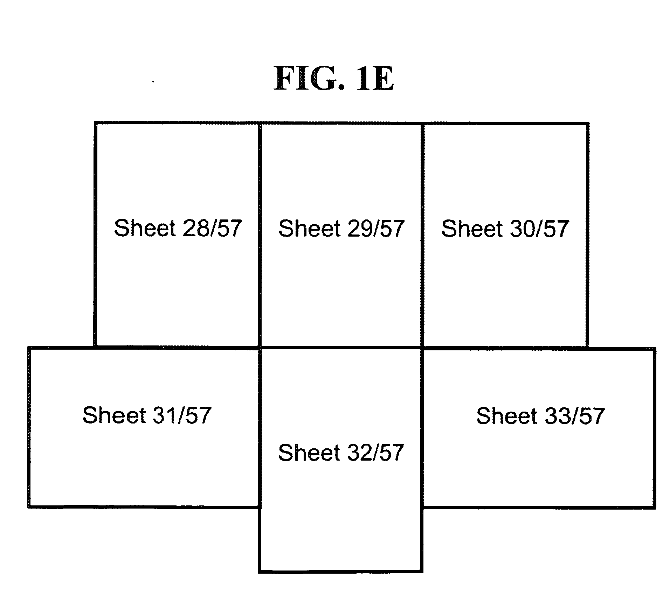

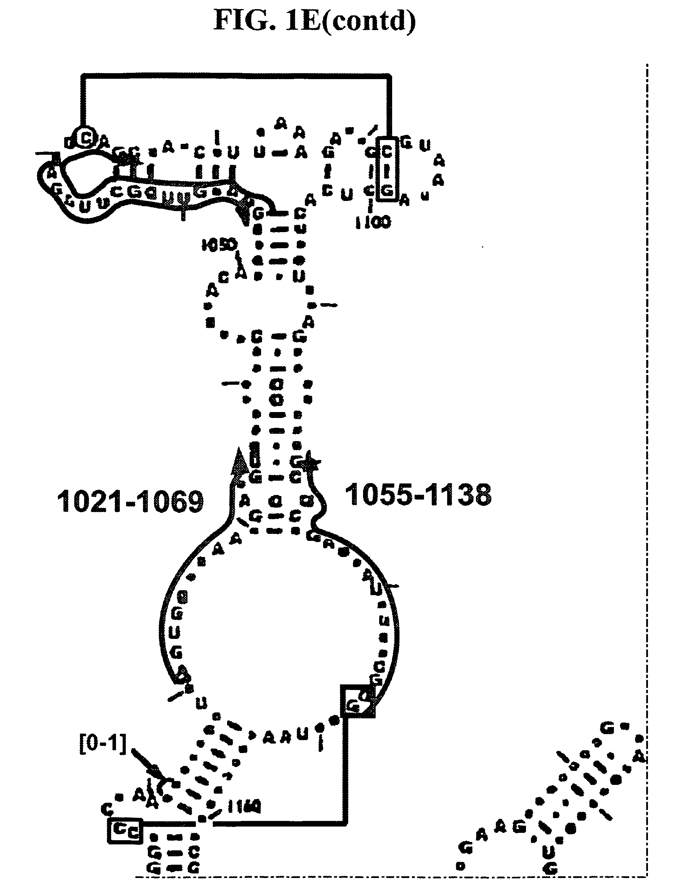

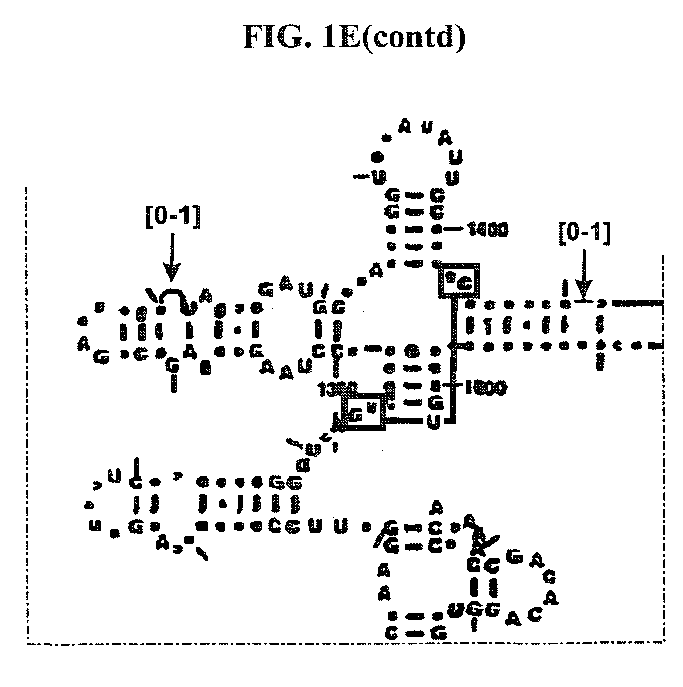

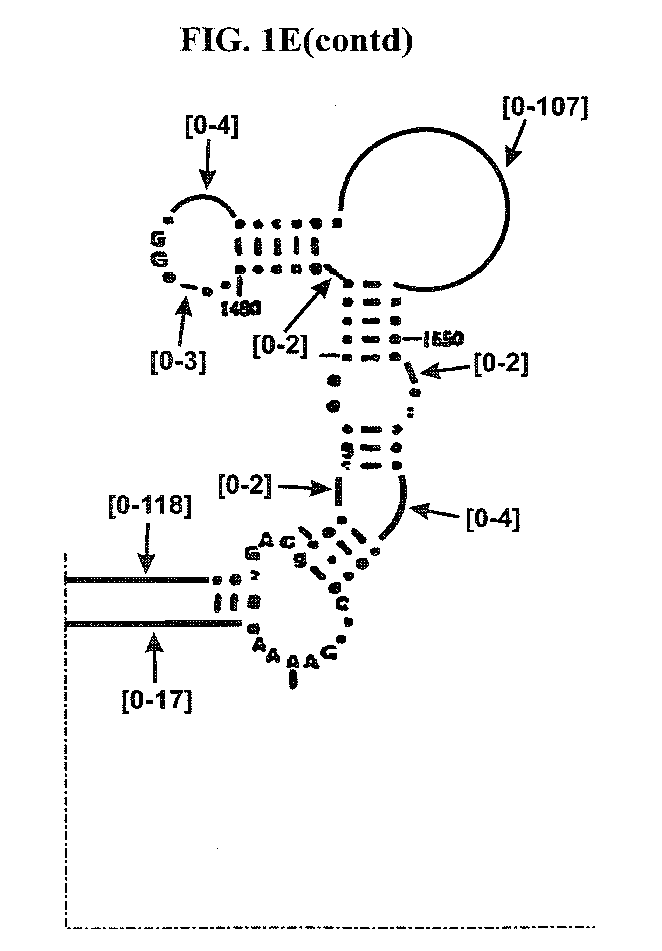

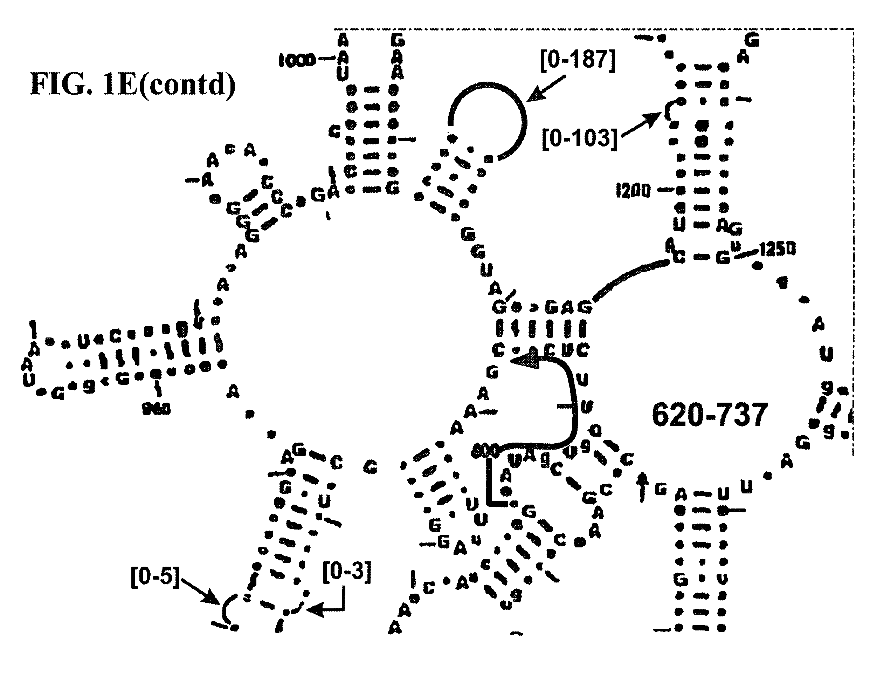

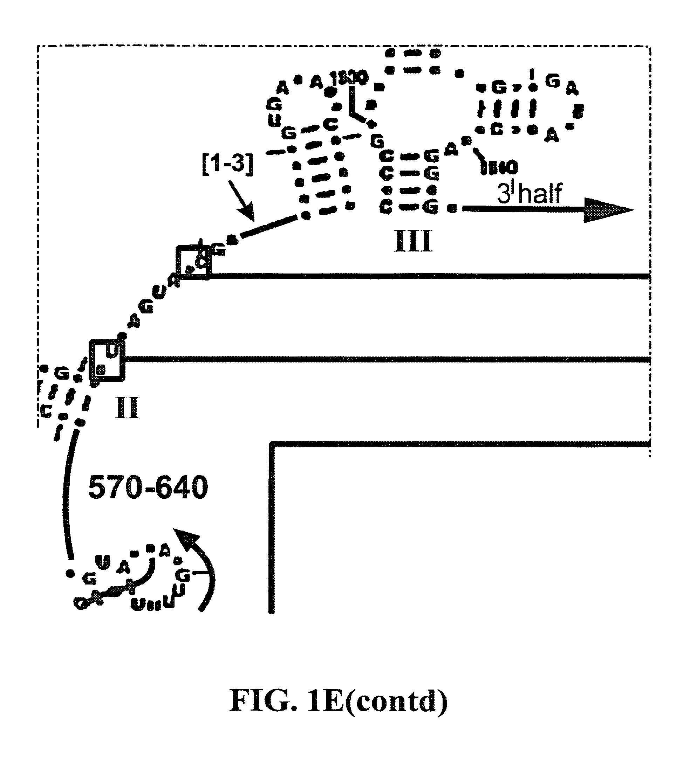

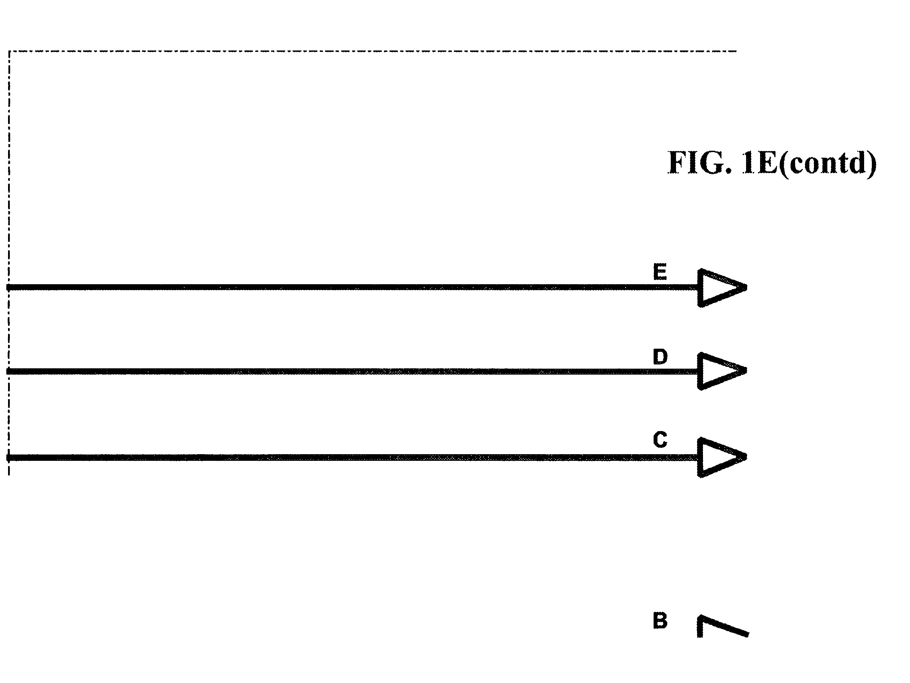

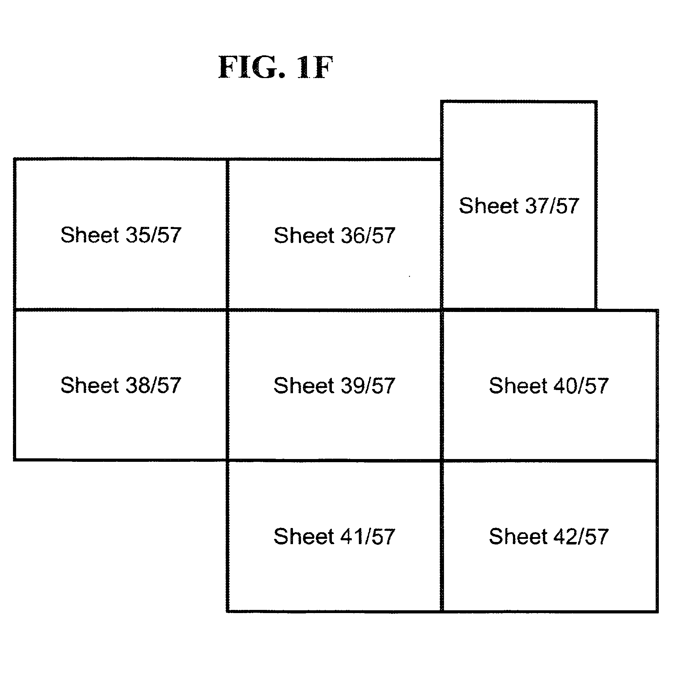

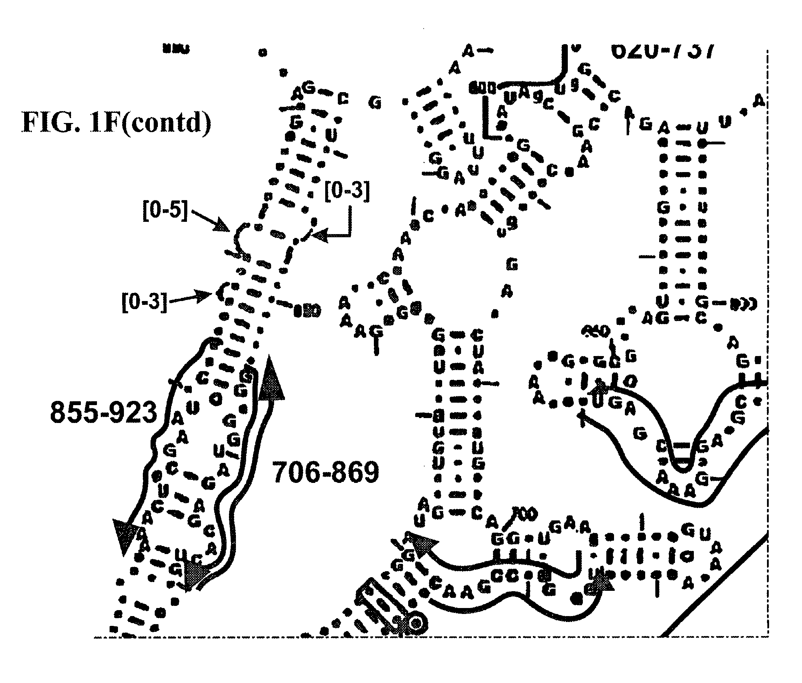

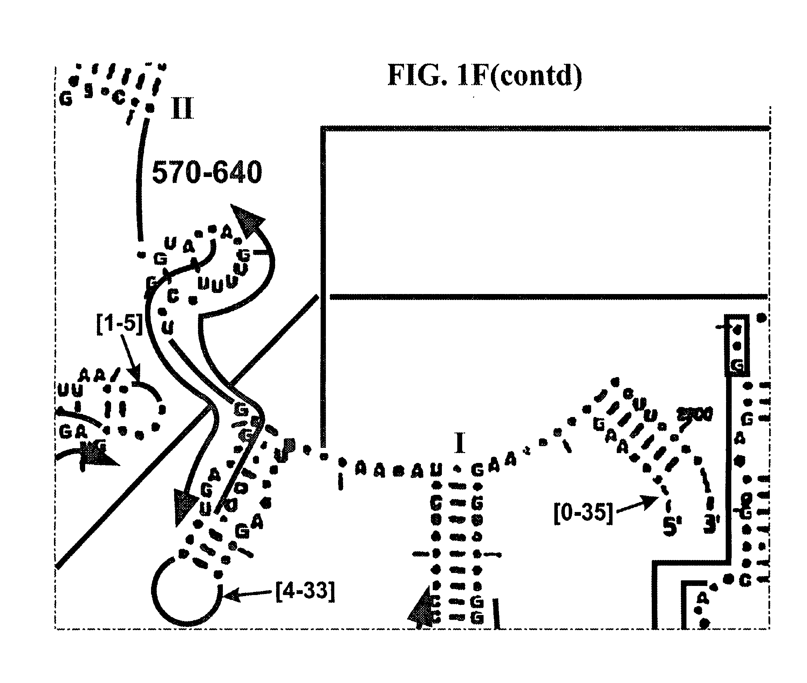

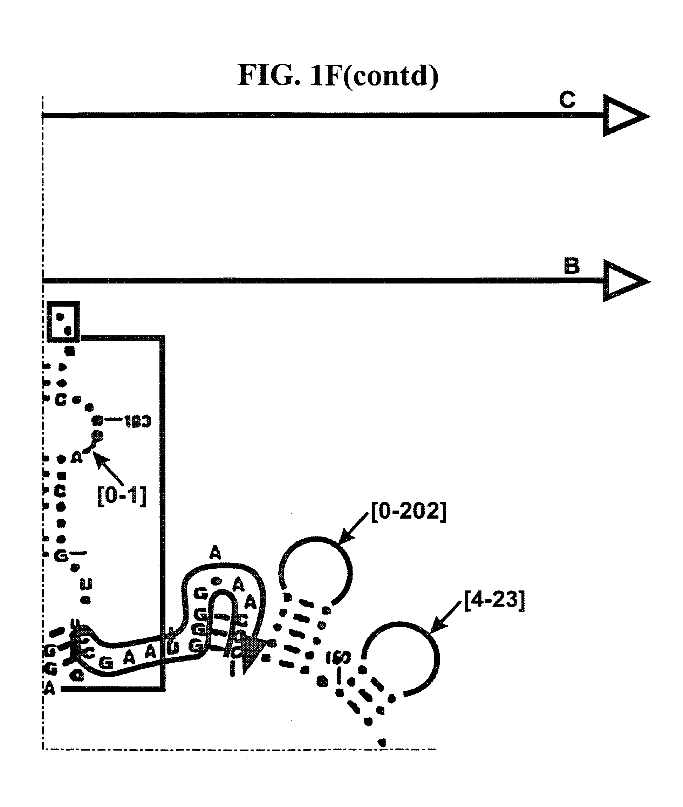

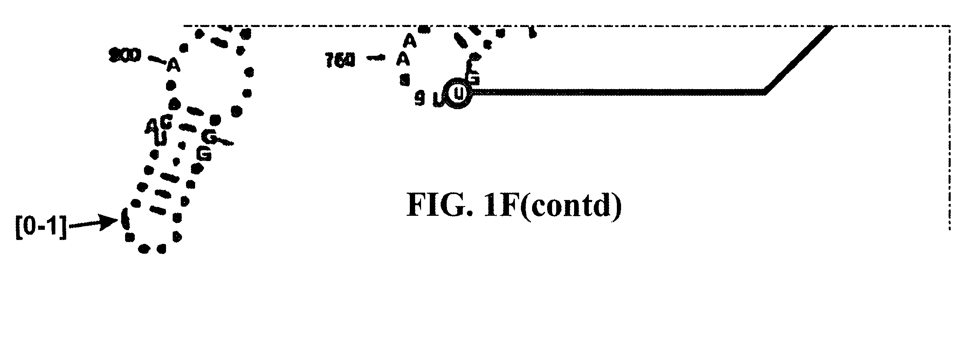

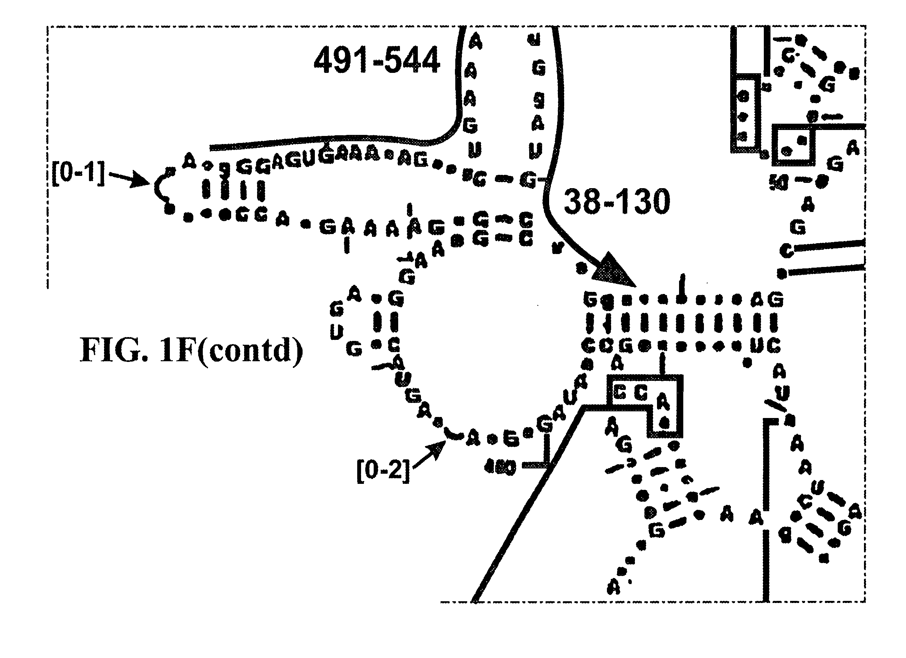

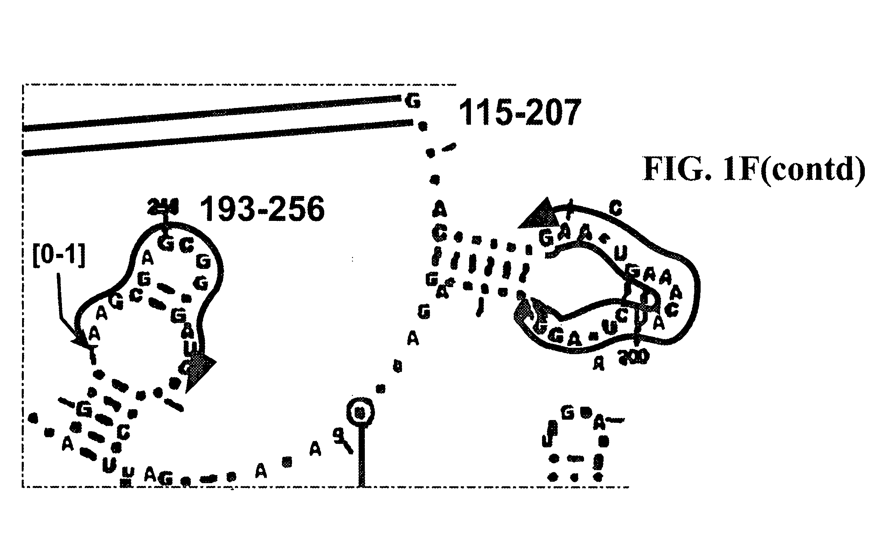

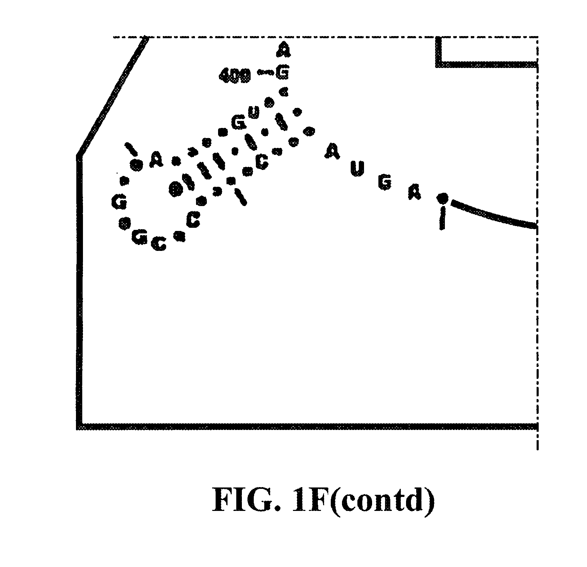

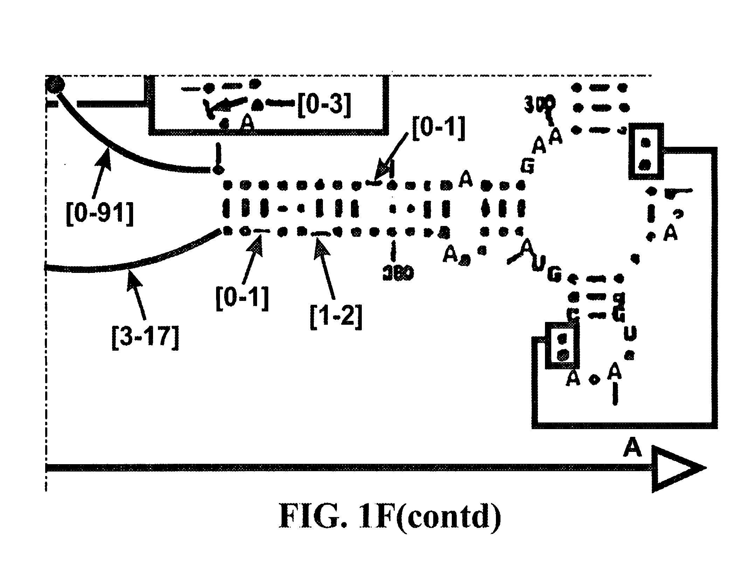

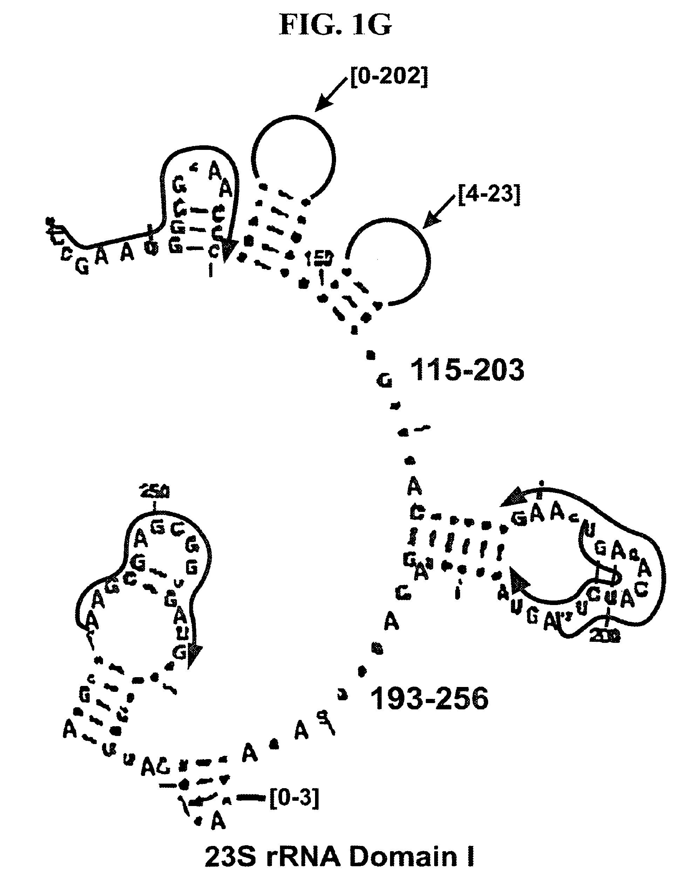

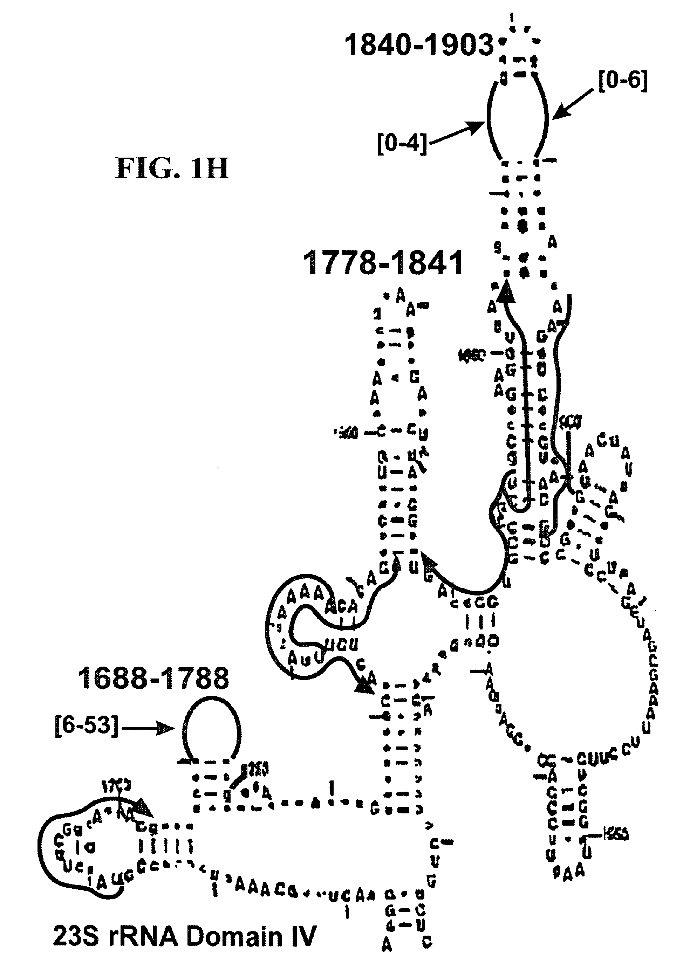

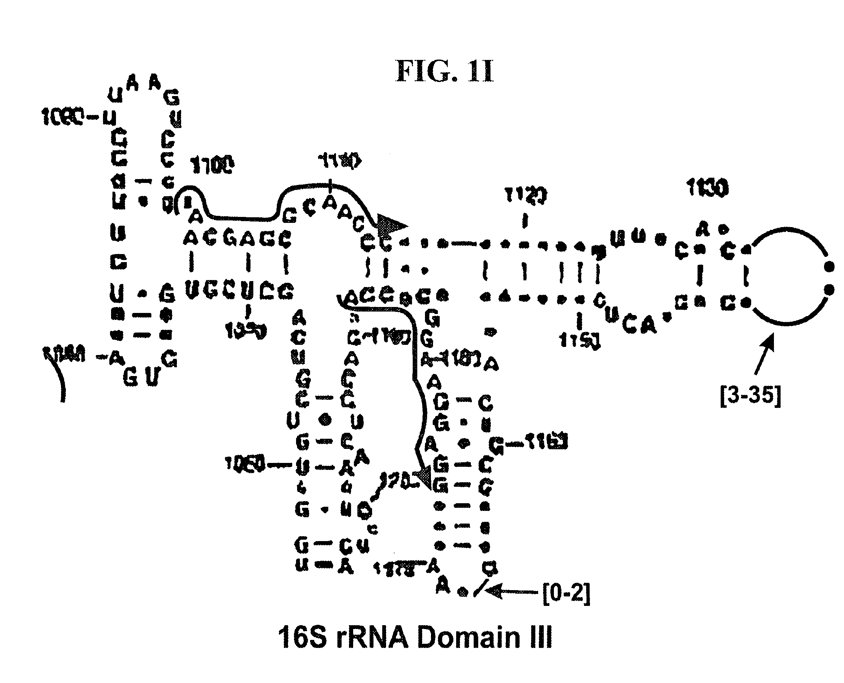

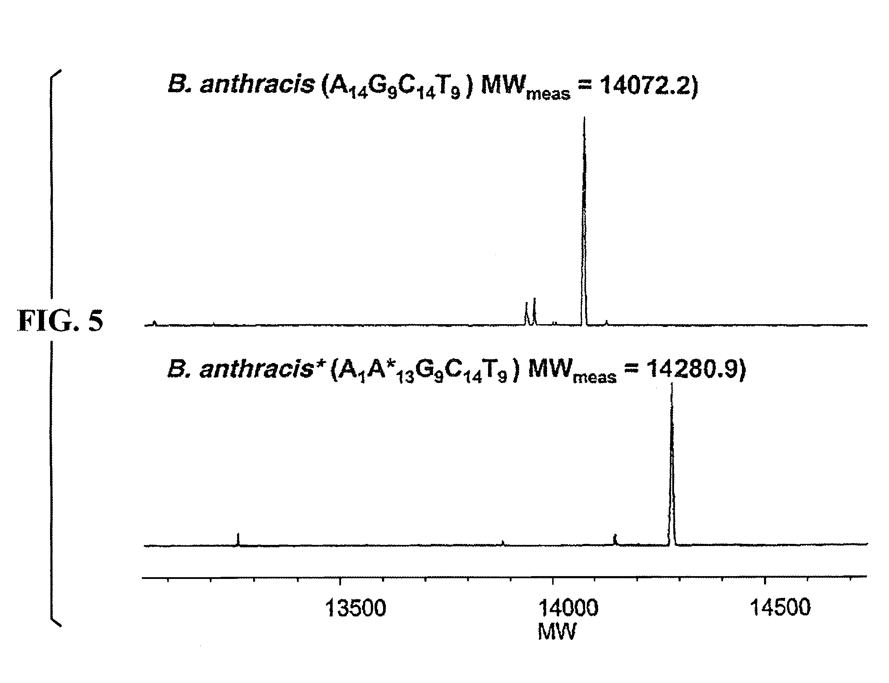

Figures 1A-1I are consensus diagrams that show examples of conserved regions from 16S rRNA (Fig. 1A-1B)(SEQ ID NO:3), 23S rRNA (3'-half, Fig. 1C-1D; 5'-half, Fig. 1E-F)(SEQ ID NO:4), 23S rRNA Domain I (Fig. 1G), 23S rRNA Domain IV (Fig. 1H) and 16S rRNA Domain III (Fig. 1I) which are suitable for use in the present invention. Lines with arrows are examples of regions to which intelligent primer pairs for PCR are designed. The label for each primer pair represents the starting and ending base number of the amplified region on the consensus diagram. Bases in capital letters are greater than 95% conserved; bases in lower case letters are 90-95% conserved, filled circles are 80-90% conserved; and open circles are less than 80% conserved. The label for each primer pair represents the starting and ending base number of the amplified region on the consensus diagram.
Figure 2 shows a typical primer amplified region from the 16S rRNA Domain III shown in Figure 1C.
Figure 3 is a schematic diagram showing conserved regions in RNase P. Bases in capital letters are greater than 90% conserved; bases in lower case letters are 80-90% conserved; filled circles designate bases which are 70-80% conserved; and open circles designate bases that are less than 70% conserved.
Figure 4 is a schematic diagram of base composition signature determination using nucleotide analog "tags" to determine base composition signatures.
Figure 5 shows the deconvoluted mass spectra of a *Bacillus anthracis* region with and without the mass tag phosphorothioate A (A*). The two spectra differ in that the measured molecular weight of the mass tag-containing sequence is greater than the unmodified sequence.
Figure 6 shows base composition signature (BCS) spectra from PCR products from *Staphylococcus aureus* (S. aureus 16S_1337F) and *Bacillus anthracus* (B. anthr. 16S_1337F), amplified using the same primers. The two strands differ by only two (AT->CG) substitutions and are clearly distinguished on the basis of their BCS.
Figure 7 shows that a single difference between two sequences (A₁₄ in *B. anthracis* vs. A₁₅ in *B. cereus*) can be easily detected using ESI-TOF mass spectrometry.
Figure 8 is an ESI-TOF of *Bacillus anthracis* spore coat protein sspE 56mer plus calibrant. The signals unambiguously identify *B. anthracis* versus other Bacillus species.
Figure 9 is an ESI-TOF of a *B. anthracis* synthetic 16S_1228 duplex (reverse and forward strands). The technique easily distinguishes between the forward and reverse strands.
Figure 10 is an ESI-FTICR-MS of a synthetic *B. anthracis* 16S_1337 46 base pair duplex.
Figure 11 is an ESI-TOF-MS of a 56mer oligonucleotide (3 scans) from the *B. anthracis* saspB gene with an internal mass standard. The internal mass standards are designated by asterisks.
Figure 12 is an ESI-TOF-MS of an internal standard with 5 mM TBA-TFA buffer showing that charge stripping with tributylammonium trifluoroacetate reduces the most abundant charge state from [M-8H⁺]⁸⁻ to [M-3H⁺]³⁻.

### DETAILED DESCRIPTION OF THE INVENTION

The present disclosure provides a combination of a non-PCR biomass detection mode, preferably high-resolution MS, with PCR-based BCS technology using "intelligent primers" which hybridize to conserved sequence regions of nucleic acids derived from a bioagent and which bracket variable sequence regions that uniquely identify the bioagent. The high-resolution MS technique is used to determine the molecular mass and base composition signature (BCS) of the amplified sequence region. This unique "base composition signature" (BCS) is then input to a maximum-likelihood detection algorithm for matching against a database of base composition signatures in the same amplified region. The present method combines PCR-based amplification technology (which provides specificity) and a molecular mass detection mode (which provides speed and does not require nucleic acid sequencing of the amplified target sequence) for bioagent detection and identification.

The present method allows extremely rapid and accurate detection and identification of bioagents compared to existing methods. Furthermore, this rapid detection and identification is possible even when sample material is impure. Thus, the method is useful in a wide variety of fields, including, but not limited to, environmental testing (e.g., detection and discrimination of pathogenic vs. non-pathogenic bacteria in water or other samples), germ warfare (allowing immediate identification of the bioagent and appropriate treatment), pharmacogenetic analysis and medical diagnosis (including cancer diagnosis based on mutations and polymorphisms; drug resistance and susceptibility testing; screening for and/or diagnosis of genetic diseases and conditions; and diagnosis of infectious diseases and conditions). The method leverages ongoing biomedical research in virulence, pathogenicity, drug resistance and genome sequencing into a method which provides greatly improved sensitivity, specificity and reliability compared to existing methods, with lower rates of false positives.

The present method can be used to detect and classify any biological agent, including bacteria, viruses, fungi and toxins. As one example, where the agent is a biological threat, the information obtained is used to determine practical information needed for countermeasures, including toxin genes, pathogenicity islands and antibiotic resistance genes. In addition, the methods can be used to identify natural or deliberate engineering events including chromosome fragment swapping, molecular breeding (gene shuffling) and emerging infectious diseases.

Bacteria have a common set of absolutely required genes. About 250 genes are present in all bacterial species (Proc. Natl. Acad. Sci. U.S.A. 93:10268, 1996; Science 270:397, 1995), including tiny genomes like *Mycoplasma*, *Ureaplasma* and *Rickettsia*. These genes encode proteins involved in translation, replication, recombination and repair, transcription, nucleotide metabolism, amino acid metabolism, lipid metabolism, energy generation, uptake, secretion and the like. Examples of these proteins are DNA polymerase III beta, elongation factor TU, heat shock protein groEL, RNA polymerase beta, phosphoglycerate kinase, NADH dehydrogenase, DNA ligase, DNA topoisomerase and elongation factor G. Operons can also be targeted using the present method. One example of an operon is the bfp operon from enteropathogenic *E.* coli. Multiple core chromosomal genes can be used to classify bacteria at a genus or genus species level to determine if an organism has threat potential. The method can also be used to detect pathogenicity markers (plasmid or chromosomal) and antibiotic resistance genes to confirm the threat potential of an organism and to direct countermeasures.

A theoretically ideal bioagent detector would identify, quantify, and report the complete nucleic acid sequence of every bioagent that reached the sensor. The complete sequence of the nucleic acid component of a pathogen would provide all relevant information about the threat, including its identity and the presence of drug-resistance or pathogenicity markers. This ideal has not yet been achieved. However, the present invention provides a straightforward strategy for obtaining information with the same practical value using base composition signatures (BCS). While the base composition of a gene fragment is not as information-rich as the sequence itself, there is no need to analyze the complete sequence of the gene if the short analyte sequence fragment is properly chosen. A database of reference sequences can be prepared in which each sequence is indexed to a unique base composition signature, so that the presence of the sequence can be inferred with accuracy from the presence of the signature. The advantage of base composition signatures is that they can be quantitatively measured in a massively parallel fashion using multiplex PCR (PCR in which two or more primer pairs amplify target sequences simultaneously) and mass spectrometry. These multiple primer amplified regions uniquely identify most threat and ubiquitous background bacteria and viruses. In addition, cluster-specific primer pairs distinguish important local clusters (e.g., anthracis group).

In the context of this invention, a "bioagent" is any organism, living or dead, or a nucleic acid derived from such an organism. Examples of bioagents include but are not limited to cells (including but not limited to human clinical samples, bacterial cells and other pathogens) viruses, toxin genes and bioregulating compounds). Samples may be alive or dead or in a vegetative state (for example, vegetative bacteria or spores) and may be encapsulated or bioengineered.

As used herein, a "base composition signature" (BCS) is the exact base composition from selected fragments of nucleic acid sequences that uniquely identifies the target gene and source organism. BCS can be thought of as unique indexes of specific genes.

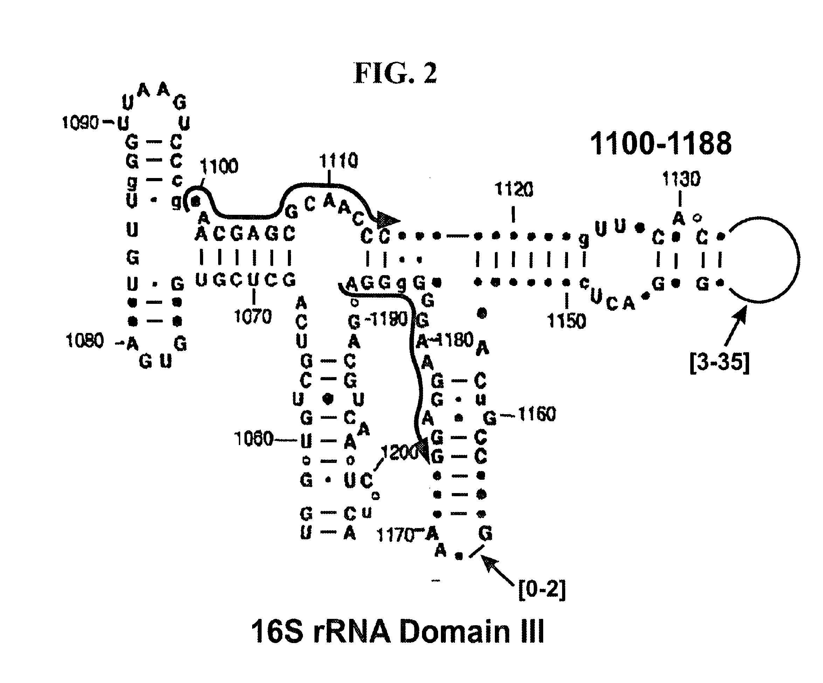

As used herein, "intelligent primers" are primers which bind to sequence regions which flank an intervening variable region. In a preferred embodiment, these sequence regions which flank the variable region are highly conserved among different species of bioagent. For example, the sequence regions may be highly conserved among all Bacillus species. By the term "highly conserved", it is meant that the sequence regions exhibit between about 80-100%, more preferably between about 90-100% and most preferably between about 95-100% identity. Examples of intelligent primers which amplify regions of the 16S and 23S rRNA are shown in Figures 1A-1I. A typical primer amplified region in 16S rRNA is shown in Figure 2. The arrows represent primers which bind to highly conserved regions which flank a variable region in 16S rRNA domain III. The amplified region is the stem-loop structure under "1100-1188."

As used herein, "match" refers to any statistically significant probabilistic determination or result.

One main advantage of the detection methods of the present disclosure is that the primers need not be specific for a particular bacterial species, or even genus, such as *Bacillus* or *Streptomyces*. Instead, the primers recognize highly conserved regions across hundreds of bacterial species including, but not limited to, the species described herein. Thus, the same primer pair can be used to identify any desired bacterium because it will bind to the conserved regions which flank a variable region specific to a single species, or common to several bacterial species, allowing nucleic acid amplification of the intervening sequence and determination of its molecular weight and base composition. For example, the 16S_971-1062, 16S_1228-1310 and 16S_1100-1188 regions are 98-99% conserved in about 900 species of bacteria (16S=16S rRNA, numbers indicate nucleotide position). In one embodiment of the present invention, primers used in the present method bind to one or more of these regions or portions thereof.

The present disclosure provides a combination of a non-PCR biomass detection mode, preferably high-resolution MS, with nucleic acid amplification-based BCS technology using "intelligent primers" which hybridize to conserved regions and which bracket variable regions that uniquely identify the bioagent(s). Although the use of PCR is preferred, other nucleic acid amplification techniques may also be used, including ligase chain reaction (LCR) and strand displacement amplification (SDA). The high-resolution MS technique allows separation of bioagent spectral lines from background spectral lines in highly cluttered environments. The resolved spectral lines are then translated to BCS which are input to a maximum-likelihood detection algorithm matched against spectra for one or more known BCS. Preferably, the bioagent BCS spectrum is matched against one or more databases of BCS from vast numbers of bioagents. Preferably, the matching is done using a maximum-likelihood detection algorithm.

In a preferred embodiment, base composition signatures are quantitatively measured in a massively parallel fashion using the polymerase chain reaction (PCR), preferably multiplex PCR, and mass spectrometric (MS) methods. Sufficient quantities of nucleic acids must be present for detection of bioagents by MS. A wide variety of techniques for preparing large amounts of purified nucleic acids or fragments thereof are well known to those of skill in the art. PCR requires one or more pairs of oligonucleotide primers which bind to regions which flank the target sequence(s) to be amplified. These primers prime synthesis of a different strand of DNA, with synthesis occurring in the direction of one primer towards the other primer. The primers, DNA to be amplified, a thermostable DNA polymerase (e.g. *Taq* polymerase), the four deoxynucleotide triphosphates, and a buffer are combined to initiate DNA synthesis. The solution is denatured by heating, then cooled to allow annealing of newly added primer, followed by another round of DNA synthesis. This process is typically repeated for about 30 cycles, resulting in amplification of the target sequence.

The "intelligent primers" define the target sequence region to be amplified and analyzed. In one embodiment, the target sequence is a ribosomal RNA (rRNA) gene sequence. With the complete sequences of many of the smallest microbial genomes now available, it is possible to identify a set of genes that defines "minimal life" and identify composition signatures that uniquely identify each gene and organism. Genes that encode core life functions such as DNA replication, transcription, ribosome structure, translation, and transport are distributed broadly in the bacterial genome and are preferred regions for BCS analysis. Ribosomal RNA (rRNA) genes comprise regions that provide useful base composition signatures. Like many genes involved in core life functions, rRNA genes contain sequences that are extraordinarily conserved across bacterial domains interspersed with regions of high variability that are more specific to each species. The variable regions can be utilized to build a database of base composition signatures. The strategy involves creating a structure-based alignment of sequences of the small (16S) and the large (23S) subunits of the rRNA genes. For example, there are currently over 13,000 sequences in the ribosomal RNA database that has been created and maintained by Robin Gutell, University of Texas at Austin, and is publicly available on the Institute for Cellular and Molecular Biology web page (www.rna.icmb.utexas.edu/). There is also a publicly available rRNA database created and maintained by the University of Antwerp, Belgium web page (www.rrna.uia.ac.be).

These databases have been analyzed to determine regions that are useful as base composition signatures. The characteristics of such regions are: a) between about 80 and 100%, preferably > about 95% identity among species of the particular bioagent of interest, of upstream and downstream nucleotide sequences which serve as sequence amplification primer sites; b) an intervening variable region which exhibits no greater than about 5% identity among species; and c) a separation of between about 30 and 1000 nucleotides, preferably no more than about 50-250 nucleotides, and more preferably no more than about 60-100 nucleotides, between the conserved regions.

Due to their overall conservation, the flanking rRNA primer sequences serve as good "universal" primer binding sites to amplify the region of interest for most, if not all, bacterial species. The intervening region between the sets of primers varies in length and/or composition, and thus provides a unique base composition signature.

It is advantageous to design the "intelligent primers" to be as universal as possible to minimize the number of primers which need to be synthesized, and to allow detection of multiple species using a single pair of primers. These primer pairs can be used to amplify variable regions in these species. Because any variation (due to codon wobble in the 3^{rd} position) in these conserved regions among species is likely to occur in the third position of a DNA triplet, oligonucleotide primers can be designed such that the nucleotide corresponding to this position is a base which can bind to more than one nucleotide, referred to herein as a "universal base". For example, under this "wobble" pairing, inosine (I) binds to U, C or A; guanine (G) binds to U or C, and uridine (U) binds to U or C. Other examples of universal bases include nitroindoles such as 5-nitroindole or 3-nitropyrrole (Loakes et al., Nucleosides and Nucleotides 14:1001-1003, 1995), the degenerate nucleotides d**P** or d**K** (Hill et al.), an acyclic nucleoside analog containing 5-nitroindazole (Van Aerschot et al., Nucleosides and Nucleotides 14:1053-1056, 1995) or the purine analog 1-(2-deoxy-β-D-ribofuranosyl)-imidazole-4-carboxamide (Sala et al., Nucl. Acids Res. 24:3302-3306, 1996).

In another embodiment of the invention, to compensate for the somewhat weaker binding by the "wobble" base, the oligonucleotide primers are designed such that the first and second positions of each triplet are occupied by nucleotide analogs which bind with greater affinity than the unmodified nucleotide. Examples of these analogs are 2,6-diaminopurine which binds to thymine, propyne T which binds to adenine and propyne C and phenoxazines, including G-clamp, which binds to G. Propynes are described in U.S. Patent Nos. 5,645,985, 5,830.653 and 5,484,908. Phenoxazines are described in U.S. Patent Nos. 5,502,177, 5,763,588, and 6,005,096. G-clamps are described in U.S. Patent Nos. 6,007,992 and 6,028,183.

Bacterial biological warfare agents capable of being detected by the present methods include *Bacillus anthracis* (anthrax), *Yersinia pestis* (pneumonic plague), *Franciscella tularensis* (tularemia), *Brucella suis*, *Brucella abortus*, *Brucella melitensis* (undulant fever), *Burkholderia mallei* (glanders), *Burkholderia pseudomalleii* (melioidosis), *Salmonella typhi* (typhoid fever), *Rickettsia typhii* (epidemic typhus), *Rickettsia prowasekii* (endemic typhus) and *Coxiella burnetii* (Q fever), *Rhodobacter capsulatus*, *Chlamydia pneumoniae*, *Escherichia coli, Shigella dysenteriae, Shigella flexneri*, *Bacillus cereus, Clostridium botulinum*, *Coxiella burnetti*, *Pseudomonas aeruginosa*, *Legionella pneumophila*, and *Vibrio cholerae*.

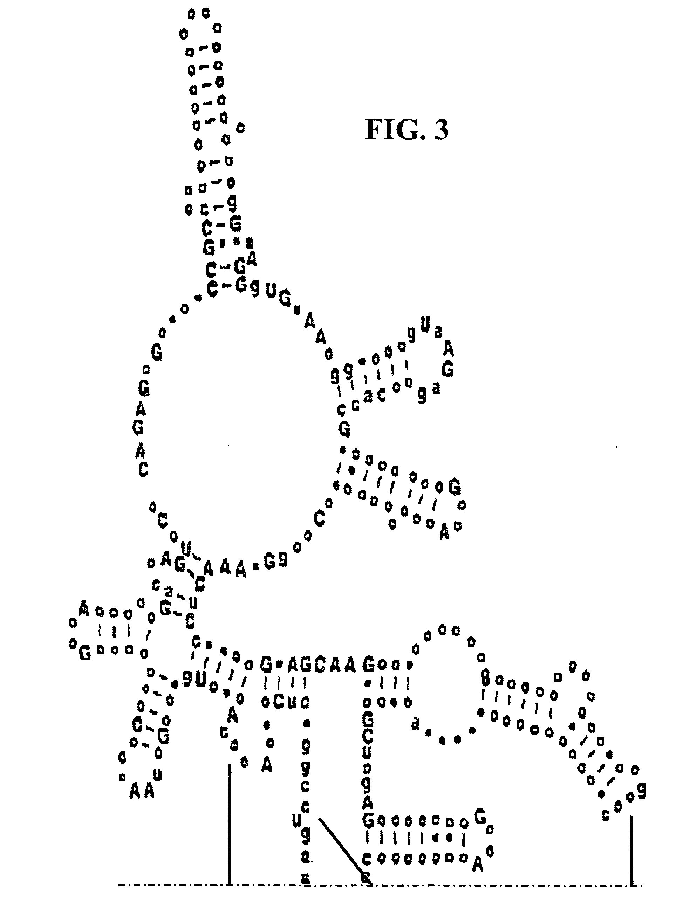

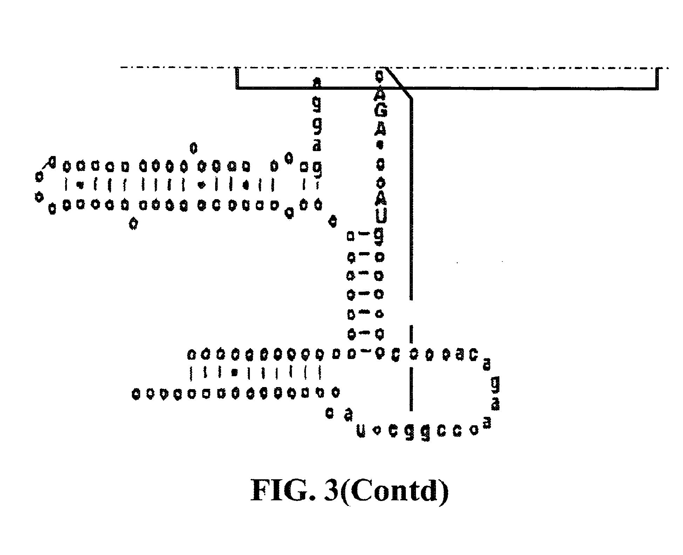

Besides 16S and 23S rRNA, other target regions suitable for use in the present disclosure for detection of bacteria include 5S rRNA and RNase P (Figure 3).

Biological warfare fungus biowarfare agents include *coccidioides immitis* (Coccidioidomycosis).

Biological warfare toxin genes capable of being detected by the methods of the present disclosure include botulism, T-2 mycotoxins, ricin, staph enterotoxin B, shigatoxin, abrin, aflatoxin, *Clostridium perfringens* epsilon toxin, conotoxins, diacetoxyscirpenol, tetrodotoxin and saxitoxin.

Biological warfare viral threat agents are mostly RNA viruses (positive-strand and negative-strand), with the exception of smallpox. Every RNA virus is a family of related viruses (quasispecies). These viruses mutate rapidly and the potential for engineered strains (natural or deliberate) is very high. RNA viruses cluster into families that have conserved RNA structural domains on the viral genome (e.g., virion components, accessory proteins) and conserved housekeeping genes that encode core viral proteins including, for single strand positive strand RNA viruses, RNA-dependent RNA polymerase, double stranded RNA helicase, chymotrypsin-like and papain-like proteases and methyltransferases.

Examples of (-)-strand RNA viruses include arenaviruses (e.g., sabia virus, lassa fever, Machupo, Argentine hemorrhagic fever, flexal virus), bunyaviruses (e.g., hantavirus, nairovirus, phlebovirus, hantaan virus, Congo-crimean hemorrhagic fever, rift valley fever), and mononegavirales (e.g., filovirus, paramyxovirus, ebola virus, Marburg, equine morbillivirus).

Examples of (+)-strand RNA viruses include picomaviruses (e.g., coxsackievirus, echovirus, human coxsackievirus A, human echovirus, human enterovirus, human poliovirus, hepatitis A virus, human parechovirus, human rhinovirus), astroviruses (e.g., human astrovirus), calciviruses (e.g., chiba virus, chitta virus, human calcivirus, norwalk virus), nidovirales (e.g., human coronavirus, human torovirus), flaviviruses (e.g., dengue virus 1-4, Japanese encephalitis virus, Kyanasur forest disease virus, Murray Valley encephalitis virus, Rocio virus, St. Louis encephalitis virus, West Nile virus, yellow fever virus, hepatitis c virus) and togaviruses (e.g., Chikugunya virus, Eastern equine encephalitis virus, Mayaro virus, O'nyong-nyong virus, Ross River virus, Venezuelan equine encephalitis virus, Rubella virus, hepatitis E virus). The hepatitis C virus has a 5'-untranslated region of 340 nucleotides, an open reading frame encoding 9 proteins having 3010 amino acids and a 3'-untranslated region of 240 nucleotides. The 5'-UTR and 3'-UTR are 99% conserved in hepatitis C viruses.

In one embodiment, the target gene is an RNA-dependent RNA polymerase or a helicase encoded by (+)-strand RNA viruses, or RNA polymerase from a (-)-strand RNA virus. (+)-strand RNA viruses are double stranded RNA and replicate by RNA-directed RNA synthesis using RNA-dependent RNA polymerase and the positive strand as a template. Helicase unwinds the RNA duplex to allow replication of the single stranded RNA. These viruses include viruses from the family picornaviridae (e.g., poliovirus, coxsackievirus, echovirus), togaviridae (e.g., alphavirus, flavivirus, rubivirus), arenaviridae (e.g., lymphocytic choriomeningitis virus, lassa fever virus), cononaviridae (e.g., human respiratory virus) and Hepatitis A virus. The genes encoding these proteins comprise variable and highly conserved regions which flank the variable regions.

In a preferred embodiment, the detection scheme for the PCR products generated from the bioagent(s) incorporates three features. First, the technique simultaneously detects and differentiates multiple (generally about 6-10) PCR products. Second, the technique provides a BCS that uniquely identifies the bioagent from the possible primer sites. Finally, the detection technique is rapid, allowing multiple PCR reactions to be run in parallel.

In one embodiment, the method can be used to detect the presence of antibiotic resistance and/or toxin genes in a bacterial species. For example, *Bacillus anthracis* comprising a tetracycline resistance plasmid and plasmids encoding one or both anthracis toxins (px01 and/or px02) can be detected by using antibiotic resistance primer sets and toxin gene primer sets. If the *B. anthracis* is positive for tetracycline resistance, then a different antibiotic, for example quinalone, is used.

Mass spectrometry (MS)-based detection of PCR products provides all of these features with additional advantages. MS is intrinsically a parallel detection scheme without the need for radioactive or fluorescent labels, since every amplification product with a unique base composition is identified by its molecular mass. The current state of the art in mass spectrometry is such that less than femtomole quantities of material can be readily analyzed to afford information about the molecular contents of the sample. An accurate assessment of the molecular mass of the material can be quickly obtained, irrespective of whether the molecular weight of the sample is several hundred, or in excess of one hundred thousand atomic mass units (amu) or Daltons. Intact molecular ions can be generated from amplification products using one of a variety of ionization techniques to convert the sample to gas phase. These ionization methods include, but are not limited to, electrospray ionization (ES), matrix-assisted laser desorption ionization (MALDI) and fast atom bombardment (FAB). For example, MALDI of nucleic acids, along with examples of matrices for use in MALDI of nucleic acids, are described in WO 98/54751 (Genetrace, Inc.).

Upon ionization, several peaks are observed from one sample due to the formation of ions with different charges. Averaging the multiple readings of molecular mass obtained from a single mass spectrum affords an estimate of molecular mass of the bioagent. Electrospray ionization mass spectrometry (ESI-MS) is particularly useful for very high molecular weight polymers such as proteins and nucleic acids having molecular weights greater than 10 kDa, since it yields a distribution of multiply-charged molecules of the sample without causing a significant amount of fragmentation.

The mass detectors used in the methods of the present disclosure include, but are not limited to, Fourier transform ion cyclotron resonance mass spectrometry (FT-ICR-MS), ion trap, quadrupole, magnetic sector, time of flight (TOF), Q-TOF, and triple quadrupole.

In general, the mass spectrometric techniques which can be used in the present disclosure include, but are not limited to, tandem mass spectrometry, infrared multiphoton dissociation and pyrolytic gas chromatography mass spectrometry (PGC-MS). In one embodiment of the invention, the bioagent detection system operates continually in bioagent detection mode using pyrolytic GC-MS without PCR for rapid detection of increases in biomass (for example, increases in fecal contamination of drinking water or of germ warfare agents). To achieve minimal latency, a continuous sample stream flows directly into the PGC-MS combustion chamber. When an increase in biomass is detected, a PCR process is automatically initiated. Bioagent presence produces elevated levels of large molecular fragments from 100-7,000 Da which are observed in the PGC-MS spectrum. The observed mass spectrum is compared to a threshold level and when levels of biomass are determined to exceed a predetermined threshold, the bioagent classification process described hereinabove(combining PCR and MS, preferably FT-ICR MS) is initiated. Optionally, alarms or other processes (halting ventilation flow, physical isolation) are also initiated by this detected biomass level.

The accurate measurement of molecular mass for large DNAs is limited by the adduction of cations from the PCR reaction to each strand, resolution of the isotopic peaks from natural abundance ¹³C and ¹⁵N isotopes, and assignment of the charge state for any ion. The cations are removed by in-line dialysis using a flow-through chip that brings the solution containing the PCR products into contact with a solution containing ammonium acetate in the presence of an electric field gradient orthogonal to the flow. The latter two problems are addressed by operating with a resolving power of >100,000 and by incorporating isotopically depleted nucleotide triphosphates into the DNA. The resolving power of the instrument is also a consideration. At a resolving power of 10,000, the modeled signal from the [M-14H+]¹⁴⁻ charge state of an 84mer PCR product is poorly characterized and assignment of the charge state or exact mass is impossible. At a resolving power of 33,000, the peaks from the individual isotopic components are visible. At a resolving power of 100,000, the isotopic peaks are resolved to the baseline and assignment of the charge state for the ion is straightforward. The [¹³C,¹⁵N]-depleted triphosphates are obtained, for example, by growing microorganisms on depleted media and harvesting the nucleotides (Batey et al., Nucl. Acids Res. 20:4515-4523, 1992).

While mass measurements of intact nucleic acid regions are believed to be adequate to determine most bioagents, tandem mass spectrometry (MSⁿ) techniques may provide more definitive information pertaining to molecular identity or sequence. Tandem MS involves the coupled use of two or more stages of mass analysis where both the separation and detection steps are based on mass spectrometry. The first stage is used to select an ion or component of a sample from which further structural information is to be obtained. The selected ion is then fragmented using, e.g., blackbody irradiation, infrared multiphoton dissociation, or collisional activation. For example, ions generated by electrospray ionization (ESI) can be fragmented using IR multiphoton dissociation. This activation leads to dissociation of glycosidic bonds and the phosphate backbone, producing two series of fragment ions, called the w-series (having an intact 3'terminus and a 5' phosphate following internal cleavage) and the a-Base series(having an intact 5'terminus and a 3' furan).

The second stage of mass analysis is then used to detect and measure the mass of these resulting fragments of product ions. Such ion selection followed by fragmentation routines can be performed multiple times so as to essentially completely dissect the molecular sequence of a sample.

If there are two or more targets of similar base composition or mass, or if a single amplification reaction results in a product which has the same mass as two or more bioagent reference standards, they can be distinguished by using mass-modifying "tags." In this embodiment of the invention, a nucleotide analog or "tag" is incorporated during amplification (e.g:, a 5-(trifluoromethyl) deoxythymidine triphosphate) which has a different molecular weight than the unmodified base so as to improve distinction of masses. Such tags are described in, for example, PCT WO97/33000. This further limits the number of possible base compositions consistent with any mass. For example, 5-(trifluoromethyl)deoxythymidine triphosphate can be used in place of dTTP in a separate nucleic acid amplification reaction. Measurement of the mass shift between a conventional amplification product and the tagged product is used to quantitate the number of thymidine nucleotides in each of the single strands. Because the strands are complementary, the number of adenosine nucleotides in each strand is also determined.

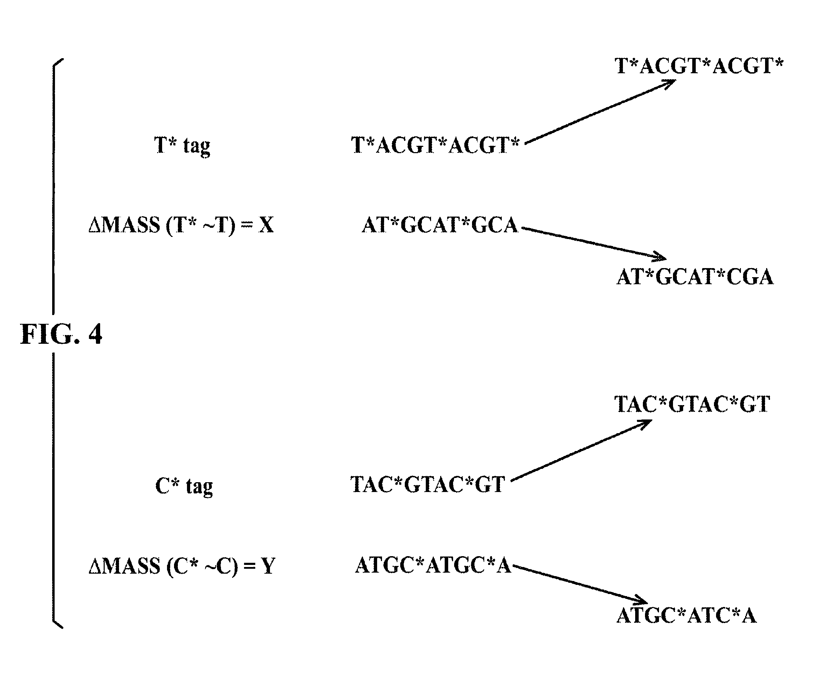

In another amplification reaction, the number of G and C residues in each strand is determined using, for example, the cytidine analog 5-methylcytosine (5-meC) or propyne C. The combination of the A/T reaction and G/C reaction, followed by molecular weight determination, provides a unique base composition. This method is summarized in Figure 4 and Table 1.

**Table 1**

| **Mass tag** | **Double strand sequence** | **Single strand sequence** | **Total Δmass this strand** | **Base info this strand** | **Base info other strand** | **Total base comp. Top strand** | **Total base comp. Bottom strand** |
|---|---|---|---|---|---|---|---|
| T*Δmass | | | 3x | 3T | 3A | 3T | 3A |
| (T*-T) = x | | | | | | 2A | 2T |
| | | | | | | 2C | 2G |
| | | | | | | 2G | 2C |
| | | | 2x | 2T | 2A | | |
| C*Δmass | | | 2x | 2C | 2G | | |
| (C*-C) = y | | | | | | | |
| | | | | | | | |
| | | | 2x | 2C | 2G | | |

The mass tag phosphorothioate A (A*) was used to distinguish a *Bacillus anthracis* cluster. The *B. anthracis* (A₁₄G₉C₁₄T₉) had an average MW of 14072.26, and the *B. anthracis* (A₁A*₁₃G₉C₁₄T₉) had an average molecular weight of 14281.11 and the phosphorothioate A had an average molecular weight of +16.06 as determined by ESI-TOF MS. The deconvoluted spectra are shown in Figure 5.

In another example, assume the measured molecular masses of each strand are 30,000.115Da and 31,000.115 Da respectively, and the measured number of dT and dA residues are (30,28) and (28,30). If the molecular mass is accurate to 100 ppm, there are 7 possible combinations of dG+dC possible for each strand. However, if the measured molecular mass is accurate to 10 ppm, there are only 2 combinations of dG+dC, and at 1 ppm accuracy there is only one possible base composition for each strand.

Signals from the mass spectrometer may be input to a maximum-likelihood detection and classification algorithm such as is widely used in radar signal processing. The detection processing uses matched filtering of BCS observed in mass-basecount space and allows for detection and subtraction of signatures from known, harmless organisms, and for detection of unknown bioagent threats. Comparison of newly observed bioagents to known bioagents is also possible, for estimation of threat level, by comparing their BCS to those of known organisms and to known forms of pathogenicity enhancement, such as insertion of antibiotic resistance genes or toxin genes.

Processing may end with a Bayesian classifier using log likelihood ratios developed from the observed signals and average background levels. The program emphasizes performance predictions culminating in probability-of-detection versus probability-of-false-alarm plots for conditions involving complex backgrounds of naturally occurring organisms and environmental contaminants. Matched filters consist of a priori expectations of signal values given the set of primers used for each of the bioagents. A genomic sequence database (e.g. GenBank) is used to define the mass basecount matched filters. The database contains known threat agents and benign background organisms. The latter is used to estimate and subtract the signature produced by the background organisms. A maximum likelihood detection of known background organisms is implemented using matched filters and a running-sum estimate of the noise covariance. Background signal strengths are estimated and used along with the matched filters to form signatures which are then subtracted. the maximum likelihood process is applied to this "cleaned up" data in a similar manner employing matched filters for the organisms and a running-sum estimate of the noise-covariance for the cleaned up data.

In one embodiment, a strategy to "triangulate" each organism by measuring signals from multiple core genes is used to reduce false negative and false positive signals, and enable reconstruction of the origin or hybrid or otherwise engineered bioagents. After identification of multiple core genes, alignments are created from nucleic acid sequence databases. The alignments are then analyzed for regions of conservation and variation, and potential primer binding sites flanking variable regions are identified. Next, amplification target regions for signature analysis are selected which distinguishes organisms based on specific genomic differences (i.e., base composition). For example, detection of signatures for the three part toxin genes typical of *B. anthracis* (Bowen, J.E. and C. P. Quinn, J. Appl. Microbiol. 1999, 87, 270-278) in the absence of the expected signatures from the *B. anthracis* genome would suggest a genetic engineering event.

The present method can also be used to detect single nucleotide polymorphisms (SNPs), or multiple nucleotide polymorphisms, rapidly and accurately. A SNP is defined as a single base pair site in the genome that is different from one individual to another. The difference can be expressed either as a deletion, an insertion or a substitution, and is frequently linked to a disease state. Because they occur every 100-1000 base pairs, SNPs are the most frequently bound type of genetic marker in the human genome.

For example, sickle cell anemia results from an A-T transition, which encodes a valine rather than a glutamic acid residue. Oligonucleotide primers may be designed such that they bind to sequences which flank a SNP site, followed by nucleotide amplification and mass determination of the amplified product. Because the molecular masses of the resulting product from an individual who does not have sickle cell anemia is different from that of the product from an individual who has the disease, the method can be used to distinguish the two individuals. Thus, the method can be used to detect any known SNP in an individual and thus diagnose or determine increased susceptibility to a disease or condition.

In one embodiment, blood is drawn from an individual and peripheral blood mononuclear cells (PBMC) are isolated and simultaneously tested, preferably in a high-throughput screening method, for one or more SNPs using appropriate primers based on the known sequences which flank the SNP region. The National Center for Biotechnology Information maintains a publicly available database of SNPs (www.ncbi.nlm.nih.gov/SNP/).

The method of the present disclosure can also be used for blood typing. The gene encoding A, B or O blood type can differ by four single nucleotide polymorphisms. If the gene contains the sequence CGTGGTGACCCTT (SEQ ID NO:5), antigen A results. If the gene contains the sequence CGTCGTCACCGCTA (SEQ ID NO:6) antigen B results. If the gene contains the sequence CGTGGT-ACCCCTT (SEQ ID NO:7), blood group O results ("-" indicates a deletion). These sequences can be distinguished by designing a single primer pair which flanks these regions, followed by amplification and mass determination.

While the present invention has been described with specificity in accordance with certain of its preferred embodiments, the following examples serve only to illustrate the invention and are not intended to limit the same.

### Example 1

### Nucleic acid isolation and PCR

In one embodiment, nucleic acid is isolated from the organisms and amplified by PCR using standard methods prior to BCS determination by mass spectrometry. Nucleic acid is isolated, for example, by detergent lysis of bacterial cells, centrifugation and ethanol precipitation. Nucleic acid isolation methods are described in, for example, *Current Protocols in Molecular Biology* (Ausubel et al.) and *Molecular Cloning; A Laboratory* Manual (Sambrook et al.). The nucleic acid is then amplified using standard methodology, such as PCR, with primers which bind to conserved regions of the nucleic acid which contain an intervening variable sequence as described below.

### Example 2

### Mass spectrometry

**FTICR Instrumentation:** The FTICR instrument is based on a 7 tesla actively shielded superconducting magnet and modified Bruker Daltonics Apex II 70e ion optics and vacuum chamber. The spectrometer is interfaced to a LEAP PAL autosampler and a custom fluidics control system for high throughput screening applications. Samples are analyzed directly from 96-well or 384-well microtiter plates at a rate of about 1 sample/minute. The Bruker data-acquisition platform is supplemented with a lab-built ancillary NT datastation which controls the autosampler and contains an arbitrary waveform generator capable of generating complex rf-excite waveforms (frequency sweeps, filtered noise, stored waveform inverse Fourier transform (SWIFT), etc.) for sophisticated tandem MS experiments. For oligonucleotides in the 20-30-mer regime typical performance characteristics include mass resolving power in excess of 100,000 (FWHM), low ppm mass measurement errors, and an operable m/z range between 50 and 5000 m/z.

**Modified ESI Source:** In sample-limited analyses, analyte solutions are delivered at 150 nL/minute to a 30 mm i.d. fused-silica ESI emitter mounted on a 3-D micromanipulator. The ESI ion optics consist of a heated metal capillary, an rf-only hexapole, a skimmer cone, and an auxiliary gate electrode. The 6.2 cm rf-only hexapole is comprised of 1 mm diameter rods and is operated at a voltage of 380 Vpp at a frequency of 5 MHz. A lab-built electro-mechanical shutter can be employed to prevent the electrospray plume from entering the inlet capillary unless triggered to the "open" position via a TTL pulse from the data station. When in the "closed" position, a stable electrospray plume is maintained between the ESI emitter and the face of the shutter. The back face of the shutter arm contains an elastomeric seal which can be positioned to form a vacuum seal with the inlet capillary. When the seal is removed, a 1 mm gap between the shutter blade and the capillary inlet allows constant pressure in the external ion reservoir regardless of whether the shutter is in the open or closed position. When the shutter is triggered, a "time slice" of ions is allowed to enter the inlet capillary and is subsequently accumulated in the external ion reservoir. The rapid response time of the ion shutter ( < 25 ms) provides reproducible, user defined intervals during which ions can be injected into and accumulated in the external ion reservoir.

**Apparatus for Infrared Multiphoton Dissociation** A 25 watt CW CO₂ laser operating at 10.6 µm has been interfaced to the spectrometer to enable infrared multiphoton dissociation (IRMPD) for oligonucleotide sequencing and other tandem MS applications. An aluminum optical bench is positioned approximately 1.5 m from the actively shielded superconducting magnet such that the laser beam is aligned with the central axis of the magnet. Using standard IR-compatible mirrors and kinematic mirror mounts, the unfocused 3 mm laser beam is aligned to traverse directly through the 3.5 mm holes in the trapping electrodes of the FTICR trapped ion cell and longitudinally traverse the hexapole region of the external ion guide finally impinging on the skimmer cone. This scheme allows IRMPD to be conducted in an *m*/*z* selective manner in the trapped ion cell (e.g. following a SWIFT isolation of the species of interest), or in a broadband mode in the high pressure region of the external ion reservoir where collisions with neutral molecules stabilize IRMPD-generated metastable fragment ions resulting in increased fragment ion yield and sequence coverage.

### Example 3

### Identification of bioagents

Table 1 shows a small cross section of a database of calculated molecular masses for over 9 primer sets and approximately 30 organisms. The primer sets were derived from rRNA alignment. Examples of regions from rRNA consensus alignments are shown in Figures 1A-1C. Lines with arrows are examples of regions to which intelligent primer pairs for PCR are designed. The primer pairs are >95% conserved in the bacterial sequence database (currently over 10,000 organisms). The intervening regions are variable in length and/or composition, thus providing the base composition "signature" (BCS) for each organism. Primer pairs were chosen so the total length of the amplified region is less than about 80-90 nucleotides. The label for each primer pair represents the starting and ending base number of the amplified region on the consensus diagram.

Included in the short bacterial database cross-section in Table 1 are many well known pathogens/biowarfare agents (shown in bold/red typeface) such as *Bacillus anthracis* or *Yersinia pestis* as well as some of the bacterial organisms found commonly in the natural environment such as *Streptomyces.* Even closely related organisms can be distinguished from each other by the appropriate choice of primers. For instance, two low G+C organisms, *Bacillus anthracis* and *Staph aureus*, can be distinguished from each other by using the primer pair defined by 16S_1337 or 23S_855 (ΔM of 4 Da).

**Table 2**

| **Cross Section Of A Database Of Calculated Molecular Masses¹** | | | | | | | | | |
|---|---|---|---|---|---|---|---|---|---|
| **Primer Regions →** | **16S_971** | **16S_1100** | **16S_1337** | **16S-1294** | **16S_1228** | **23S_1021** | **23S_855** | **23S_193** | **23S_115** |
| **Bug Name** | | | | | | | | | |
| Acinetobacter calcoaceticus | 55619.1 | 55004 | 28446.7 | 35854.9 | 51295.4 | 30299 | 42654 | 39557.5 | 54999 |
| **Bacillus anthracis** | **55005** | **54388** | **28448** | **35238** | **51296** | **30295** | **42651** | **39560** | **56850** |
| Bacillus cereus | 55622.1 | 54387.9 | 28447.6 | 35854.9 | 51296.4 | 30295 | 42651 | 39560.5 | 56850.3 |
| Bordetella bronchiseptica | 56857.3 | 51300.4 | 28446.7 | 35857.9 | 51307.4 | 30299 | 42653 | 39559.5 | 51920.5 |
| Borrelia burgdorferi | 56231.2 | 55621.1 | 28440.7 | 35852.9 | 51295.4 | 30297 | 42029.9 | 38941.4 | 52524.6 |
| **Brucella abortus** | **58098** | **55011** | **28448** | **35854** | **50683** | | | | |
| Campylobacter jejuni | 58088.5 | 54386.9 | 29061.8 | 35656.9 | 50674.3 | 30294 | 42032.9 | 39558.5 | 45732.5 |
| Chlamydia pnuemoniae | 55000 | 55007 | 29063 | 35855 | 50676 | 30295 | 42036 | 38941 | 56230 |
| Clostridium botulinum | 55006 | 53767 | 28445 | 35855 | 51291 | 30300 | 42656 | 39562 | 54999 |
| Clostridium difficile | 56855.3 | 54386.9 | 28444.7 | 35853.9 | 51296.4 | 30294 | 41417.8 | 39556.5 | 55612.2 |
| Enterococcus faecalis | 55620.1 | 54387.9 | 28447.6 | 35858.9 | 51296.4 | 30297 | 42652 | 39559.5 | 56849.3 |
| **Escherichia coli** | **55622** | **55009** | **28445** | **35857** | **51301** | **30301** | **42656** | **39562** | **54999** |
| **Francisella tularensis** | **53769** | **54365** | **28445** | **35856** | **51298** | | | | |
| Haemophilus influenzae | 55620.1 | 55006 | 28444.7 | 35855.9 | 51298.4 | 30298 | 42656 | 39560.5 | 55613.1 |
| Klebsiella pneumoniae | 55622.1 | 55008 | 28442.7 | 35856.9 | 51297.4 | 30300 | 42655 | 39562.5 | 55000 |
| **Legionelia pneumophila** | **55618** | **55626** | **28446** | **35857** | **51303** | | | | |
| Mycobacterium avium | 54390.9 | 55631.1 | 29064.8 | 35858.9 | 51915.5 | 30298 | 42656 | 38942.4 | 56241.2 |
| Mycobacterium leprae | 54389.9 | 55629.1 | 29064.6 | 35860.9 | 51917.5 | 30298 | 42656 | 39559.5 | 56240.2 |
| Mycobacterium tuberculosis | 54390.9 | 55629.1 | 29064.8 | 35860.9 | 51301.4 | 30299 | 42656 | 39560.5 | 56243.2 |
| Mycoplasma genitalium | 53143.7 | 45115.4 | 29061.8 | 35854.9 | 50671.3 | 30294 | 43264.1 | 39558.5 | 56842.4 |
| Mycoplasma pneumoniae | 53143.7 | 45118.4 | 29061.8 | 35854.9 | 50673.3 | 30294 | 43264.1 | 39559.5 | 56843.4 |
| Neisseria gonorrhoeae | 55627.1 | 54389.9 | 28445.7 | 35855.9 | 51302.4 | 30300 | 42649 | 39561.5 | 55000 |
| **Pseudomonas aeruginosa** | **55623** | **55010** | **28443** | **35858** | **51301** | **30298** | **43272** | **39558** | **55619** |
| **Rickettsia prowazekii** | **58093** | **55621** | **28448** | **35853** | **50677** | **30293** | **42650** | **39559** | **53139** |
| **Rickettsia rickettsii** | **58094** | **55623** | **28448** | **35853** | **50679** | **30293** | **42648** | **39559** | **53755** |
| **Salmonella typhimurium** | **55622** | **55005** | **28445** | **35857** | **51301** | **30301** | **42658** | | |
| **Shigella dysenteriae** | **55623** | **55009** | **28444** | **35857** | **51301** | | | | |
| Staphylococcus aureus | 56854.3 | 54386.9 | 28443.7 | 35852.9 | 51294.4 | 30298 | 42655 | 39559.5 | 57466.4 |
| Streptomyces | 54389.9 | 59341.6 | 29063.8 | 35858.9 | 51300.4 | | | 39563.5 | 56864.3 |
| Treponema pallidum | 56245.2 | 55631.1 | 28445.7 | 35851.9 | 51297.4 | 30299 | 42034.9 | 38939.4 | 57473.4 |
| **Vibrio cholerae** | **55625** | **55626** | **28443** | **35857** | **52536** | **29063** | **30303** | **35241** | **50675** |
| Vibrio parahaemolyticus | 54384.9 | 55626.1 | 28444.7 | 34620.7 | 50064.2 | | | | |
| **Yersinia pestis** | **55620** | **65626** | **28443** | **35857** | **51299** | | | | |

| | | | | | | | | | |
|---|---|---|---|---|---|---|---|---|---|
| ¹Molecular mass distribution of PCR amplified regions for a selection of organisms (rows) across various primer pairs (columns). Pathogens are shown in **bold**. Empty cells indicate presently incomplete or missing data. | | | | | | | | | |

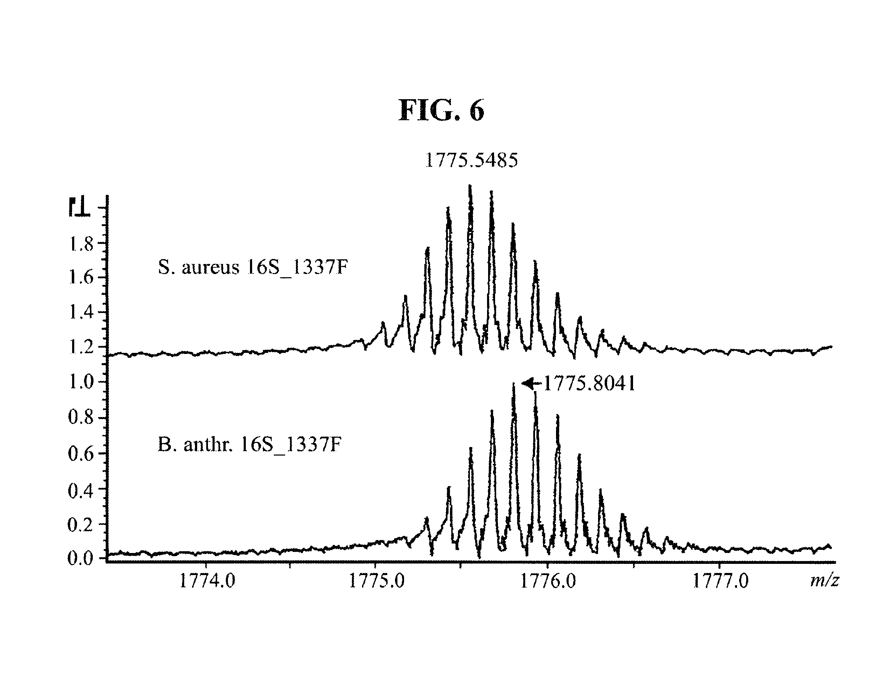

Figure 6 shows the use of ESI-FT-ICR MS for measurement of exact mass. The spectra from 46mer PCR products originating at position 1337 of the 16S rRNA from *S. aureus* (upper) and *B. anthracis* (lower) are shown. These data are from the region of the spectrum containing signals from the [M-8H+]⁸⁻ charge states of the respective 5'-3' strands. The two strands differ by two (AT→CG) substitutions, and have measured masses of 14206.396 and 14208.373 ± 0.010 Da, respectively. The possible base compositions derived from the masses of the forward and reverse strands for the *B. anthracis* products are listed in Table 3.

**Table 3**

| **Possible base composition for *B. anthracis* products** | | |
|---|---|---|
| **Calc. Mass** | **Error** | **Base Comp.** |
| 14208.2935 | 0.079520 | A1 G17 C10 T18 |
| 14208.3160 | 0.056980 | A1 G20 C15 T10 |
| 14208.3386 | 0.034440 | A1 G23 C20 T2 |
| 14208.3074 | 0.065560 | A6 G11 C3 T26 |
| 14208.3300 | 0.043020 | A6 G14 C8 T18 |
| 14208.3525 | 0.020480 | A6 G17 C13 T10 |
| 14208.3751 | 0.002060 | A6 G20 C18 T2 |
| 14208.3439 | 0.029060 | A11 G8 C1 T26 |
| 14208.3665 | 0.006520 | A11 G11 C6 T18 |
| **14208.3890** | **0.016020** | **A11 G14 C11 T10** |
| 14208.4116 | 0.038560 | A11 G17 C16 T2 |
| 14208.4030 | 0.029980 | A16 G8 C4 T18 |
| 14208.4255 | 0.052520 | A16 G11 C9 T10 |
| 14208.4481 | 0.075060 | A16 G14 C14 T2 |
| 14208.4395 | 0.066480 | A21 G5 C2 T18 |
| 14208.4620 | 0.089020 | A21 G8 C7 T10 |
| 14079.2624 | 0.080600 | A0 G14 C13 T19 |
| 14079.2849 | 0.058060 | A0 G17 C18 T11 |
| 14079.3075 | 0.035520 | A0 G20 C23 T3 |
| 14079.2538 | 0.089180 | A5 G5 C1 T35 |
| 14079.2764 | 0.066640 | A5 G8 C6 T27 |
| 14079.2989 | 0.044100 | A5 G11 C11 T19 |
| 14079.3214 | 0.021560 | A5 G14 C16 T11 |
| 14079.3440 | 0.000980 | A5 G17 C21 T3 |
| 14079.3129 | 0.030140 | A10 G5 C4 T27 |
| 14079.3354 | 0.007600 | A10 G8 C9 T19 |
| **14079.3579** | **0.014940** | **A10 G11 C14 T11** |
| 14079.3805 | 0.037480 | A10 G14 C19 T3 |
| 14079.3494 | 0.006360 | A15 G2 C2 T27 |
| 14079.3719 | 0.028900 | A15 G5 C7 T19 |
| 14079.3944 | 0.051440 | A15 G8 C12 T11 |
| 14079.4170 | 0.073980 | A15 G11 C17 T3 |
| 14079.4084 | 0.065400 | A20 G2 C5 T19 |
| 14079.4309 | 0.087940 | A20 G5 C10 T13 |

Among the 16 compositions for the forward strand and the 18 compositions for the reverse strand that were calculated, only one pair (shown in **bold**) are complementary, corresponding to the actual base compositions of the *B. anthracis* PCR products.

### Example 4

### BCS of region from Bacillus anthracis and Bacillus cereus

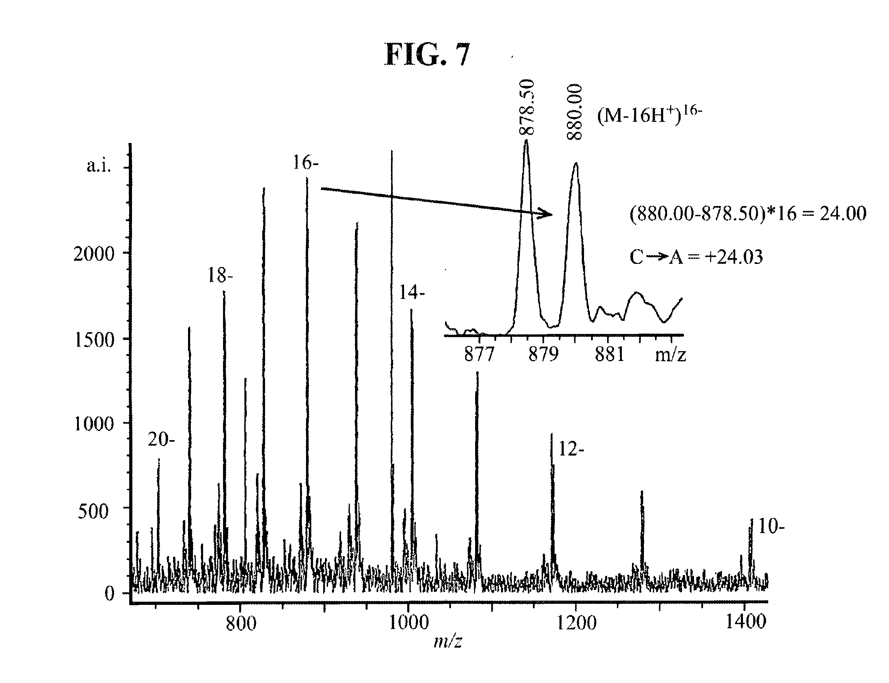

A conserved Bacillus region from *B. anthracis* (A₁₄G₉C₁₄T₉) and *B. cereus* (A₁₅G₉C₁₃T₉) having a C to A base change was synthesized and subjected to ESI-TOF MS. The results are shown in Figure 7 in which the two regions are clearly distinguished using the method of the present invention (MW=14072.26 vs. 14096.29).

### Example 5

### Identification of additional bioagents

In other examples of the present invention, the pathogen *Vibrio cholera* can be distinguished from *Vibrio parahemolyticus* with ΔM > 600 Da using one of three 16S primer sets shown in Table 2 (16S_971, 16S_1228 or 168_1294) as shown in Table 4. The two mycoplasma species in the list (*M. genitalium* and *M. pneumoniae*) can also be distinguished from each other, as can the three mycobacteriae. While the direct mass measurements of amplified products can identify and distinguish a large number of organisms, measurement of the base composition signature provides dramatically enhanced resolving power for closely related organisms. In cases such as *Bacillus anthracis* and *Bacillus cereus* that are virtually indistinguishable from each other based solely on mass differences, compositional analysis or fragmentation patterns are used to resolve the differences. The single base difference between the two organisms yields different fragmentation patterns, and despite the presence of the ambiguous/unidentified base N at position 20 in *B. anthracis*, the two organisms can be identified.

Tables 4a-b show examples of primer pairs from Table 1 which distinguish pathogens from background.

**Table 4a**

| **Organism name** | **23S_855** | **16S_1337** | **23S_1021** |
|---|---|---|---|
| *Bacillus anthracis* | 42650.98 | 28447.65 | 30294.98 |
| *Staphylococcus aureus* | 42654.97 | 28443.67 | 30297.96 |

**Table 4b**

| **Organism name** | **16S_971** | **16S_1294** | **16S_1228** |
|---|---|---|---|
| *Vibrio cholerae* | 55625.09 | 35856.87 | 52535.59 |
| *Vibrio parahaemolyticus* | 54384.91 | 34620.67 | 50064.19 |

Table 4 shows the expected molecular weight and base composition of region 16S_1100-1188 in *Mycobacterium avium* and *Streptomyces sp.*

**Table 5**

| **Region** | **Organism name** | **Length** | **Molecular weight** | **Base comp.** |
|---|---|---|---|---|
| 16S_1100-1188 | *Mycobacterium avium* | 82 | 25624.1728 | A₁₆G₃₂C₁₈T₁₆ |
| 16S_1100-1188 | *Streptomyces sp.* | 96 | 29904.871 | A₁₇G₃₈C₂₇T₁₄ |

Table 5 shows base composition (single strand) results for 16S_1100-1188 primer amplification reactions different species of bacteria. Species which are repeated in the table (e.g., Clostridium botulinum) are different strains which have different base compositions in the 16S_1100-1188 region.

**Table 6**

| **Organism name** | **Base comp.** | **Organism name** | **Base comp.** |
|---|---|---|---|
| Mycobacterium avium | A₁₆G₃₂C₁₈T₁₆ | Vibrio cholerae | A₂₃G₃₀C₂₁T₁₆ |
| Streptomyces sp. | A₁₇G₃₈C₂₇T₁₄ | **Aeromonas hydrophila** | **A₂₃G₃₁C₂₁T₁₅** |
| Ureaplasma urealyticum | A₁₈G₃₀C₁₇T₁₇ | **Aeromonas salmonicida** | **A₂₃G₃₁C₂₁T₁₅** |
| Streptomyces sp. | A₁₉G₃₆C₂₄T₁₈ | Mycoplasma genitalium | A₂₄G₁₉C₁₂T₁₈ |
| Mycobacterium leprae | A₂₀G₃₂C₂₂T₁₆ | Clostridium botulinum | A₂₄G₂₅C₁₈T₂₀ |
| **M. tuberculosis** | **A₂₀G₃₃C₂₁T₁₆** | Bordetella bronchiseptica | A₂₄G₂₆C₁₉T₁₄ |
| **Nocardia asteroides** | **A₂₀C₃₃C₂₁T₁₆** | Francisella tularensis | A₂₄G₂₆C₁₉T₁₉ |
| Fusobacterium necroforum | A₂₁G₂₆C₂₂T₁₈ | **Bacillus anthracis** | **A₂₄G₂₆C₂₀T₁₈** |
| Listeria monocytogenes | A₂₁G₂₇C₁₉T₁₉ | **Campylobacter jejuni** | **A₂₄G₂₆C₂₀T₁₈** |
| Clostridium botulinum | A₂₁G₂₇C₁₉T₂₁ | **Staphylococcus aureus** | **A₂₄G₂₆C₂₀T₁₈** |
| Neisseria gonorrhoeae | A₂₁G₂₈C₂₁T₁₈ | Helicobacter pylori | A₂₄G₂₆C₂₀T₁₉ |
| Bartonella quintana | A₂₁G₃₀C₂₂T₁₆ | Helicobacter pylori | A₂₄G₂₆C₂₁T₁₈ |
| Enterococcus faecalis | A₂₂C₂₇C₂₀T₁₉ | Moraxella catarrhalis | A₂₄G₂₆C₂₃T₁₆ |
| Bacillus megaterium | A₂₂G₂₈C₂₀T₁₈ | Haemophilus influenzae Rd | A₂₄G₂₈C₂₀T₁₇ |
| Bacillus subtilis | A₂₂G₂₈C₂₁T₁₇ | **Chlamydia trachomatis** | **A₂₄G₂₈C₂₁T₁₆** |
| Pseudomonas aeruginosa | A₂₂G₂₉C₂₃T₁₅ | **Chlamydophila pneumoniae** | **A₂₄G₂₈C₂₁T₁₆** |
| Legionella pneumophila | A₂₂CT₃₂C₂₀T₁₆ | **C. pneumonia AR39** | **A₂₄G₂₈C₂₁T₁₆** |
| Mycoplasma pneumoniae | A₂₃G₂₀C₁₄T₁₆ | Pseudomonas putida | A₂₄G₂₉C₂₁T₁₆ |
| Clostridium botulinum | A₂₃G₂₆C₂₀T₁₉ | **Proteus vulgaris** | **A₂₄G₃₀C₂₁T₁₅** |
| Enterococcus faecium | A₂₃G₂₆C₂₁T₁₈ | **Yersinia pestis** | **A₂₄G₃₀C₂₁T₁₅** |
| Acinetobacter calcoaceti | A₂₃G₂₆C₂₁T₁₉ | **Yersinia pseudotuberculos** | **A₂₄G₃₀C₂₁T₁₅** |
| **Leptospira borgpeterseni** | **A₂₃G₂₆C₂₄T₁₅** | Clostridium botulinum | A₂₅G₂₄C₁₈T₂₁ |
| **Leptospira interrogans** | **A₂₃G₂₆C₂₄T₁₅** | Clostridium tetani | A₂₅G₂₅C₁₈T₂₀ |
| Clostridium perfringens | A₂₃G₂₇C₁₉T₁₉ | Francisella tularensis | A₂₅G₂₅C₁₉T₁₉ |
| **Bacillus anthracis** | **A₂₃G₂₇C₂₀T₁₈** | Acinetobacter calcoacetic | A₂₅G₂₆C₂₀T₁₉ |
| **Bacillus cereus** | **A₂₃G₂₇C₂₀T₁₈** | Bacteriodes fragilis | A₂₅G₂₇C₁₆T₂₂ |
| **Bacillus thuringiensis** | **A₂₃G₂₇C₂₀T₁₈** | Chlamydophila psittaci | A₂ₛG₂₇C₂₁T₁₆ |
| *Aeromonas hydrophila* | *A₂₃G₂₉C₂₇T₁₆* | Borrelia burgdorferi | A₂₅G₂₉C₁₇T₁₉ |
| *Escherichia coli* | *A₂₃G₂₉C₂₁T₁₆* | Streptobacillus monilifor | A₂₆G₂₆C₂₀T₁₆ |
| Pseudomonas putida | A₂₃G₂₉C₂₁T₁₇ | Rickettsia prowazekii | A₂₆G₂₈C₁₈T₁₈ |
| **Escherichia coli** | **A₂₃G₂₉C₂₂T₁₅** | Rickettsia rickettsii | A₂₆G₂₈C₂₀T₁₆ |
| **Shigella dysenteriae** | **A₂₃G₂₉C₂₂T₁₅** | Mycoplasma mycoides | A₂₈G₂₃C₁₆T₂₀ |

The same organism having different base compositions are different strains. Groups of organisms which are highlighted or in italics have the same base compositions in the amplified region. Some of these organisms can be distinguished using multiple primers. For example, *Bacillus anthracis* can be distinguished from *Bacillus cereus* and *Bacillus thuringiensis* using the primer 16S_971-1062 (Table 6). Other primer pairs which produce unique base composition signatures are shown in Table 6 (bold). Clusters containing very similar threat and ubiquitous non-threat organisms (e.g. *anthracis* cluster) are distinguished at high resolution with focused sets of primer pairs. The known biowarfare agents in Table 6 are *Bacillus anthracis*, *Yersinia pestis*, *Francisella tularensis* and *Rickettsia prowazekii*.

**Table 7**

| **Organism** | **16S_971-1062** | **16S_1228-1310** | **16S_1100-1188** |
|---|---|---|---|
| *Aeromonas hydrophila* | A₂₁G₂₉C₂₂T₂₀ | A₂₂G₂₇C_{2I}T₁₃ | A₂₃G₃₁C₂₁T₁₅ |
| *Aeromonas salmonicida* | A₂₁G₂₉C₂₂T₂₀ | A₂₂G₂₇C₂₁T₁₃ | A₂₃G₃₁C₂₁T₁₅ |
| *Bacillus anthracis* | **A₂₁G₂₇C₂₂T₂₂** | A₂₄G₂₂C₁₉T₁₈ | A₂₃G₂₇C₂₀T₁₈ |
| *Bacillus cereus* | A₂₂G₂₇C₂₁T₂₂ | A₂₄G₂₂C₁₉T₁₈ | A₂₃G₂₇C₂₀T₁₈ |
| *Bacillus thuringiensis* | A₂₂G₂₇C₂₁T₂₂ | A₂₄G₂₂C₁₉T₁₈ | A₂₃G₂₇C₂₀T₁₈ |
| *Chlamydia trachomatis* | **A₂₂G₂₆C₂₀T₂₃** | **A₂₄G₂₃C₁₉T₁₆** | A₂₄G₂₈C₂₁T₁₆ |
| *Chlamydia pneumoniae AR39* | A₂₆G₂₃C₂₀T₂₂ | A₂₆G₂₂C₁₆T₁₈ | A₂₄G₂₈C₂₁T₁₆ |
| *Leptospira borgpetersenii* | A₂₂G₂₆C₂₀T₂₁ | A₂₂G₂₅C₂₁T₁₅ | A₂₃G₂₆C₂₄T₁₅ |
| *Leptospira interrogans* | A₂₂G₂₆C₂₀T₂₁ | A₂₂G₂₅C₂₁T₁₅ | A₂₃G₂₆C₂₄T₁₅ |
| *Mycoplasma genitalium* | A₂₈G₂₃C₁₅T₂₂ | **A₃₀G₁₈C₁₅T₁₉** | **A₂₄G₁₉C₁₂T₁₈** |
| *Mycoplasma pneumoniae* | A₂₈G₂₃C₁₅T₂₂ | **A₂₇G₁₉C₁₆T₂₀** | **A₂₃G₂₀C₁₄T₁₆** |
| *Escherichia coli* | **A₂₂G₂₈C₂₀T₂₂** | A₂₄G₂₅C₂₁T₁₃ | A₂₃G₂₉C₂₂T₁₅ |
| *Shigella dysenteriae* | **A₂₂G₂₈C₂₁T₂₁** | A₂₄G₂₅C₂₁T₁₃ | A₂₃G₂₉C₂₂T₁₅ |
| *Proteus vulgaris* | **A₂₃G₂₆C₂₂T₂₁** | **A₂₆G₂₄C₁₉T₁₄** | A₂₄G₃₀C₂₁T₁₅ |
| *Yersinia pestis* | A₂₄G₂₅C₂₁T₂₂ | A₂₅G₂₄C₂₀T₁₄ | A₂₄G₃₀C₂₁T₁₅ |
| *Yersinia pseudotuberculosis* | A₂₄G₂₅C₂₁T₂₂ | A₂₅G₂₄C₂₀T₁₄ | A₂₄G₃₀C₂₁T₁₅ |
| *Francisella tularensis* | **A₂₀G₂₅C₂₁T₂₃** | **A₂₃G₂₆C₁₇T₁₇** | **A₂₄G₂₆C₁₉T₁₉** |
| *Rickettsia prowazekii* | **A₂₁G₂₆C₂₄T₂₅** | **A₂₄G₂₃C₁₆T₁₉** | **A₂₆G₂₈C₁₈T₁₈** |
| *Rickettsia rickettsii* | **A₂₁G₂₆C₂₅T₂₄** | **A₂₄G₂₄C₁₇T₁₇** | **A₂₆G₂₈C₂₀T₁₆** |

The sequence of *B. anthracis* and *B. cereus* in region 16S_971 is shown below. Shown in bold is the single base difference between the two species which can be detected using the methods of the present invention. *B. anthracis* has an ambiguous base at position 20.
*B.anthracis*_16S_971 *B.cereus*_16S_971

### Example 6

### ESI-TOF MS of sspE 56-mer plus calibrant

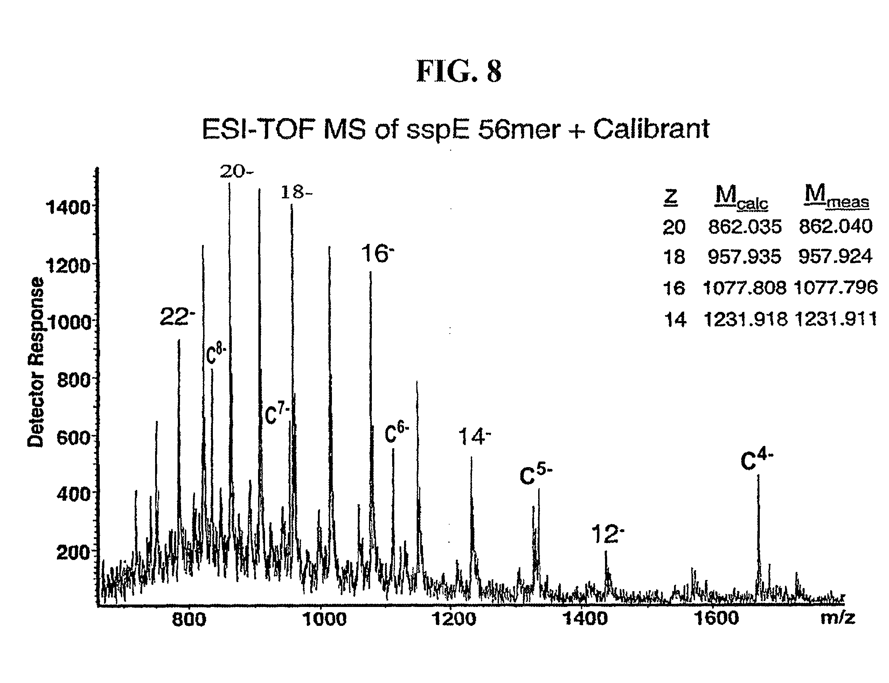

The mass measurement accuracy that can be obtained using an internal mass standard in the ESI-MS study of PCR products is shown in Fig.8. The mass standard was a 20-mer phosphorothioate oligonucleotide added to a solution containing a 56-mer PCR product from the *B. anthracis* spore coat protein sspE. The mass of the expected PCR product distinguishes *B. anthracis* from other species of Bacillus such as *B. thuringiensis and B. cereus.*

### Example 7

### B. anthracis ESI-TOF synthetic 16S_1228 duplex

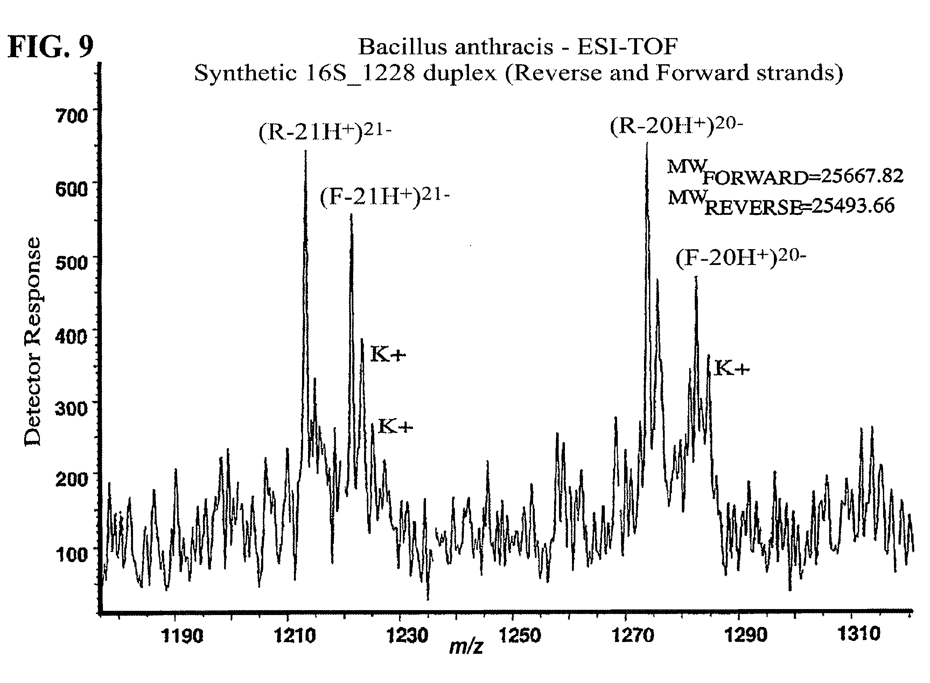

An ESI-TOF MS spectrum was obtained from an aqueous solution containing 5 µM each of synthetic analogs of the expected forward and reverse PCR products from the nucleotide 1228 region of the *B. anthracis* 16S rRNA gene. The results (Fig. 9) show that the molecular weights of the forward and reverse strands can be accurately determined and easily distinguish the two strands. The [M-21H⁺]²¹- and [M-20H⁺]²⁰⁻ charge states are shown.

### Example 8

### ESI-FTICR-MS of synthetic B. anthracis 16S_1337 46 base pair duplex

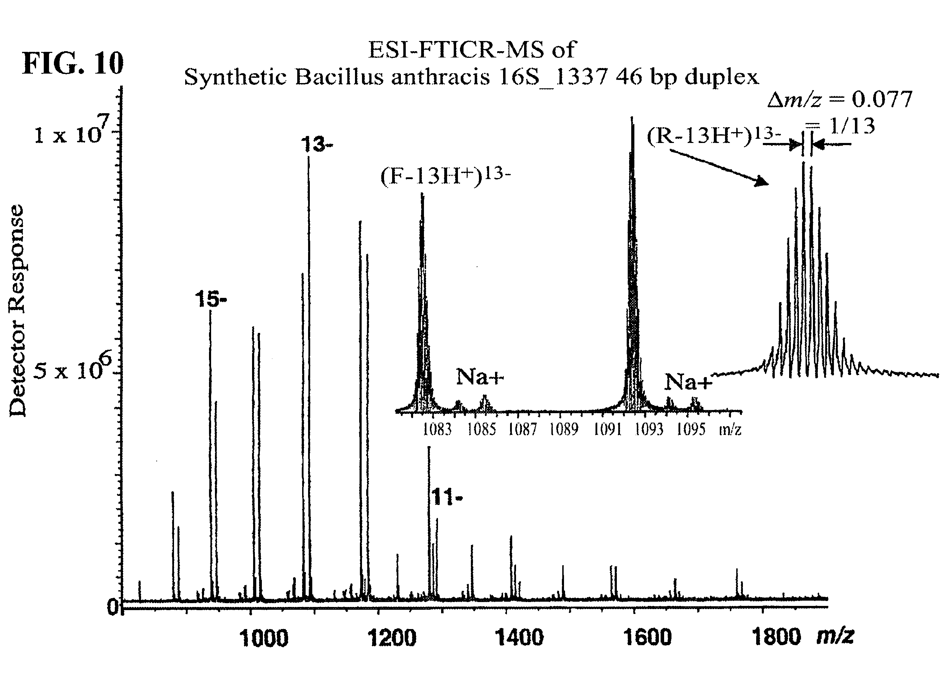

An ESI-FTICR-MS spectrum was obtained from an aqueous solution containing 5 µM each of synthetic analogs of the expected forward and reverse PCR products from the nucleotide 1337 region of the *B. anthracis* 16S rRNA gene. The results (Fig. 10) show that the molecular weights of the strands can be distinguished by this method. The [M-16H⁺]¹⁶⁻ through [M-10H⁺]¹⁰⁻ charge states are shown. The insert highlights the resolution that can be realized on the FTICR-MS instrument, which allows the charge state of the ion to be determined from the mass difference between peaks differing by a single 13C substitution.

### Example 9

### ESI-TOF MS of 56-mer oligonucleotide from saspB gene of B. anthracis with internal mass standard

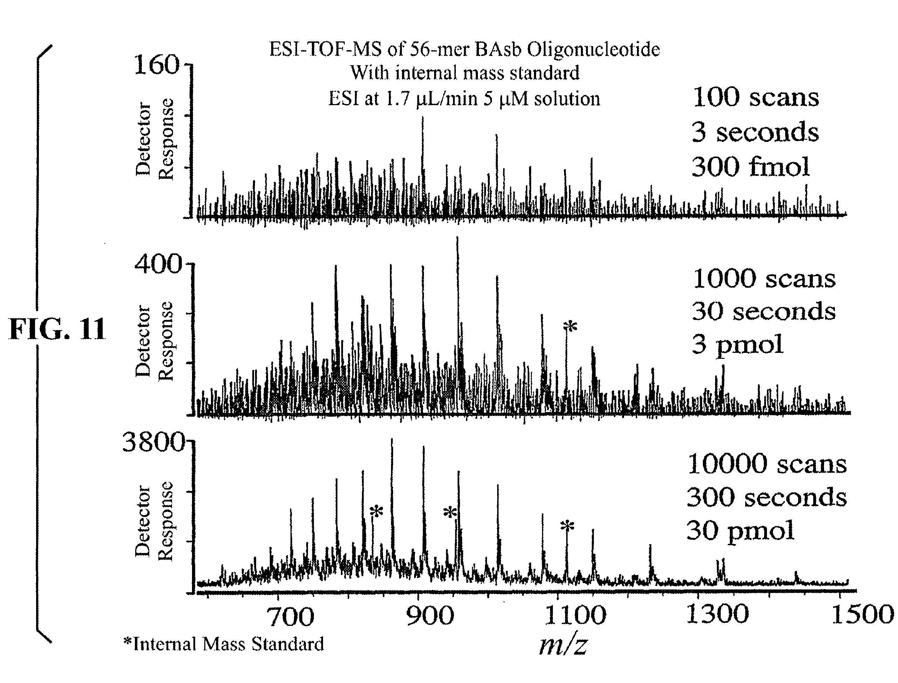

ESI-TOF MS spectra were obtained on a synthetic 56-mer oligonucleotide (5 µM) from the saspB gene of *B. anthracis* containing an internal mass standard at an ESI of 1.7 µL/min as a function of sample consumption. The results (Fig. 11) show that the signal to noise is improved as more scans are summed, and that the standard and the product are visible after only 100 scans.

### Example 10

### ESI-TOF MS of an internal standard with tributylammonium (TBA)-trifluoroacetate (TFA) buffer

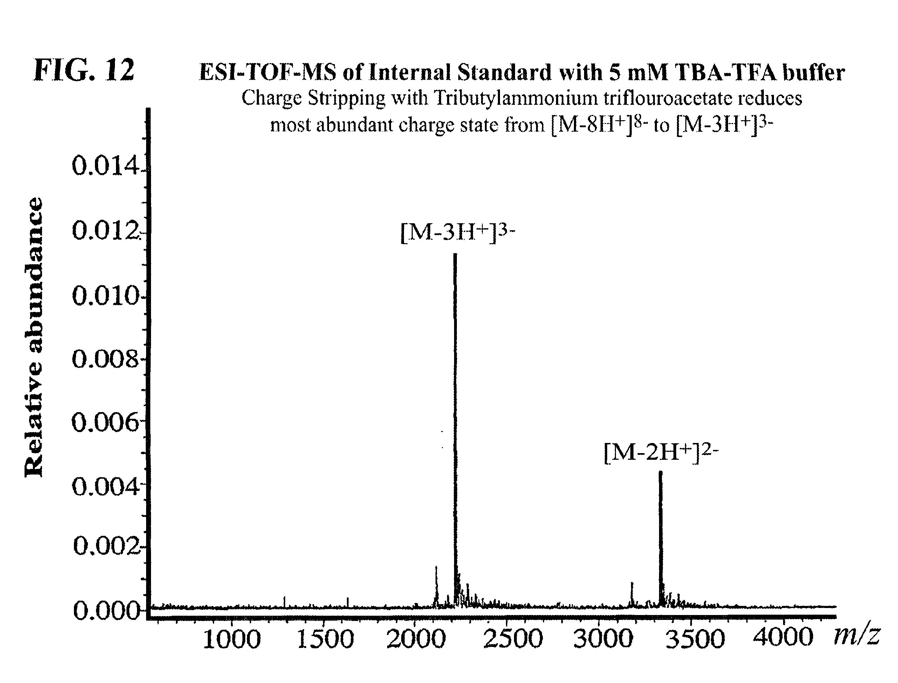

An ESI-TOF-MS spectrum of a 20-mer phosphorothioate mass standard was obtained following addition of 5 mM TBA-TFA buffer to the solution. This buffer strips charge from the oligonucleotide and shifts the most abundant charge state from [M-8H⁺]⁸⁻ to [M-3H⁺]³⁻ (Fig. 12).

## Claims

1. A method of identifying an unknown bioagent comprising:
a) aligning a plurality of nucleic acid gene sequences of a plurality of organisms of known identity;
b) analyzing said plurality of sequences for regions of conservation and variation;
c) identifying a plurality of primer binding sites flanking variable regions for priming and obtaining one or more amplifiable regions within said plurality of sequences;
d) identifying the base compositions of said one or more amplifiable regions for members of said plurality of known organisms to obtain a plurality of base compositions of amplifiable regions indexed to the identity of one or more members of said plurality of organisms of known identity;
e) preparing a database containing said base compositions and indexed identities of members of said plurality of organisms obtained in step d).
f) determining a base composition of a nucleic acid of said unknown bioagent, by contacting the nucleic acid with at least one pair of oligonucleotide primers which hybridize to sequences of said one or more amplifiable regions, amplifying the variable nucleic acid sequence to produce an amplification product, determining the molecular mass of said amplification product by mass spectrometry and then determining the base composition of the amplification product; and
g) identifying said unknown bioagent by comparing said base composition of said nucleic acid of said unknown bioagent to said database.

2. The method of claim 1, wherein step (d) further comprises calculating the molecular masses of said amplifiable regions and indexing said molecular masses to said identities of members of said plurality of organisms.

3. The method of claim 1, wherein said organism is a bacterium, virus, or microbial pathogen.

4. The method of claim 1, wherein said nucleic acid sequences comprise at least one gene encoding ribosomal RNA or at least one gene encoding at least one protein that participates in translation, replication, recombination and repair, transcription, nucleotide metabolism, amino acid metabolism, lipid metabolism, energy generation or secretion.

5. The method of claim 4, wherein said at least one gene encoding said at least one protein encodes DNA polymerase, RNA polymerase, elongation factor Tu, heat shock protein groEL, phosphoglycerate kinase, NADH dehydrogenase, DNA ligase, DNA topoisomerase, or elongation factor G.

6. The method of claim 1, wherein said primer binding sites are 80% to 100% conserved among said aligned sequences of step a).

7. The method of claim 1 wherein the intervening region between said primer binding sites has no greater than 5% sequence identity among said aligned sequences of step a).

8. The method of claim 1, wherein said amplifiable regions are between 50 and 250 nucleotides in length.

9. The method of claim 1, wherein said identity is a genus name, a species name, or a strain name.

## Patentansprüche

1. Verfahren zur Identifizierung eines unbekannten Bioagens, bei dem man:
a) mehrere Nukleinsäure-Gensequenzen aus mehreren Organismen mit bekannter Identität ausrichtet;
b) die mehreren Sequenzen auf Bereiche mit Konservierung und Variation analysiert;
c) mehrere, variable Bereiche flankierende Primer-Bindungsstellen für das Priming identifiziert und einen oder mehrere amplifizierbare Bereiche innerhalb der mehreren Sequenzen erhält;
d) die Basenzusammensetzungen der einen oder mehreren amplifizierbaren Bereiche für Mitglieder der mehreren bekannten Organismen identifiziert, so dass man mehrere Basenzusammensetzungen amplifizierbarer Bereiche erhält, die in einem Index der Identität eines oder mehrerer Mitglieder der mehreren Organismen mit bekannter Identität zugeordnet sind;
e) eine Datenbank anlegt, die die in Schritt d) erhaltenen Basenzusammensetzungen und indexierten Identitäten von Mitgliedern der mehreren Organismen enthält;
f) eine Basenzusammensetzung einer Nukleinsäure des unbekannten Bioagens bestimmt, indem man die Nukleinsäure mit wenigstens einem Paar Oligonukleotid-Primer, die an Sequenzen der einen oder mehreren amplifizierbaren Bereiche hybridisieren, in Kontakt bringt, die variable Nukleinsäuresequenz unter Erhalt eines Amplifikationsprodukts amplifiziert, die Molekülmasse des Amplifikationsprodukts mittels Massenspektrometrie bestimmt und dann die Basenzusammensetzung des Amplifikationsprodukts bestimmt; und
g) das unbekannte Bioagens identifiziert, indem man die Basenzusammensetzung des unbekannten Bioagens mit der Datenbank vergleicht.

2. Verfahren nach Anspruch 1, wobei man in Schritt (d) ferner die Molekülmassen der amplifizierbaren Bereiche berechnet und die Molekülmassen den Identitäten von Mitgliedern der mehreren Organismen zuordnet.

3. Verfahren nach Anspruch 1, wobei es sich bei dem Organismus um ein Bakterium, Virus oder einen mikrobiellen Krankheitserreger handelt.

4. Verfahren nach Anspruch 1, wobei die Nukleinsäuresequenzen wenigstens ein für ribosomale RNA codierendes Gen oder wenigstens ein für wenigstens ein an Translation, Replikation, Rekombination und Reparatur, Transkription, Nukleotidstoffwechsel, Aminosäurestoffwechsel, Lipidstoffwechsel, Energieerzeugung oder Sekretion beteiligtes Protein codierendes Gen umfassen.

5. Verfahren nach Anspruch 4, wobei das wenigstens eine für das wenigstens eine Protein codierende Gen für DNA-Polymerase, RNA-Polymerase, Elongationsfaktor Tu, Hitzeschockprotein groEL, Phosphoglyceratkinase, NADH-Dehydrogenase, DNA-Ligase, DNA-Topoisomerase oder Elongationsfaktor G codiert.

6. Verfahren nach Anspruch 1, wobei die Primer-Bindungsstellen unter den ausgerichteten Sequenzen aus Schritt a) zu 80% bis 100% konserviert sind.

7. Verfahren nach Anspruch 1, wobei der zwischen den Primer-Bindungsstellen liegende Bereich nicht mehr als 5% Sequenzidentität unter den ausgerichteten Sequenzen aus Schritt a) aufweist.

8. Verfahren nach Anspruch 1, wobei die amplifizierbaren Bereiche eine Länge zwischen 50 und 250 Nukleotiden aufweisen.

9. Verfahren nach Anspruch 1, wobei es sich bei der Identität um einen Gattungsnamen, einen Speziesnamen oder einen Stammnamen handelt.

## Revendications

1. Procédé d'identification d'un bioagent inconnu comprenant :
a) l'alignement d'une pluralité de séquences géniques d'acide nucléique d'une pluralité d'organismes d'identité connue ;
b) l'analyse de ladite pluralité de séquences pour les régions de conservation et variation ;
c) l'identification d'une pluralité de sites de liaison d'amorce flanquant des régions variables pour amorcer et obtenir une ou plusieurs régions amplifiables dans ladite pluralité de séquences ;
d) l'identification des compositions de base desdites une ou plusieurs régions amplifiables pour des membres de ladite pluralité d'organismes connus pour obtenir une pluralité de compositions de base de régions amplifiables indexées à l'identité d'un ou plusieurs membres de ladite pluralité d'organismes d'identité connue ;
e) la préparation d'une base de données contenant lesdites compositions de base et identités indexées de membres de ladite pluralité d'organismes obtenues dans l'étape d),
f) la détermination d'une composition de bases d'un acide nucléique dudit bioagent inconnu, par mise en contact de l'acide nucléique avec au moins une paire d'amorces oligonucléotidiques qui s'hybrident aux séquences desdites une ou plusieurs régions amplifiables, amplification de la séquence d'acide nucléique variable pour produire un produit d'amplification, détermination de la masse moléculaire dudit produit d'amplification par spectrométrie de masse et ensuite détermination de la composition de bases du produit d'amplification ; et
g) l'identification dudit bioagent inconnu par comparaison de ladite composition de bases dudit acide nucléique dudit bioagent inconnu à ladite base de données.

2. Procédé de la revendication 1, dans lequel l'étape (d) comprend en outre le calcul des masses moléculaires desdites régions amplifiables et l'indexation desdites masses moléculaires auxdites identités de membres de ladite pluralité d'organismes.

3. Procédé de la revendication 1, dans lequel ledit organisme est une bactérie, un virus, ou un pathogène microbien.

4. Procédé de la revendication 1, dans lequel lesdites séquences d'acide nucléique comprennent au moins un gène codant pour l'ARN ribosomique ou au moins un gène codant pour au moins une protéine qui participe à la traduction, la réplication, la recombinaison et la réparation, la transcription, le métabolisme des nucléotides, le métabolisme des acides aminés, le métabolisme des lipides, la génération d'énergie ou la sécrétion.

5. Procédé de la revendication 4, dans lequel ledit au moins un gène codant pour ladite au moins une protéine code pour l'ADN polymérase, l'ARN polymérase, le facteur d'allongement Tu, la protéine de choc thermique groEL, la phosphoglycérate kinase, la NADH déshydrogénase, l'ADN ligase, l'ADN topoisomérase, ou le facteur d'allongement G.

6. Procédé de la revendication 1, dans lequel lesdits sites de liaison d'amorce sont de 80 % à 100 % conservés entre lesdites séquences alignées de l'étape a).

7. Procédé de la revendication 1 dans lequel la région intercalée entre lesdits sites de liaison d'amorce n'a pas plus de 5 % d'identité de séquence entre lesdites séquences alignées de l'étape a).

8. Procédé de la revendication 1, dans lequel lesdites régions amplifiables sont d'entre 50 et 250 nucléotides de longueur.

9. Procédé de la revendication 1, dans lequel ladite identité est un nom de genre, un nom d'espèce, ou un nom de souche.


### SEQUENCE LISTING

<110> Ibis Biosciences, Inc.
<120> Method For Rapid Detection And Identification of Bioagents
<130> P055604EP
<150> US20010798007
   <151> 2001-03-02
<160> 7
<170> PatentIn version 3.0
<210> 1
   <211> 90
   <212> RNA
   <213> Bacillus anthracis
<220>
   <221> misc_feature
   <222> (20)..(20)
   <223> N = A, U, G or C
<400> 1
   gcgaagaacc uuaccaggun uugacauccu cugacaaccc uagagauagg gcuucuccuu 60
   cgggagcaga gugacaggug gugcaugguu 90
<210> 2
   <211> 90
   <212> RNA
   <213> Bacillus cereus
<400> 2
   gcgaagaacc uuaccagguc uugacauccu cugaaaaccc uagagauagg gcuucuccuu 60
   cgggagcaga gugacaggug gugcaugguu 90
<210> 3
   <211> 1542
   <212> RNA
   <213> Artificial Sequence
<220>
   <221> misc_feature
   <223> 16S rRNA consensus sequence
<220>
   <221> misc_feature
   <222> (1)..(7)
   <223> N= A, U, G or C
<220>
   <221> misc_feature
   <222> (19)..(19)
   <223> N= A, U, G or C
<220>
   <221> misc_feature
   <222> (28)..(30)
   <223> N= A, U, G or C
<220>
   <221> misc_feature
   <222> (43)..(45)
   <223> N= A, U, G or C
<220>
   <221> misc_feature
   <222> (48)..(48)
   <223> N= A, U, G or C
<220>
   <221> misc_feature
   <222> (50)..(50)
   <223> N= A, U, G or C
<220>
   <221> misc_feature
   <222> (52)..(52)
   <223> N= A, U, G or C
<220>
   <221> misc_feature
   <222> (66)..(66)
   <223> N= A, U, G or C
<220>
   <221> misc_feature
   <222> (69)..(69)
   <223> N= A, U, G or C
<220>
   <221> misc_feature
   <222> (100)..(100)
   <223> N= A, U, G or C
<220>
   <221> misc_feature
   <222> (103)..(103)
   <223> N= A, U, G or C
<220>
   <221> misc_feature
   <222> (107)..(108)
   <223> N= A, U, G or C
<220>
   <221> misc_feature
   <222> (121)..(122)
   <223> N= A, U, G or C
<220>
   <221> misc_feature
   <222> (124)..(124)
   <223> N= A, U, G or C
<220>
   <221> misc_feature
   <222> (126)..(129)
   <223> N= A, U, G or C
<220>
   <221> misc_feature
   <222> (131)..(132)
   <223> N= A, U, G or C
<220>
   <221> misc_feature
   <222> (134)..(134)
   <223> N= A, U, G or C
<220>
   <221> misc_feature
   <222> (137)..(145)
   <223> N= A, U, G or C
<220>
   <221> misc_feature
   <222> (148)..(148)
   <223> N= A, U, G or C
<220>
   <221> misc_feature
   <222> (150)..(150)
   <223> N= A, U, G or C
<220>
   <221> misc_feature
   <222> (152)..(158)
   <223> N= A, U, G or C
<220>
   <221> misc_feature
   <222> (163)..(169)
   <223> N= A, U, G or C
<220>
   <221> misc_feature
   <222> (177)..(178)
   <223> N= A, U, G or C
<220>
   <221> misc_feature
   <222> (181)..(194)
   <223> N= A, U, G or C
<220>
   <221> misc_feature
   <222> (199)..(226)
   <223> N= A, U, G or C
<220>
   <221> misc_feature
   <222> (229)..(237)
   <223> N= A, U, G or C
<220>
   <221> misc_feature
   <222> (239)..(242)
   <223> N= A, U, G or C
<220>
   <221> misc_feature
   <222> (245)..(245)
   <223> N= A, U, G or C
<220>
   <221> misc_feature
   <222> (248)..(248)
   <223> N= A, U, G or C
<220>
   <221> misc_feature
   <222> (250)..(250)
   <223> N= A, U, G or C
<220>
   <221> misc_feature
   <222> (257)..(258)
   <223> N= A, U, G or C
<220>
   <221> misc_feature
   <222> (264)..(264)
   <223> N= A, U, G or C
<220>
   <221> misc_feature
   <222> (268)..(269)
   <223> N= A, U, G or C
<220>
   <221> misc_feature
   <222> (276)..(276)
   <223> N= A, U, G or C
<220>
   <221> misc_feature
   <222> (278)..(280)
   <223> N= A, U, G or C
<220>
   <221> misc_feature
   <222> (283)..(286)
   <223> N= A, U, G or C
<220>
   <221> misc_feature
   <222> (291)..(291)
   <223> N= A, U, G or C
<220>
   <221> misc_feature
   <222> (293)..(294)
   <223> N= A, U, G or C
<220>
   <221> misc_feature
   <222> (303)..(304)
   <223> N= A, U, G or C
<220>
   <221> misc_feature
   <222> (306)..(307)
   <223> N= A, U, G or C
<220>
   <221> misc_feature
   <222> (309)..(309)
   <223> N= A, U, G or C
<220>
   <221> misc_feature
   <222> (316)..(316)
   <223> N= A, U, G or C
<220>
   <221> misc_feature
   <222> (320)..(320)
   <223> N= A, U, G or C
<220>
   <221> misc_feature
   <222> (328)..(328)
   <223> N= A, U, G or C
<220>
   <221> misc_feature
   <222> (333)..(333)
   <223> N= A, U, G or C
<220>
   <221> misc_feature
   <222> (337)..(337)
   <223> N= A, U, G or C
<220>
   <221> misc_feature
   <222> (359)..(360)
   <223> N= A, U, G or C
<220>
   <221> misc_feature
   <222> (366)..(366)
   <223> N= A, U, G or C
<220>
   <221> misc_feature
   <222> (369)..(371)
   <223> N= A, U, G or C
<220>
   <221> misc_feature
   <222> (378)..(379)
   <223> N= A, U, G or C
<220>
   <221> misc_feature
   <222> (381)..(381)
   <223> N= A, U, G or C
<220>
   <221> misc_feature
   <222> (384)..(385)
   <223> N= A, U, G or C
<220>
   <221> misc_feature
   <222> (390)..(392)
   <223> N= A, U, G or C
<220>
   <221> misc_feature
   <222> (396)..(396)
   <223> N= A, U, G or C
<220>
   <221> misc_feature
   <222> (398)..(399)
   <223> N= A, U, G or C
<220>
   <221> misc_feature
   <222> (407)..(409)
   <223> N= A, U, G or C
<220>
   <221> misc_feature
   <222> (412)..(412)
   <223> N= A, U, G or C
<220>
   <221> misc_feature
   <222> (415)..(415)
   <223> N= A, U, G or C
<220>
   <221> misc_feature
   <222> (418)..(419)
   <223> N= A, U, G or C
<220>
   <221> misc_feature
   <222> (421)..(423)
   <223> N= A, U, G or C
<220>
   <221> misc_feature
   <222> (425)..(425)
   <223> N= A, U, G or C
<220>
   <221> misc_feature
   <222> (427)..(427)
   <223> N= A, U, G or C
<220>
   <221> misc_feature
   <222> (433)..(435)
   <223> N= A, U, G or C
<220>
   <221> misc_feature
   <222> (438)..(438)
   <223> N= A, U, G or C
<220>
   <221> misc_feature
   <222> (440)..(446)
   <223> N= A, U, G or C
<220>
   <221> misc_feature
   <222> (449)..(449)
   <223> N= A, U, G or C
<220>
   <221> misc_feature
   <222> (452)..(479)
   <223> N= A, U, G or C
<220>
   <221> misc_feature
   <222> (484)..(485)
   <223> N= A, U, G or C
<220>
   <221> misc_feature
   <222> (488)..(494)
   <223> N= A, U, G or C
<220>
   <221> misc_feature
   <222> (496)..(497)
   <223> N= A, U, G or C
<220>
   <221> misc_feature
   <222> (501)..(503)
   <223> N= A, U, G or C
<220>
   <221> misc_feature
   <222> (508)..(508)
   <223> N= A, U, G or C
<220>
   <221> misc_feature
   <222> (513)..(513)
   <223> N= A, U, G or C
<220>
   <221> misc_feature
   <222> (537)..(537)
   <223> N= A, U, G or C
<220>
   <221> misc_feature
   <222> (542)..(543)
   <223> N= A, U, G or C
<220>
   <221> misc_feature
   <222> (546)..(546)
   <223> N= A, U, G or C
<220>
   <221> misc_feature
   <222> (553)..(555)
   <223> N= A, U, G or C
<220>
   <221> misc_feature
   <222> (560)..(560)
   <223> N= A, U, G or C
<220>
   <221> misc_feature
   <222> (562)..(562)
   <223> N= A, U, G or C
<220>
   <221> misc_feature
   <222> (564)..(564)
   <223> N= A, U, G or C
<220>
   <221> misc_feature
   <222> (576)..(576)
   <223> N= A, U, G or C
<220>
   <221> misc_feature
   <222> (578)..(580)
   <223> N= A, U, G or C
<220>
   <221> misc_feature
   <222> (582)..(582)
   <223> N= A, U, G or C
<220>
   <221> misc_feature
   <222> (586)..(586)
   <223> N= A, U, G or C
<220>
   <221> misc_feature
   <222> (589)..(596)
   <223> N= A, U, G or C
<220>
   <221> misc_feature
   <222> (599)..(603)
   <223> N= A, U, G or C
<220>
   <221> misc_feature
   <222> (606)..(606)
   <223> N= A, U, G or C
<220>
   <221> misc_feature
   <222> (610)..(616)
   <223> N= A, U, G or C
<220>
   <221> misc_feature
   <222> (620)..(620)
   <223> N= A, U, G or C
<220>
   <221> misc_feature
   <222> (624)..(633)
   <223> N= A, U, G or C
<220>
   <221> misc_feature
   <222> (635)..(641)
   <223> N= A, U, G or C
<220>
   <221> misc_feature
   <222> (644)..(650)
   <223> N= A, U, G or C
<220>
   <221> misc_feature
   <222> (653)..(653)
   <223> N= A, U, G or C
<220>
   <221> misc_feature
   <222> (657)..(662)
   <223> N= A, U, G or C
<220>
   <221> misc_feature
   <222> (665)..(665)
   <223> N= A, U, G or C
<220>
   <221> misc_feature
   <222> (668)..(673)
   <223> N= A, U, G or C
<220>
   <221> misc_feature
   <222> (679)..(682)
   <223> N= A, U, G or C
<220>
   <221> misc_feature
   <222> (689)..(689)
   <223> N= A, U, G or C
<220>
   <221> misc_feature
   <222> (694)..(694)
   <223> N= A, U, G or C
<220>
   <221> misc_feature
   <222> (697)..(697)
   <223> N= A, U, G or C
<220>
   <221> misc_feature
   <222> (701)..(701)
   <223> N= A, U, G or C
<220>
   <221> misc_feature
   <222> (705)..(705)
   <223> N= A, U, G or C
<220>
   <221> misc_feature
   <222> (708)..(709)
   <223> N= A, U, G or C
<220>
   <221> misc_feature
   <222> (711)..(711)
   <223> N= A, U, G or C
<220>
   <221> misc_feature
   <222> (713)..(713)
   <223> N= A, U, G or C
<220>
   <221> misc_feature
   <222> (717)..(717)
   <223> N= A, U, G or C
<220>
   <221> misc_feature
   <222> (721)..(722)
   <223> N= A, U, G or C
<220>
   <221> misc_feature
   <222> (724)..(724)
   <223> N= A, U, G or C
<220>
   <221> misc_feature
   <222> (733)..(738)
   <223> N= A, U, G or C
<220>
   <221> misc_feature
   <222> (743)..(748)
   <223> N= A, U, G or C
<220>
   <221> misc_feature
   <222> (755)..(755)
   <223> N= A, U, G or C
<220>
   <221> misc_feature
   <222> (758)..(758)
   <223> N= A, U, G or C
<220>
   <221> misc_feature
   <222> (760)..(763)
   <223> N= A, U, G or C
<220>
   <221> misc_feature
   <222> (770)..(770)
   <223> N= A, U, G or C
<220>
   <221> misc_feature
   <222> (775)..(775)
   <223> N= A, U, G or C
<220>
   <221> misc_feature
   <222> (780)..(780)
   <223> N= A, U, G or C
<220>
   <221> misc_feature
   <222> (808)..(808)
   <223> N= A, U, G or C
<220>
   <221> misc_feature
   <222> (811)..(812)
   <223> N= A, U, G or C
<220>
   <221> misc_feature
   <222> (819)..(819)
   <223> N= A, U, G or C
<220>
   <221> misc_feature
   <222> (822)..(826)
   <223> N= A, U, G or C
<220>
   <221> misc_feature
   <222> (828)..(831)
   <223> N= A, U, G or C
<220>
   <221> misc_feature
   <222> (833)..(835)
   <223> N= A, U, G or C
<220>
   <221> misc_feature
   <222> (837)..(859)
   <223> N= A, U, G or C
<220>
   <221> misc_feature
   <222> (861)..(863)
   <223> N= A, U, G or C
<220>
   <221> misc_feature
   <222> (868)..(870)
   <223> N= A, U, G or C
<220>
   <221> misc_feature
   <222> (874)..(878)
   <223> N= A, U, G or C
<220>
   <221> misc_feature
   <222> (895)..(896)
   <223> N= A, U, G or C
<220>
   <221> misc_feature
   <222> (903)..(904)
   <223> N= A, U, G or C
<220>
   <221> misc_feature
   <222> (906)..(906)
   <223> N= A, U, G or C
<220>
   <221> misc_feature
   <222> (916)..(916)
   <223> N= A, U, G or C
<220>
   <221> misc_feature
   <222> (929)..(929)
   <223> N= A, U, G or C
<220>
   <221> misc_feature
   <222> (932)..(932)
   <223> N= A, U, G or C
<220>
   <221> misc_feature
   <222> (941)..(941)
   <223> N= A, U, G or C
<220>
   <221> misc_feature
   <222> (943)..(943)
   <223> N= A, U, G or C
<220>
   <221> misc_feature
   <222> (948)..(948)
   <223> N= A, U, G or C
<220>
   <221> misc_feature
   <222> (955)..(955)
   <223> N= A, U, G or C
<220>
   <221> misc_feature
   <222> (965)..(965)
   <223> N= A, U, G or C
<220>
   <221> misc_feature
   <222> (967)..(968)
   <223> N= A, U, G or C
<220>
   <221> misc_feature
   <222> (974)..(974)
   <223> N= A, U, G or C
<220>
   <221> misc_feature
   <222> (976)..(976)
   <223> N= A, U, G or C
<220>
   <221> misc_feature
   <222> (986)..(990)
   <223> N= A, U, G or C
<220>
   <221> misc_feature
   <222> (998)..(1012)
   <223> N= A, U, G or C
<220>
   <221> misc_feature
   <222> (1015)..(1015)
   <223> N= A, U, G or C
<220>
   <221> misc_feature
   <222> (1017)..(1043)
   <223> N= A, U, G or C
<220>
   <221> misc_feature
   <222> (1051)..(1051)
   <223> N= A, U, G or C
<220>
   <221> misc_feature
   <222> (1059)..(1059)
   <223> N= A, U, G or C
<220>
   <221> misc_feature
   <222> (1075)..(1076)
   <223> N= A, U, G or C
<220>
   <221> misc_feature
   <222> (1082)..(1082)
   <223> N= A, U, G or C
<220>
   <221> misc_feature
   <222> (1100)..(1100)
   <223> N= A, U, G or C
<220>
   <221> misc_feature
   <222> (1115)..(1123)
   <223> N= A, U, G or C
<220>
   <221> misc_feature
   <222> (1127)..(1127)
   <223> N= A, U, G or C
<220>
   <221> misc_feature
   <222> (1129)..(1130)
   <223> N= A, U, G or C
<220>
   <221> misc_feature
   <222> (1132)..(1141)
   <223> N= A, U, G or C
<220>
   <221> misc_feature
   <222> (1143)..(1143)
   <223> N= A, U, G or C
<220>
   <221> misc_feature
   <222> (1145)..(1145)
   <223> N= A, U, G or C
<220>
   <221> misc_feature
   <222> (1150)..(1156)
   <223> N= A, U, G or C
<220>
   <221> misc_feature
   <222> (1163)..(1165)
   <223> N= A, U, G or C
<220>
   <221> misc_feature
   <222> (1167)..(1168)
   <223> N= A, U, G or C
<220>
   <221> misc_feature
   <222> (1171)..(1173)
   <223> N= A, U, G or C
<220>
   <221> misc_feature
   <222> (1183)..(1183)
   <223> N= A, U, G or C
<220>
   <221> misc_feature
   <222> (1189)..(1189)
   <223> N= A, U, G or C
<220>
   <221> misc_feature
   <222> (1198)..(1198)
   <223> N= A, U, G or C
<220>
   <221> misc_feature
   <222> (1201)..(1201)
   <223> N= A, U, G or C
<220>
   <221> misc_feature
   <222> (1207)..(1207)
   <223> N= A, U, G or C
<220>
   <221> misc_feature
   <222> (1214)..(1214)
   <223> N= A, U, G or C
<220>
   <221> misc_feature
   <222> (1216)..(1219)
   <223> N= A, U, G or C
<220>
   <221> misc_feature
   <222> (1225)..(1225)
   <223> N= A, U, G or C
<220>
   <221> misc_feature
   <222> (1231)..(1231)
   <223> N= A, U, G or C
<220>
   <221> misc_feature
   <222> (1233)..(1233)
   <223> N= A, U, G or C
<220>
   <221> misc_feature
   <222> (1243)..(1247)
   <223> N= A, U, G or C
<220>
   <221> misc_feature
   <222> (1251)..(1252)
   <223> N= A, U, G or C
<220>
   <221> misc_feature
   <222> (1254)..(1254)
   <223> N= A, U, G or C
<220>
   <221> misc_feature
   <222> (1256)..(1257)
   <223> N= A, U, G or C
<220>
   <221> misc_feature
   <222> (1260)..(1260)
   <223> N= A, U, G or C
<220>
   <221> misc_feature
   <222> (1262)..(1265)
   <223> N= A, U, G or C
<220>
   <221> misc_feature
   <222> (1267)..(1268)
   <223> N= A, U, G or C
<220>
   <221> misc_feature
   <222> (1270)..(1274)
   <223> N= A, U, G or C
<220>
   <221> misc_feature
   <222> (1278)..(1278)
   <223> N= A, U, G or C
<220>
   <221> misc_feature
   <222> (1281)..(1281)
   <223> N= A, U, G or C
<220>
   <221> misc_feature
   <222> (1283)..(1286)
   <223> N= A, U, G or C
<220>
   <221> misc_feature
   <222> (1290)..(1294)
   <223> N= A, U, G or C
<220>
   <221> misc_feature
   <222> (1297)..(1298)
   <223> N= A, U, G or C
<220>
   <221> misc_feature
   <222> (1302)..(1302)
   <223> N= A, U, G or C
<220>
   <221> misc_feature
   <222> (1308)..(1308)
   <223> N= A, U, G or C
<220>
   <221> misc_feature
   <222> (1310)..(1313)
   <223> N= A, U, G or C
<220>
   <221> misc_feature
   <222> (1324)..(1327)
   <223> N= A, U, G or C
<220>
   <221> misc_feature
   <222> (1329)..(1329)
   <223> N= A, U, G or C
<220>
   <221> misc_feature
   <222> (1335)..(1336)
   <223> N= A, U, G or C
<220>
   <221> misc_feature
   <222> (1340)..(1340)
   <223> N= A, U, G or C
<220>
   <221> misc_feature
   <222> (1354)..(1356)
   <223> N= A, U, G or C
<220>
   <221> misc_feature
   <222> (1362)..(1362)
   <223> N= A, U, G or C
<220>
   <221> misc_feature
   <222> (1364)..(1364)
   <223> N= A, U, G or C
<220>
   <221> misc_feature
   <222> (1366)..(1368)
   <223> N= A, U, G or C
<220>
   <221> misc_feature
   <222> (1383)..(1383)
   <223> N= A, U, G or C
<220>
   <221> misc_feature
   <222> (1388)..(1388)
   <223> N= A, U, G or C
<220>
   <221> misc_feature
   <222> (1409)..(1411)
   <223> N= A, U, G or C
<220>
   <221> misc_feature
   <222> (1414)..(1414)
   <223> N= A, U, G or C
<220>
   <221> misc_feature
   <222> (1416)..(1417)
   <223> N= A, U, G or C
<220>
   <221> misc_feature
   <222> (1420)..(1428)
   <223> N= A, U, G or C
<220>
   <221> misc_feature
   <222> (1431)..(1432)
   <223> N= A, U, G or C
<220>
   <221> misc_feature
   <222> (1436)..(1447)
   <223> N= A, U, G or C
<220>
   <221> misc_feature
   <222> (1449)..(1454)
   <223> N= A, U, G or C
<220>
   <221> misc_feature
   <222> (1456)..(1465)
   <223> N= A, U, G or C
<220>
   <221> misc_feature
   <222> (1467)..(1467)
   <223> N= A, U, G or C
<220>
   <221> misc_feature
   <222> (1469)..(1469)
   <223> N= A, U, G or C
<220>
   <221> misc_feature
   <222> (1472)..(1481)
   <223> N= A, U, G or C
<220>
   <221> misc_feature
   <222> (1484)..(1484)
   <223> N= A, U, G or C
<220>
   <221> misc_feature
   <222> (1489)..(1491)
   <223> N= A, U, G or C
<220>
   <221> misc_feature
   <222> (1508)..(1508)
   <223> N= A, U, G or C
<220>
   <221> misc_feature
   <222> (1511)..(1511)
   <223> N= A, U, G or C
<220>
   <221> misc_feature
   <222> (1514)..(1516)
   <223> N= A, U, G or C
<220>
   <221> misc_feature
   <222> (1520)..(1521)
   <223> N= A, U, G or C
<220>
   <221> misc_feature
   <222> (1524)..(1524)
   <223> N= A, U, G or C
<220>
   <221> misc_feature
   <222> (1527)..(1527)
   <223> N= A, U, G or C
<220>
   <221> misc_feature
   <222> (1542)..(1542)
   <223> N= A, U, G or C
<400> 3
   nnnnnnnaga guuugaucnu ggcucagnnn gaacgcuggc ggnnngcnun anacaugcaa     60
   gucgancgnn nnnnnnnnnn nnnnnnnnnn nnnnnnnnnn agnggcnnac gggugaguaa    120
   nncnunnnna nnunccnnnn nnnnnggnan annnnnnnga aannnnnnnu aauaccnnau    180
   nnnnnnnnnn nnnnaaagnn nnnnnnnnnn nnnnnnnnnn nnnnnngann nnnnnnngnn    240
   nnaunagnun guuggunngg uaanggcnna ccaagncnnn gannnnuagc ngnncugaga    300
   ggnngnncng ccacanuggn acugaganac ggnccanacu ccuacgggag gcagcagunn    360
   ggaaunuunn ncaauggnng naanncugan nnagcnannc cgcgugnnng anganggnnu    420
   nnngnungua aannncunun nnnnnngang annnnnnnnn nnnnnnnnnn nnnnnnnnnu    480
   gacnnuannn nnnnannaag nnncggcnaa cuncgugcca gcagccgcgg uaauacgnag    540
   gnngcnagcg uunnncggan unanugggcg uaaagngnnn gnaggnggnn nnnnnngunn    600
   nnngunaaan nnnnnngcun aacnnnnnnn nnncnnnnnn nacnnnnnnn cungagnnnn    660
   nnagnggnnn nnngaauunn nnguguagng gugnaauncg naganaunng nangaanacc    720
   nnungcgaag gcnnnnnncu ggnnnnnnac ugacncunan nnncgaaagc nugggnagcn    780
   aacaggauua gauacccugg uaguccangc nnuaaacgnu gnnnnnunnn ngnnngnnnn    840
   nnnnnnnnnn nnnnnnnnna nnnaacgnnn uaannnnncc gccuggggag uacgnncgca    900
   agnnunaaac ucaaangaau ugacggggnc cngcacaagc ngnggagnau guggnuuaau    960
   ucgangnnac gcgnanaacc uuaccnnnnn uugacaunnn nnnnnnnnnn nnganannnn   1020
   nnnnnnnnnn nnnnnnnnnn nnnacaggug nugcauggnu gucgucagcu cgugnnguga   1080
   gnuguugggu uaagucccgn aacgagcgca acccnnnnnn nnnguuncna ncnnnnnnnn   1140
   ngngnacucn nnnnnnacug ccnnngnnaa nnnggaggaa ggnggggang acgucaanuc   1200
   nucaugnccc uuangnnnng ggcuncacac nuncuacaau ggnnnnnaca nngngnngcn   1260
   annnngnnan nnnnagcnaa ncnnnnaaan nnnnucnnag uncggaungn nnncugcaac   1320
   ucgnnnncnu gaagnnggan ucgcuaguaa ucgnnnauca gnangnnncg gugaauacgu   1380
   ucncgggncu uguacacacc gcccgucann ncangnnagn nnnnnnnncc nnaagnnnnn   1440
   nnnnnnncnn nnnngnnnnn nnnnncnang gnnnnnnnnn nganugggnn naagucguaa   1500
   caagguancc nuannngaan nugnggnugg aucaccuccu un                      1542
<210> 4
   <211> 2904
   <212> RNA
   <213> Artificial Sequence
<220>
   <221> misc_feature
   <223> 23S rRNA consensus sequence
<220>
   <221> misc_feature
   <222> (1)..(4)
   <223> N= A, U, G or C
<220>
   <221> misc_feature
   <222> (8)..(12)
   <223> N= A, U, G or C
<220>
   <221> misc_feature
   <222> (16)..(16)
   <223> N= A,U, G or C
<220>
   <221> misc_feature
   <222> (18)..(22)
   <223> N= A,U, G or C
<220>
   <221> misc_feature
   <222> (34)..(34)
   <223> N= A,U, G or C
<220>
   <221> misc_feature
   <222> (38)..(43)
   <223> N= A,U, G or C
<220>
   <221> misc_feature
   <222> (46)..(46)
   <223> N= A,U, G or C
<220>
   <221> misc_feature
   <222> (50)..(50)
   <223> N= A,U, G or C
<220>
   <221> misc_feature
   <222> (57)..(57)
   <223> N= A, U, G or C
<220>
   <221> misc_feature
   <222> (59)..(65)
   <223> N= A, U, G or C
<220>
   <221> misc_feature
   <222> (67)..(68)
   <223> N= A, U, G or C
<220>
   <221> misc_feature
   <222> (70)..(72)
   <223> N= A, U, G or C
<220>
   <221> misc_feature
   <222> (74)..(75)
   <223> N= A, U, G or C
<220>
   <221> misc_feature
   <222> (77)..(79)
   <223> N= A, U, G or C
<220>
   <221> misc_feature
   <222> (82)..(83)
   <223> N= A, U, G or C
<220>
   <221> misc_feature
   <222> (86)..(87)
   <223> N= A, U, G or C
<220>
   <221> misc_feature
   <222> (89)..(96)
   <223> N= A, U, G or C
<220>
   <221> misc_feature
   <222> (98)..(102)
   <223> N= A, U, G or C
<220>
   <221> misc_feature
   <222> (104)..(104)
   <223> N= A, U, G or C
<220>
   <221> misc_feature
   <222> (107)..(109)
   <223> N= A, U, G or C
<220>
   <221> misc_feature
   <222> (111)..(113)
   <223> N= A, U, G or C
<220>
   <221> misc_feature
   <222> (113)..(113)
   <223> N= A, U, G or C
<220>
   <221> misc_feature
   <222> (125)..(125)
   <223> N= A, U, G or C
<220>
   <221> misc_feature
   <222> (131)..(148)
   <223> N= A, U, G or C
<220>
   <221> misc_feature
   <222> (150)..(177)
   <223> N= A, U, G or C
<220>
   <221> misc_feature
   <222> (179)..(181)
   <223> N= A, U, G or C
<220>
   <221> misc_feature
   <222> (184)..(188)
   <223> N= A, U, G or C
<220>
   <221> misc_feature
   <222> (192)..(192)
   <223> N= A, U, G or C
<220>
   <221> misc_feature
   <222> (203)..(203)
   <223> N= A, U, G or C
<220>
   <221> misc_feature
   <222> (208)..(212)
   <223> N= A, U, G or C
<220>
   <221> misc_feature
   <222> (218)..(218)
   <223> N= A, U, G or C
<220>
   <221> misc_feature
   <222> (224)..(225)
   <223> N= A, U, G or C
<220>
   <221> misc_feature
   <222> (228)..(231)
   <223> N= A, U, G or C
<220>
   <221> misc_feature
   <222> (236)..(236)
   <223> N= A, U, G or C
<220>
   <221> misc_feature
   <222> (238)..(241)
   <223> N= A, U, G or C
<220>
   <221> misc_feature
   <222> (246)..(246)
   <223> N= A, U, G or C
<220>
   <221> misc_feature
   <222> (257)..(259)
   <223> N= A, U, G or C
<220>
   <221> misc_feature
   <222> (261)..(261)
   <223> N= A, U, G or C
<220>
   <221> misc_feature
   <222> (263)..(264)
   <223> N= A, U, G or C
<220>
   <221> misc_feature
   <222> (267)..(267)
   <223> N= A, U, G or C
<220>
   <221> misc_feature
   <222> (269)..(293)
   <223> N= A, U, G or C
<220>
   <221> misc_feature
   <222> (295)..(297)
   <223> N= A, U, G or C
<220>
   <221> misc_feature
   <222> (301)..(305)
   <223> N= A, U, G or C
<220>
   <221> misc_feature
   <222> (309)..(309)
   <223> N= A, U, G or C
<220>
   <221> misc_feature
   <222> (313)..(321)
   <223> N= A, U, G or C
<220>
   <221> misc_feature
   <222> (323)..(325)
   <223> N= A, U, G or C
<220>
   <221> misc_feature
   <222> (329)..(329)
   <223> N= A, U, G or C
<220>
   <221> misc_feature
   <222> (331)..(331)
   <223> N= A, U, G or C
<220>
   <221> misc_feature
   <222> (333)..(334)
   <223> N= A, U, G or C
<220>
   <221> misc_feature
   <222> (337)..(337)
   <223> N= A, U, G or C
<220>
   <221> misc_feature
   <222> (341)..(344)
   <223> N= A, U, G or C
<220>
   <221> misc_feature
   <222> (348)..(370)
   <223> N= A, U, G or C
<220>
   <221> misc_feature
   <222> (375)..(377)
   <223> N= A, U, G or C
<220>
   <221> misc_feature
   <222> (379)..(382)
   <223> N= A, U, G or C
<220>
   <221> misc_feature
   <222> (384)..(384)
   <223> N= A, U, G or C
<220>
   <221> misc_feature
   <222> (387)..(387)
   <223> N= A, U, G or C
<220>
   <221> misc_feature
   <222> (389)..(390)
   <223> N= A, U, G or C
<220>
   <221> misc_feature
   <222> (392)..(395)
   <223> N= A, U, G or C
<220>
   <221> misc_feature
   <222> (398)..(399)
   <223> N= A, U, G or C
<220>
   <221> misc_feature
   <222> (403)..(405)
   <223> N= A, U, G or C
<220>
   <221> misc_feature
   <222> (407)..(410)
   <223> N= A, U, G or C
<220>
   <221> misc_feature
   <222> (416)..(421)
   <223> N= A, U, G or C
<220>
   <221> misc_feature
   <222> (425)..(425)
   <223> N= A, U, G or C
<220>
   <221> misc_feature
   <222> (435)..(441)
   <223> N= A, U, G or C
<220>
   <221> misc_feature
   <222> (446)..(446)
   <223> N= A, U, G or C
<220>
   <221> misc_feature
   <222> (451)..(451)
   <223> N= A, U, G or C
<220>
   <221> misc_feature
   <222> (453)..(453)
   <223> N= A, U, G or C

<220>
   <221> misc_feature
   <222> (455)..(456)
   <223> N= A, U, G or C
<220>
   <221> misc_feature
   <222> (462)..(462)
   <223> N= A, U, G or C
<220>
   <221> misc_feature
   <222> (467)..(467)
   <223> N= A, U, G or C
<220>
   <221> misc_feature
   <222> (475)..(475)
   <223> N= A, U, G or C
<220>
   <221> misc_feature
   <222> (482)..(482)
   <223> N= A, U, G or C
<220>
   <221> misc_feature
   <222> (487)..(491)
   <223> N= A, U, G or C
<220>
   <221> misc_feature
   <222> (493)..(493)
   <223> N= A, U, G or C
<220>
   <221> misc_feature
   <222> (504)..(504)
   <223> N= A, U, G or C
<220>
   <221> misc_feature
   <222> (507)..(508)
   <223> N= A, U, G or C
<220>
   <221> misc_feature
   <222> (518)..(522)
   <223> N= A, U, G or C
<220>
   <221> misc_feature
   <222> (524)..(524)
   <223> N= A, U, G or C
<220>
   <221> misc_feature
   <222> (527)..(527)
   <223> N= A, U, G or C
<220>
   <221> misc_feature
   <222> (530)..(532)
   <223> N= A, U, G or C
<220>
   <221> misc_feature
   <222> (535)..(537)
   <223> N= A, U, G or C
<220>
   <221> misc_feature
   <222> (540)..(553)
   <223> N= A, U, G or C
<220>
   <221> misc_feature
   <222> (557)..(558)
   <223> N= A, U, G or C
<220>
   <221> misc_feature
   <222> (563)..(563)
   <223> N= A, U, G or C
<220>
   <221> misc_feature
   <222> (571)..(571)
   <223> N= A, U, G or C
<220>
   <221> misc_feature
   <222> (573)..(574)
   <223> N= A, U, G or C
<220>
   <221> misc_feature
   <222> (578)..(580)
   <223> N= A, U, G or C
<220>
   <221> misc_feature
   <222> (582)..(582)
   <223> N= A, U, G or C
<220>
   <221> misc_feature
   <222> (584)..(584)
   <223> N= A, U, G or C
<220>
   <221> misc_feature
   <222> (587)..(587)
   <223> N= A, U, G or C
<220>
   <221> misc_feature
   <222> (590)..(593)
   <223> N= A, U, G or C
<220>
   <221> misc_feature
   <222> (595)..(599)
   <223> N= A, U, G or C
<220>
   <221> misc_feature
   <222> (602)..(602)
   <223> N= A, U, G or C
<220>
   <221> misc_feature
   <222> (605)..(605)
   <223> N= A, U, G or C
<220>
   <221> misc_feature
   <222> (610)..(618)
   <223> N= A, U, G or C
<220>
   <221> misc_feature
   <222> (620)..(620)
   <223> N= A, U, G or C
<220>
   <221> misc_feature
   <222> (623)..(623)
   <223> N= A, U, G or C
<220>
   <221> misc_feature
   <222> (626)..(626)
   <223> N= A, U, G or C
<220>
   <221> misc_feature
   <222> (629)..(629)
   <223> N= A, U, G or C
<220>
   <221> misc_feature
   <222> (634)..(634)
   <223> N= A, U, G or C
<220>
   <221> misc_feature
   <222> (640)..(642)
   <223> N= A, U, G or C
<220>
   <221> misc_feature
   <222> (645)..(646)
   <223> N= A, U, G or C
<220>
   <221> misc_feature
   <222> (648)..(648)
   <223> N= A, U, G or C
<220>
   <221> misc_feature
   <222> (652)..(654)
   <223> N= A, U, G or C
<220>
   <221> misc_feature
   <222> (658)..(662)
   <223> N= A, U, G or C
<220>
   <221> misc_feature
   <222> (664)..(667)
   <223> N= A, U, G or C
<220>
   <221> misc_feature
   <222> (672)..(672)
   <223> N= A, U, G or C
<220>
   <221> misc_feature
   <222> (677)..(677)
   <223> N= A, U, G or C
<220>
   <221> misc_feature
   <222> (679)..(681)
   <223> N= A, U, G or C
<220>
   <221> misc_feature
   <222> (686)..(686)
   <223> N= A, U, G or C
<220>
   <221> misc_feature
   <222> (690)..(692)
   <223> N= A, U, G or C
<220>
   <221> misc_feature
   <222> (696)..(697)
   <223> N= A, U, G or C
<220>
   <221> misc_feature
   <222> (702)..(702)
   <223> N= A, U, G or C
<220>
   <221> misc_feature
   <222> (708)..(712)
   <223> N= A, U, G or C
<220>
   <221> misc_feature
   <222> (717)..(717)
   <223> N= A, U, G or C
<220>
   <221> misc_feature
   <222> (719)..(723)
   <223> N= A, U, G or C
<220>
   <221> misc_feature
   <222> (730)..(730)
   <223> N= A, U, G or C
<220>
   <221> misc_feature
   <222> (737)..(744)
   <223> N= A, U, G or C
<220>
   <221> misc_feature
   <222> (753)..(758)
   <223> N= A, U, G or C
<220>
   <221> misc_feature
   <222> (765)..(766)
   <223> N= A, U, G or C
<220>
   <221> misc_feature
   <222> (771)..(772)
   <223> N= A, U, G or C
<220>
   <221> misc_feature
   <222> (774)..(774)
   <223> N= A, U, G or C
<220>
   <221> misc_feature
   <222> (776)..(776)
   <223> N= A, U, G or C
<220>
   <221> misc_feature
   <222> (779)..(779)
   <223> N= A, U, G or C
<220>
   <221> misc_feature
   <222> (784)..(785)
   <223> N= A, U, G or C
<220>
   <221> misc_feature
   <222> (787)..(787)
   <223> N= A, U, G or C
<220>
   <221> misc_feature
   <222> (790)..(790)
   <223> N= A, U, G or C
<220>
   <221> misc_feature
   <222> (792)..(792)
   <223> N= A, U, G or C
<220>
   <221> misc_feature
   <222> (796)..(798)
   <223> N= A, U, G or C
<220>
   <221> misc_feature
   <222> (800)..(801)
   <223> N= A, U, G or C
<220>
   <221> misc_feature
   <222> (815)..(816)
   <223> N= A, U, G or C
<220>
   <221> misc_feature
   <222> (822)..(825)
   <223> N= A, U, G or C
<220>
   <221> misc_feature
   <222> (832)..(835)
   <223> N= A, U, G or C
<220>
   <221> misc_feature
   <222> (838)..(838)
   <223> N= A, U, G or C
<220>
   <221> misc_feature
   <222> (840)..(854)
   <223> N= A, U, G or C
<220>
   <221> misc_feature
   <222> (857)..(857)
   <223> N= A, U, G or C
<220>
   <221> misc_feature
   <222> (870)..(879)
   <223> N= A, U, G or C
<220>
   <221> misc_feature
   <222> (882)..(894)
   <223> N= A, U, G or C
<220>
   <221> misc_feature
   <222> (898)..(899)
   <223> N= A, U, G or C
<220>
   <221> misc_feature
   <222> (901)..(907)
   <223> N= A, U, G or C
<220>
   <221> misc_feature
   <222> (914)..(914)
   <223> N= A, U, G or C
<220>
   <221> misc_feature
   <222> (920)..(920)
   <223> N= A, U, G or C
<220>
   <221> misc_feature
   <222> (923)..(938)
   <223> N= A, U, G or C
<220>
   <221> misc_feature
   <222> (940)..(940)
   <223> N= A, U, G or C
<220>
   <221> misc_feature
   <222> (943)..(944)
   <223> N= A, U, G or C
<220>
   <221> misc_feature
   <222> (946)..(947)
   <223> N= A, U, G or C
<220>
   <221> misc_feature
   <222> (949)..(951)
   <223> N= A, U, G or C
<220>
   <221> misc_feature
   <222> (953)..(953)
   <223> N= A, U, G or C
<220>
   <221> misc_feature
   <222> (955)..(955)
   <223> N= A, U, G or C
<220>
   <221> misc_feature
   <222> (957)..(957)
   <223> N= A, U, G or C
<220>
   <221> misc_feature
   <222> (961)..(962)
   <223> N= A, U, G or C
<220>
   <221> misc_feature
   <222> (964)..(964)
   <223> N= A, U, G or C
<220>
   <221> misc_feature
   <222> (966)..(968)
   <223> N= A, U, G or C
<220>
   <221> misc_feature
   <222> (971)..(972)
   <223> N= A, U, G or C
<220>
   <221> misc_feature
   <222> (974)..(974)
   <223> N= A, U, G or C
<220>
   <221> misc_feature
   <222> (979)..(979)
   <223> N= A, U, G or C
<220>
   <221> misc_feature
   <222> (984)..(984)
   <223> N= A, U, G or C
<220>
   <221> misc_feature
   <222> (991)..(991)
   <223> N= A, U, G or C
<220>
   <221> misc_feature
   <222> (993)..(994)
   <223> N= A, U, G or C
<220>
   <221> misc_feature
   <222> (996)..(998)
   <223> N= A, U, G or C
<220>
   <221> misc_feature
   <222> (1004)..(1008)
   <223> N= A, U, G or C
<220>
   <221> misc_feature
   <222> (1011)..(1018)
   <223> N= A, U, G or C
<220>
   <221> misc_feature
   <222> (1026)..(1026)
   <223> N= A, U, G or C
<220>
   <221> misc_feature
   <222> (1030)..(1030)
   <223> N= A, U, G or C
<220>
   <221> misc_feature
   <222> (1033)..(1033)
   <223> N= A, U, G or C
<220>
   <221> misc_feature
   <222> (1037)..(1042)
   <223> N= A, U, G or C
<220>
   <221> misc_feature
   <222> (1044)..(1045)
   <223> N= A, U, G or C
<220>
   <221> misc_feature
   <222> (1047)..(1047)
   <223> N= A, U, G or C
<220>
   <221> misc_feature
   <222> (1051)..(1053)
   <223> N= A, U, G or C
<220>
   <221> misc_feature
   <222> (1058)..(1058)
   <223> N= A, U, G or C
<220>
   <221> misc_feature
   <222> (1078)..(1078)
   <223> N= A, U, G or C
<220>
   <221> misc_feature
   <222> (1080)..(1080)
   <223> N= A, U, G or C
<220>
   <221> misc_feature
   <222> (1083)..(1083)
   <223> N= A, U, G or C
<220>
   <221> misc_feature
   <222> (1089)..(1090)
   <223> N= A, U, G or C
<220>
   <221> misc_feature
   <222> (1097)..(1097)
   <223> N= A, U, G or C
<220>
   <221> misc_feature
   <222> (1106)..(1107)
   <223> N= A, U, G or C
<220>
   <221> misc_feature
   <222> (1110)..(1110)
   <223> N= A, U, G or C
<220>
   <221> misc_feature
   <222> (1113)..(1119)
   <223> N= A, U, G or C
<220>
   <221> misc_feature
   <222> (1124)..(1124)
   <223> N= A, U, G or C
<220>
   <221> misc_feature
   <222> (1127)..(1128)
   <223> N= A, U, G or C
<220>
   <221> misc_feature
   <222> (1131)..(1131)
   <223> N= A, U, G or C
<220>
   <221> misc_feature
   <222> (1134)..(1134)
   <223> N= A, U, G or C
<220>
   <221> misc_feature
   <222> (1139)..(1139)
   <223> N= A, U, G or C
<220>
   <221> misc_feature
   <222> (1144)..(1151)
   <223> N= A, U, G or C
<220>
   <221> misc_feature
   <222> (1157)..(1162)
   <223> N= A, U, G or C
<220>
   <221> misc_feature
   <222> (1164)..(1185)
   <223> N= A, U, G or C
<220>
   <221> misc_feature
   <222> (1191)..(1192)
   <223> N= A, U, G or C
<220>
   <221> misc_feature
   <222> (1199)..(1211)
   <223> N= A, U, G or C
<220>
   <221> misc_feature
   <222> (1216)..(1222)
   <223> N= A, U, G or C
<220>
   <221> misc_feature
   <222> (1224)..(1225)
   <223> N= A, U, G or C
<220>
   <221> misc_feature
   <222> (1227)..(1233)
   <223> N= A, U, G or C
<220>
   <221> misc_feature
   <222> (1238)..(1246)
   <223> N= A, U, G or C
<220>
   <221> misc_feature
   <222> (1251)..(1251)
   <223> N= A, U, G or C
<220>
   <221> misc_feature
   <222> (1253)..(1253)
   <223> N= A, U, G or C
<220>
   <221> misc_feature
   <222> (1257)..(1258)
   <223> N= A, U, G or C
<220>
   <221> misc_feature
   <222> (1260)..(1261)
   <223> N= A, U, G or C
<220>
   <221> misc_feature
   <222> (1264)..(1264)
   <223> N= A, U, G or C
<220>
   <221> misc_feature
   <222> (1269)..(1269)
   <223> N= A, U, G or C
<220>
   <221> misc_feature
   <222> (1273)..(1280)
   <223> N= A, U, G or C
<220>
   <221> misc_feature
   <222> (1285)..(1285)
   <223> N= A, U, G or C
<220>
   <221> misc_feature
   <222> (1287)..(1288)
   <223> N= A, U, G or C
<220>
   <221> misc_feature
   <222> (1290)..(1294)
   <223> N= A, U, G or C
<220>
   <221> misc_feature
   <222> (1296)..(1296)
   <223> N= A, U, G or C
<220>
   <221> misc_feature
   <222> (1300)..(1300)
   <223> N= A, U, G or C
<220>
   <221> misc_feature
   <222> (1302)..(1304)
   <223> N= A, U, G or C
<220>
   <221> misc_feature
   <222> (1306)..(1306)
   <223> N= A, U, G or C
<220>
   <221> misc_feature
   <222> (1311)..(1311)
   <223> N= A, U, G or C
<220>
   <221> misc_feature
   <222> (1316)..(1321)
   <223> N= A, U, G or C
<220>
   <221> misc_feature
   <222> (1323)..(1323)
   <223> N= A, U, G or C
<220>
   <221> misc_feature
   <222> (1325)..(1325)
   <223> N= A, U, G or C
<220>
   <221> misc_feature
   <222> (1327)..(1328)
   <223> N= A, U, G or C
<220>
   <221> misc_feature
   <222> (1331)..(1336)
   <223> N= A, U, G or C
<220>
   <221> misc_feature
   <222> (1341)..(1341)
   <223> N= A, U, G or C
<220>
   <221> misc_feature
   <222> (1347)..(1349)
   <223> N= A, U, G or C
<220>
   <221> misc_feature
   <222> (1356)..(1357)
   <223> N= A, U, G or C
<220>
   <221> misc_feature
   <222> (1361)..(1361)
   <223> N= A, U, G or C
<220>
   <221> misc_feature
   <222> (1363)..(1363)
   <223> N= A, U, G or C
<220>
   <221> misc_feature
   <222> (1366)..(1366)
   <223> N= A, U, G or C
<220>
   <221> misc_feature
   <222> (1368)..(1368)
   <223> N= A, U, G or C
<220>
   <221> misc_feature
   <222> (1370)..(1371)
   <223> N= A, U, G or C
<220>
   <221> misc_feature
   <222> (1375)..(1376)
   <223> N= A, U, G or C
<220>
   <221> misc_feature
   <222> (1382)..(1383)
   <223> N= A, U, G or C
<220>
   <221> misc_feature
   <222> (1385)..(1387)
   <223> N= A, U, G or C
<220>
   <221> misc_feature
   <222> (1391)..(1392)
   <223> N= A, U, G or C
<220>
   <221> misc_feature
   <222> (1400)..(1402)
   <223> N= A, U, G or C
<220>
   <221> misc_feature
   <222> (1405)..(1425)
   <223> N= A, U, G or C
<220>
   <221> misc_feature
   <222> (1430)..(1435)
   <223> N= A, U, G or C
<220>
   <221> misc_feature
   <222> (1437)..(1454)
   <223> N= A, U, G or C
<220>
   <221> misc_feature
   <222> (1566)..(1567)
   <223> N= A, U, G or C
<220>
   <221> misc_feature
   <222> (1573)..(1599)
   <223> N= A, U, G or C
<220>
   <221> misc_feature
   <222> (1606)..(1607)
   <223> N= A, U, G or C
<220>
   <221> misc_feature
   <222> (1622)..(1622)
   <223> N= A, U, G or C
<220>
   <221> misc_feature
   <222> (1624)..(1627)
   <223> N= A, U, G or C
<220>
   <221> misc_feature
   <222> (1629)..(1630)
   <223> N= A, U, G or C
<220>
   <221> misc_feature
   <222> (1634)..(1634)
   <223> N= A, U, G or C
<220>
   <221> misc_feature
   <222> (1636)..(1637)
   <223> N= A, U, G or C
<220>
   <221> misc_feature
   <222> (1639)..(1640)
   <223> N= A, U, G or C
<220>
   <221> misc_feature
   <222> (1644)..(1644)
   <223> N= A, U, G or C
<220>
   <221> misc_feature
   <222> (1646)..(1647)
   <223> N= A, U, G or C
<220>
   <221> misc_feature
   <222> (1650)..(1653)
   <223> N= A, U, G or C
<220>
   <221> misc_feature
   <222> (1656)..(1663)
   <223> N= A, U, G or C
<220>
   <221> misc_feature
   <222> (1672)..(1673)
   <223> N= A, U, G or C
<220>
   <221> misc_feature
   <222> (1679)..(1679)
   <223> N= A, U, G or C
<220>
   <221> misc_feature
   <222> (1681)..(1684)
   <223> N= A, U, G or C
<220>
   <221> misc_feature
   <222> (1690)..(1690)
   <223> N= A, U, G or C
<220>
   <221> misc_feature
   <222> (1697)..(1697)
   <223> N= A, U, G or C
<220>
   <221> misc_feature
   <222> (1699)..(1699)
   <223> N= A, U, G or C
<220>
   <221> misc_feature
   <222> (1704)..(1707)
   <223> N= A, U, G or C
<220>
   <221> misc_feature
   <222> (1709)..(1749)
   <223> N= A, U, G or C
<220>
   <221> misc_feature
   <222> (1751)..(1754)
   <223> N= A, U, G or C
<220>
   <221> misc_feature
   <222> (1756)..(1758)
   <223> N= A, U, G or C
<220>
   <221> misc_feature
   <222> (1760)..(1762)
   <223> N= A, U, G or C
<220>
   <221> misc_feature
   <222> (1764)..(1770)
   <223> N= A, U, G or C
<220>
   <221> misc_feature
   <222> (1772)..(1772)
   <223> N= A, U, G or C
<220>
   <221> misc_feature
   <222> (1781)..(1782)
   <223> N= A, U, G or C
<220>
   <221> misc_feature
   <222> (1793)..(1794)
   <223> N= A, U, G or C
<220>
   <221> misc_feature
   <222> (1796)..(1797)
   <223> N= A, U, G or C
<220>
   <221> misc_feature
   <222> (1801)..(1801)
   <223> N= A, U, G or C
<220>
   <221> misc_feature
   <222> (1804)..(1805)
   <223> N= A, U, G or C
<220>
   <221> misc_feature
   <222> (1808)..(1808)
   <223> N= A, U, G or C
<220>
   <221> misc_feature
   <222> (1812)..(1813)
   <223> N= A, U, G or C
<220>
   <221> misc_feature
   <222> (1816)..(1816)
   <223> N= A, U, G or C
<220>
   <221> misc_feature
   <222> (1822)..(1822)
   <223> N= A, U, G or C
<220>
   <221> misc_feature
   <222> (1825) .. (1826)
   <223> N= A, U, G or C
<220>
   <221> misc_feature
   <222> (1831)..(1831)
   <223> N= A, U, G or C
<220>
   <221> misc_feature
   <222> (1839)..(1839)
   <223> N= A, U, G or C
<220>
   <221> misc_feature
   <222> (1844) .. (1845)
   <223> N= A, U, G or C
<220>
   <221> misc_feature
   <222> (1855) .. (1856)
   <223> N= A, U, G or C
<220>
   <221> misc_feature
   <222> (1858)..(1866)
   <223> N= A, U, G or C
<220>
   <221> misc_feature
   <222> (1868)..(1872)
   <223> N= A, U, G or C
<220>
   <221> misc_feature
   <222> (1874) .. (1884)
   <223> N= A, U, G or C
<220>
   <221> misc_feature
   <222> (1886)..(1888)
   <223> N= A, U, G or C
<220>
   <221> misc_feature
   <222> (1895)..(1896)
   <223> N= A, U, G or C
<220>
   <221> misc_feature
   <222> (1899)..(1899)
   <223> N= A, U, G or C
<220>
   <221> misc_feature
   <222> (1908)..(1909)
   <223> N= A, U, G or C
<220>
   <221> misc_feature
   <222> (1921)..(1922)
   <223> N= A, U, G or C
<220>
   <221> misc_feature
   <222> (1963)..(1963)
   <223> N= A, U, G or C
<220>
   <221> misc_feature
   <222> (1971)..(1971)
   <223> N= A, U, G or C
<220>
   <221> misc_feature
   <222> (1974)..(1974)
   <223> N= A, U, G or C
<220>
   <221> misc_feature
   <222> (1976)..(1976)
   <223> N= A, U, G or C
<220>
   <221> misc_feature
   <222> (1979)..(1979)
   <223> N= A, U, G or C
<220>
   <221> misc_feature
   <222> (1982)..(1989)
   <223> N= A, U, G or C
<220>
   <221> misc_feature
   <222> (1997)..(2005)
   <223> N= A, U, G or C
<220>
   <221> misc_feature
   <222> (2007)..(2007)
   <223> N= A, U, G or C
<220>
   <221> misc_feature
   <222> (2009)..(2009)
   <223> N= A, U, G or C
<220>
   <221> misc_feature
   <222> (2011)..(2011)
   <223> N= A, U, G or C
<220>
   <221> misc_feature
   <222> (2015)..(2015)
   <223> N= A, U, G or C
<220>
   <221> misc_feature
   <222> (2018)..(2019)
   <223> N= A, U, G or C
<220>
   <221> misc_feature
   <222> (2021)..(2021)
   <223> N= A, U, G or C
<220>
   <221> misc_feature
   <222> (2023)..(2026)
   <223> N= A, U, G or C
<220>
   <221> misc_feature
   <222> (2029)..(2029)
   <223> N= A, U, G or C
<220>
   <221> misc_feature
   <222> (2037)..(2040)
   <223> N= A, U, G or C
<220>
   <221> misc_feature
   <222> (2041)..(2041)
   <223> N= A, U, G or C
<220>
   <221> misc_feature
   <222> (2043)..(2043)
   <223> N= A, U, G or C
<220>
   <221> misc_feature
   <222> (2047)..(2052)
   <223> N= A, U, G or C
<220>
   <221> misc_feature
   <222> (2067)..(2068)
   <223> N= A, U, G or C
<220>
   <221> misc_feature
   <222> (2070)..(2070)
   <223> N= A, U, G or C
<220>
   <221> misc_feature
   <222> (2072)..(2072)
   <223> N= A, U, G or C
<220>
   <221> misc_feature
   <222> (2080)..(2081)
   <223> N= A, U, G or C
<220>
   <221> misc_feature
   <222> (2083)..(2085)
   <223> N= A, U, G or C
<220>
   <221> misc_feature
   <222> (2087)..(2089)
   <223> N= A, U, G or C
<220>
   <221> misc_feature
   <222> (2091)..(2091)
   <223> N= A, U, G or C
<220>
   <221> misc_feature
   <222> (2094)..(2108)
   <223> N= A, U, G or C
<220>
   <221> misc_feature
   <222> (2112)..(2113)
   <223> N= A, U, G or C
<220>
   <221> misc_feature
   <222> (2116)..(2116)
   <223> N= A, U, G or C
<220>
   <221> misc_feature
   <222> (2123)..(2123)
   <223> N= A, U, G or C
<220>
   <221> misc_feature
   <222> (2128)..(2128)
   <223> N= A, U, G or C
<220>
   <221> misc_feature
   <222> (2130)..(2132)
   <223> N= A, U, G or C
<220>
   <221> misc_feature
   <222> (2135)..(2142)
   <223> N= A, U, G or C
<220>
   <221> misc_feature
   <222> (2145)..(2146)
   <223> N= A, U, G or C
<220>
   <221> misc_feature
   <222> (2149)..(2155)
   <223> N= A, U, G or C
<220>
   <221> misc_feature
   <222> (2160)..(2160)
   <223> N= A, U, G or C
<220>
   <221> misc_feature
   <222> (2162)..(2166)
   <223> N= A, U, G or C
<220>
   <221> misc_feature
   <222> (2169)..(2170)
   <223> N= A, U, G or C
<220>
   <221> misc_feature
   <222> (2175)..(2175)
   <223> N= A, U, G or C
<220>
   <221> misc_feature
   <222> (2178)..(2178)
   <223> N= A, U, G or C
<220>
   <221> misc_feature
   <222> (2181)..(2194)
   <223> N= A, U, G or C
<220>
   <221> misc_feature
   <222> (2201)..(2211)
   <223> N= A, U, G or C
<220>
   <221> misc_feature
   <222> (2213)..(2213)
   <223> N= A, U, G or C
<220>
   <221> misc_feature
   <222> (2215)..(2223)
   <223> N= A, U, G or C
<220>
   <221> misc_feature
   <222> (2228)..(2228)
   <223> N= A, U, G or C
<220>
   <221> misc_feature
   <222> (2231)..(2233)
   <223> N= A, U, G or C
<220>
   <221> misc_feature
   <222> (2235)..(2236)
   <223> N= A, U, G or C
<220>
   <221> misc_feature
   <222> (2240)..(2240)
   <223> N= A, U, G or C
<220>
   <221> misc_feature
   <222> (2246)..(2246)
   <223> N= A, U, G or C
<220>
   <221> misc_feature
   <222> (2258)..(2259)
   <223> N= A, U, G or C
<220>
   <221> misc_feature
   <222> (2265)..(2265)
   <223> N= A, U, G or C
<220>
   <221> misc_feature
   <222> (2269)..(2270)
   <223> N= A, U, G or C
<220>
   <221> misc_feature
   <222> (2281)..(2281)
   <223> N= A, U, G or C
<220>
   <221> misc_feature
   <222> (2283)..(2284)
   <223> N= A, U, G or C
<220>
   <221> misc_feature
   <222> (2292)..(2294)
   <223> N= A, U, G or C
<220>
   <221> misc_feature
   <222> (2297)..(2297)
   <223> N= A, U, G or C
<220>
   <221> misc_feature
   <222> (2299)..(2302)
   <223> N= A, U, G or C
<220>
   <221> misc_feature
   <222> (2305)..(2306)
   <223> N= A, U, G or C
<220>
   <221> misc_feature
   <222> (2309)..(2310)
   <223> N= A, U, G or C
<220>
   <221> misc_feature
   <222> (2314)..(2321)
   <223> N= A, U, G or C
<220>
   <221> misc_feature
   <222> (2325)..(2326)
   <223> N= A, U, G or C
<220>
   <221> misc_feature
   <222> (2329)..(2330)
   <223> N= A, U, G or C
<220>
   <221> misc_feature
   <222> (2332)..(2332)
   <223> N= A, U, G or C
<220>
   <221> misc_feature
   <222> (2334)..(2334)
   <223> N= A, U, G or C
<220>
   <221> misc_feature
   <222> (2338)..(2340)
   <223> N= A, U, G or C
<220>
   <221> misc_feature
   <222> (2343)..(2343)
   <223> N= A, U, G or C

<220>
   <221> misc_feature
   <222> (2345)..(2345)
   <223> N= A, U, G or C
<220>
   <221> misc_feature
   <222> (2350)..(2351)
   <223> N= A, U, G or C
<220>
   <221> misc_feature
   <222> (2354)..(2357)
   <223> N= A, U, G or C
<220>
   <221> misc_feature
   <222> (2360)..(2363)
   <223> N= A, U, G or C
<220>
   <221> misc_feature
   <222> (2371)..(2373)
   <223> N= A, U, G or C
<220>
   <221> misc_feature
   <222> (2380)..(2381)
   <223> N= A, U, G or C
<220>
   <221> misc_feature
   <222> (2384)..(2386)
   <223> N= A, U, G or C
<220>
   <221> misc_feature
   <222> (2398)..(2398)
   <223> N= A, U, G or C
<220>
   <221> misc_feature
   <222> (2402)..(2407)
   <223> N= A, U, G or C
<220>
   <221> misc_feature
   <222> (2414)..(2414)
   <223> N= A, U, G or C
<220>
   <221> misc_feature
   <222> (2418)..(2418)
   <223> N= A, U, G or C
<220>
   <221> misc_feature
   <222> (2437)..(2437)
   <223> N= A, U, G or C
<220>
   <221> misc_feature
   <222> (2441)..(2441)
   <223> N= A, U, G or C
<220>
   <221> misc_feature
   <222> (2443)..(2443)
   <223> N= A, U, G or C
<220>
   <221> misc_feature
   <222> (2458)..(2458)
   <223> N= A, U, G or C
<220>
   <221> misc_feature
   <222> (2461)..(2464)
   <223> N= A, U, G or C
<220>
   <221> misc_feature
   <222> (2474)..(2474)
   <223> N= A, U, G or C
<220>
   <221> misc_feature
   <222> (2477)..(2477)
   <223> N= A, U, G or C
<220>
   <221> misc_feature
   <222> (2486)..(2489)
   <223> N= A, U, G or C
<220>
   <221> misc_feature
   <222> (2513)..(2513)
   <223> N= A, U, G or C
<220>
   <221> misc_feature
   <222> (2516)..(2516)
   <223> N= A, U, G or C
<220>
   <221> misc_feature
   <222> (2530)..(2530)
   <223> N= A, U, G or C
<220>
   <221> misc_feature
   <222> (2533)..(2534)
   <223> N= A, U, G or C
<220>
   <221> misc_feature
   <222> (2547)..(2548)
   <223> N= A, U, G or C
<220>
   <221> misc_feature
   <222> (2560)..(2561)
   <223> N= A, U, G or C
<220>
   <221> misc_feature
   <222> (2568)..(2568)
   <223> N= A, U, G or C
<220>
   <221> misc_feature
   <222> (2571)..(2571)
   <223> N= A, U, G or C
<220>
   <221> misc_feature
   <222> (2575)..(2575)
   <223> N= A, U, G or C
<220>
   <221> misc_feature
   <222> (2586)..(2586)
   <223> N= A, U, G or C
<220>
   <221> misc_feature
   <222> (2588)..(2588)
   <223> N= A, U, G or C
<220>
   <221> misc_feature
   <222> (2606)..(2606)
   <223> N= A, U, G or C
<220>
   <221> misc_feature
   <222> (2617)..(2617)
   <223> N= A, U, G or C
<220>
   <221> misc_feature
   <222> (2619)..(2620)
   <223> N= A, U, G or C
<220>
   <221> misc_feature
   <222> (2622)..(2622)
   <223> N= A, U, G or C
<220>
   <221> misc_feature
   <222> (2624)..(2624)
   <223> N= A, U, G or C
<220>
   <221> misc_feature
   <222> (2626)..(2626)
   <223> N= A, U, G or C
<220>
   <221> misc_feature
   <222> (2628)..(2630)
   <223> N= A, U, G or C
<220>
   <221> misc_feature
   <222> (2633)..(2635)
   <223> N= A, U, G or C
<220>
   <221> misc_feature
   <222> (2640)..(2642)
   <223> N= A, U, G or C
<220>
   <221> misc_feature
   <222> (2644)..(2646)
   <223> N= A, U, G or C
<220>
   <221> misc_feature
   <222> (2649)..(2650)
   <223> N= A, U, G or C
<220>
   <221> misc_feature
   <222> (2652)..(2652)
   <223> N= A, U, G or C
<220>
   <221> misc_feature
   <222> (2670)..(2674)
   <223> N= A, U, G or C
<220>
   <221> misc_feature
   <222> (2677)..(2678)
   <223> N= A, U, G or C
<220>
   <221> misc_feature
   <222> (2680)..(2680)
   <223> N= A, U, G or C
<220>
   <221> misc_feature
   <222> (2682)..(2682)
   <223> N= A, U, G or C
<220>
   <221> misc_feature
   <222> (2689)..(2691)
   <223> N= A, U, G or C
<220>
   <221> misc_feature
   <222> (2693)..(2693)
   <223> N= A, U, G or C
<220>
   <221> misc_feature
   <222> (2699)..(2701)
   <223> N= A, U, G or C
<220>
   <221> misc_feature
   <222> (2706)..(2708)
   <223> N= A, U, G or C
<220>
   <221> misc_feature
   <222> (2712)..(2713)
   <223> N= A, U, G or C
<220>
   <221> misc_feature
   <222> (2716)..(2716)
   <223> N= A, U, G or C
<220>
   <221> misc_feature
   <222> (2718)..(2719)
   <223> N= A, U, G or C
<220>
   <221> misc_feature
   <222> (2726)..(2727)
   <223> N= A, U, G or C
<220>
   <221> misc_feature
   <222> (2729)..(2730)
   <223> N= A, U, G or C
<220>
   <221> misc_feature
   <222> (2733)..(2736)
   <223> N= A, U, G or C
<220>
   <221> misc_feature
   <222> (2742)..(2743)
   <223> N= A, U, G or C
<220>
   <221> misc_feature
   <222> (2750)..(2750)
   <223> N= A, U, G or C
<220>
   <221> misc_feature
   <222> (2760)..(2762)
   <223> N= A, U, G or C
<220>
   <221> misc_feature
   <222> (2766)..(2766)
   <223> N= A, U, G or C
<220>
   <221> misc_feature
   <222> (2768)..(2770)
   <223> N= A, U, G or C
<220>
   <221> misc_feature
   <222> (2771)..(2774)
   <223> N= A, U, G or C
<220>
   <221> misc_feature
   <222> (2778)..(2779)
   <223> N= A, U, G or C
<220>
   <221> misc_feature
   <222> (2783)..(2785)
   <223> N= A, U, G or C
<220>
   <221> misc_feature
   <222> (2788)..(2788)
   <223> N= A, U, G or C
<220>
   <221> misc_feature
   <222> (2790)..(2809)
   <223> N= A, U, G or C
<220>
   <221> misc_feature
   <222> (2812)..(2814)
   <223> N= A, U, G or C
<220>
   <221> misc_feature
   <222> (2816)..(2820)
   <223> N= A, U, G or C
<220>
   <221> misc_feature
   <222> (2824)..(2825)
   <223> N= A, U, G or C
<220>
   <221> misc_feature
   <222> (2827)..(2830)
   <223> N= A, U, G or C
<220>
   <221> misc_feature
   <222> (2833)..(2833)
   <223> N= A, U, G or C
<220>
   <221> misc_feature
   <222> (2840)..(2842)
   <223> N= A, U, G or C
<220>
   <221> misc_feature
   <222> (2844)..(2846)
   <223> N= A, U, G or C
<220>
   <221> misc_feature
   <222> (2849)..(2849)
   <223> N= A, U, G or C
<220>
   <221> misc_feature
   <222> (2853)..(2856)
   <223> N= A, U, G or C
<220>
   <221> misc_feature
   <222> (2858)..(2859)
   <223> N= A, U, G or C
<220>
   <221> misc_feature
   <222> (2861)..(2864)
   <223> N= A, U, G or C
<220>
   <221> misc_feature
   <222> (2866)..(2867)
   <223> N= A, U, G or C
<220>
   <221> misc_feature
   <222> (2870)..(2872)
   <223> N= A, U, G or C
<220>
   <221> misc_feature
   <222> (2875)..(2877)
   <223> N= A, U, G or C
<220>
   <221> misc_feature
   <222> (2885)..(2888)
   <223> N= A, U, G or C
<220>
   <221> misc_feature
   <222> (2890)..(2895)
   <223> N= A, U, G or C
<220>
   <221> misc_feature
   <222> (2899)..(2904)
   <223> N= A, U, G or C
<400> 4
   nnnnaagnnn nnaagngnnn nngguggaug ccunggcnnn nnnagncgan gaaggangnn 60
   nnnnncnncn nnanncnnng gnnagnngnn nnnnnncnnn nnanccnnng nunuccgaau 120
   ggggnaaccc nnnnnnnnnn nnnnnnnnan nnnnnnnnnn nnnnnnnnnn nnnnnnngnn 180
   nacnnnnnga anugaaacau cunaguannn nnaggaanag aaannaannn ngauuncnnn 240
   nguagnggcg agcgaannng nannagncnn nnnnnnnnnn nnnnnnnnnn nnnannngaa 300
   nnnnnuggna agnnnnnnnn nannngguna nannccngua nnnnaaannn nnnnnnnnnn 360
   nnnnnnnnnn aguannncnn nncncgngnn annnngunng aannngnnnn gaccannnnn 420
   naagncuaaa uacunnnnnn ngaccnauag ngnannagua cngugangga aaggngaaaa 480
   gnacccnnnn nangggagug aaanagnncc ugaaaccnnn nncnuanaan nngunnnagn 540
   nnnnnnnnnn nnnuganngc gunccuuuug nannaugnnn cngnganuun nnnunnnnng 600
   cnagnuuaan nnnnnnnngn agncgnagng aaancgagun nnaanngngc gnnnagunnn 660
   nngnnnnaga cncgaancnn ngugancuan nnaugnncag gnugaagnnn nnguaanann 720
   nnnuggaggn ccgaacnnnn nnnnguugaa aannnnnngg augannugug nnungnggng 780
   aaanncnaan cnaacnnngn nauagcuggu ucucnncgaa annnnuuuag gnnnngcnun 840
   nnnnnnnnnn nnnnggnggu agagcacugn nnnnnnnnng gnnnnnnnnn nnnnuacnna 900
   nnnnnnnnaa acuncgaaun ccnnnnnnnn nnnnnnnngn agnnanncnn ngngngnuaa 960
   nnuncnnngu nnanagggna acancccaga ncnncnnnua aggncccnaa nnnnnnnnua 1020
   aguggnaaan gangugnnnn nncnnanaca nnnaggangu uggcuuagaa gcagccancn 1080
   uunaaagann gcguaanagc ucacunnucn agnnnnnnng cgcngannau nuancgggnc 1140
   uaannnnnnn nccgaannnn nngnnnnnnn nnnnnnnnnn nnnnngguag nngagcgunn 1200
   nnnnnnnnnn ngaagnnnnn nngnnannnn nnnuggannn nnnnnnagug ngnaugnngn 1260
   naunaguanc gannnnnnnn gugananncn nnnncnccgn annncnaagg nuuccnnnnn 1320
   nangnunnuc nnnnnngggu nagucgnnnc cuaagnngag ncnganangn nuagnngaug 1380
   gnnannnggu nnauauuccn nnacnnnnnn nnnnnnnnnn nnnnngacgn nnnnngnnnn 1440
   nnnnnnnnnn nnnnggnnnn nnnnnnnnnn nnnnnnnnnn nnnnnnnnnn nnnnnnnnnn 1500
   nnnnnnnnnn nnnnnnnnnn nnnnnnnnnn nnnnnnnnnn nnnnnnnnnn nnnnnnnnnn 1560
   nnnncnngaa aannnnnnnn nnnnnnnnnn nnnnnnnnnc guaccnnaaa ccgacacagg 1620
   ungnnnngnn gagnanncnn aggngnnngn nnnaannnnn nnnaaggaac unngcaaanu 1680
   nnnnccguan cuucggnana aggnnnncnn nnnnnnnnnn nnnnnnnnnn nnnnnnnnnn 1740
   nnnnnnnnng nnnnannnan nngnnnnnnn cnacuguuua nnaaaaacac agnncnnugc 1800
   naanncgnaa gnnganguau anggnnugac nccugcccng ugcnngaagg uuaanngnnn 1860
   nnnnnngnnn nngnnnnnnn nnnnannnaa gcccnnguna acggcggnng uaacuauaac   1920
   nnuccuaagg uagcgaaauu ccuugucggg uaaguuccga ccngcacgaa nggngnaang   1980
   annnnnnnnc ugucucnnnn nnnnncncng ngaanuunna nunnnnguna agaugcnnnn   2040
   uncncgcnnn nngacggaaa gaccccnngn ancuuuacun nannnunnna nugnnnnnnn   2100
   nnnnnnnnug unnagnauag gunggagncn nngannnnnn nncgnnagnn nnnnnggagn   2160
   cnnnnnugnn auacnacncu nnnnnnnnnn nnnnucuaac nnnnnnnnnn nancnnnnnn   2220
   nnngacanug nnngnngggn aguuunacug gggcggunnc cuccnaaann guaacggagg   2280
   ngnncnaagg unnncunann nnggnnggnn aucnnnnnnn nagunnaann gnanaagnnn   2340
   gcnunacugn nagnnnnacn nnncgagcag nnncgaaagn nggnnnuagu gauccggngg   2400
   unnnnnnugg aagngccnuc gcucaacgga uaaaagnuac ncnggggaua acaggcunau   2460
   nnnncccaag aguncanauc gacggnnnng uuuggcaccu cgaugucggc ucnucncauc   2520
   cuggggcugn agnngguccc aagggunngg cuguucgccn nuuaaagngg nacgngagcu   2580
   ggguunanaa cgucgugaga caguungguc ccuaucngnn gngngngnnn gannnuugan   2640
   nngnnnugnn cnuaguacga gaggaccggn nngnacnnan cncuggugnn ncnguugunn   2700
   ngccannngc anngcngnnu agcuannunn ggnnnngaua anngcugaan gcaucuaagn   2760
   nngaancnnn cnnnnagann agnnnucncn nnnnnnnnnn nnnnnnnnna gnnncnnnnn   2820
   agannannnn gungauaggn nngnnnugna agnnnngnna nnnnunnagn nnacnnnuac   2880
   uaaunnnncn nnnnncuunn nnnn                                          2904
<210> 5
   <211> 13
   <212> DNA
   <213> Artificial Sequence
<220>
   <221> misc_feature
   <223> Primer
<400> 5
   cgtggtgacc ctt 13
<210> 6
   <211> 14
   <212> DNA
   <213> Artificial sequence
<220>
   <221> misc_feature
   <223> Primer
<400> 6
   cgtcgtcacc gcta 14
<210> 7
   <211> 13
   <212> DNA
   <213> Artificial Sequence
<220>
   <221> misc_features
   <223> Primer
<400> 7
   cgtggtaccc ctt 13